# EUROPEAN PATENT APPLICATION

(11) **EP 4 599 882 A2**
(43) Date of publication of application: **13.08.2025**
(21) Application number: 25158443.9
(22) Date of filing: 06.03.2020
(51) Int. Cl.: A61N 1/36

(54) **WEARABLE PERIPHERAL NERVE STIMULATION FOR THE TREATMENT OF DISEASES UTILIZING RHYTHMIC BIOLOGICAL PROCESSES**

(30) Priority: 08.03.2019 US 201962815920 P
(62) Divisional of application: 20770557.5
(71) Applicant: Cala Health, Inc., San Mateo, CA 94404 (US)
(72) Inventor: Hamner, Samuel Richard, Burlingame, 94010 (US); Barreau, Ariana, Burlingame, 94010 (US); Colombo, John Vincent, Burlingame, 94010 (US); Liberatore, Jessica M., Burlingame, 94010 (US); Rosenbluth, Kathryn H., Burlingame, 94010 (US)
(74) Representative: DTS Patent- und Rechtsanwälte PartmbB

(57) **Abstract**

Systems and methods of transcutaneously stimulating one or more peripheral nerves of a patient with a wearable neurostimulation device regulated by a measured rhythmic biological signal to treat a patient are disclosed, and can include measuring a rhythmic biological signal of the patient and determining one or more features of the measured rhythmic biological signal of the patient in real-time; delivering a first electrical nerve stimulation signal transcutaneously to the first peripheral nerve effector to stimulate the first peripheral nerve sufficient to modify a first neural circuit to modulate nervous system activity of the patient at least in part based on the measured rhythmic biological signal; and delivering a second electrical nerve stimulation signal transcutaneously to the second peripheral nerve effector to stimulate the second peripheral nerve sufficient to modify the first or a second neural feedback loop to modulate nervous system activity of the patient at least in part based on the measured rhythmic biological signal.

## Description

### FIELD

This application claims the benefit under 35 U.S.C. § 119(e) as a nonprovisional application of U.S. App. No. 62/815,920 filed on March 8, 2019, which is hereby incorporated by reference in its entirety.

### BACKGROUND

The autonomic nervous system controls visceral function of the body, such as blood pressure, cardiac rhythm, bladder function, stress level, sleep function, and hormonal signaling. The autonomic nervous system is made up of two limbs, the sympathetic nervous system and the parasympathetic nervous system.

The sympathetic nervous system activates what is often termed the fight or flight response. Like other parts of the nervous system, the sympathetic nervous system operates through a series of interconnected neurons. Sympathetic neurons are frequently considered part of the peripheral nervous system, although there are many that lie within the central nervous system. Sympathetic neurons of the spinal cord (which is part of the central nervous system) communicate with peripheral sympathetic neurons via a series of sympathetic ganglia. Within the ganglia, spinal cord sympathetic neurons join peripheral sympathetic neurons through chemical synapses. Spinal cord sympathetic neurons are therefore called presynaptic (or preganglionic) neurons, while peripheral sympathetic neurons are called postsynaptic (or postganglionic) neurons. At synapses within the sympathetic ganglia, preganglionic sympathetic neurons release acetylcholine, a chemical messenger that binds and activates nicotinic acetylcholine receptors on postganglionic neurons. In response to this stimulus, postganglionic neurons principally release noradrenaline (norepinephrine). Prolonged activation can elicit the release of adrenaline from the adrenal medulla.

Once released, noradrenaline and adrenaline bind adrenergic receptors on peripheral tissues. Binding to adrenergic receptors causes the effects seen during the fight-or-flight response. These include pupil dilation, increased sweating, increased heart rate, and increased blood pressure. Sympathetic nerves originate inside the vertebral column, toward the middle of the spinal cord in the intermediolateral cell column (or lateral horn), beginning at the first thoracic segment of the spinal cord and are thought to extend to the second or third lumbar segments. Because its cells begin in the thoracic and lumbar regions of the spinal cord, the CNS is said to have a thoracolumbar outflow. Axons of these nerves leave the spinal cord in the ventral branches (rami) of the spinal nerves, and then separate out as 'white rami' (so called from the shiny white sheaths of myelin around each axon) which connect to two chain ganglia extending alongside the vertebral column on the left and right. These elongated ganglia are also known as paravertebral ganglia or sympathetic trunks. In these hubs, connections (synapses) are made which then distribute the nerves to major organs, glands, and other parts of the body.

Tonic rhythmic discharges within the sympathetic nervous system controls resting vasomotor tone, and modulates of the arterial baroreflex to maintain blood pressure (BP) homeostasis. The sympathetic nervous system also controls many other physiological processes including metabolism, renal control, inflammatory pathways, cardiac rhythm, and more. Activity within specific regions and locations of the central nervous system that govern sympathetic outflow, which can be modulated by electrical stimulation of peripheral nerve. Many diseases are associated with abnormal central sympathetic regulation, which leads to chronically elevated sympathetic activity or sympathetic outflow.

The parasympathetic system is responsible for stimulation of rest-and-digest or feed-and-breed activities that occur when the body is at rest, especially after eating, including sexual arousal, salivation, lacrimation (tears), urination, digestion and defecation. Its action is described as being complementary to that of the sympathetic nervous system, which is responsible for stimulating activities associated with the fight-or-flight response. Nerve fibers of the parasympathetic nervous system arise from the central nervous system. Specific nerves include several cranial nerves, specifically the oculomotor nerve, facial nerve, glossopharyngeal nerve, and vagus nerve. Three spinal nerves in the sacrum (S2-4), commonly referred to as the pelvic splanchnic nerves, also act as parasympathetic nerves. Owing to its location, the parasympathetic system is commonly referred to as having craniosacral outflow, which stands in contrast to the sympathetic nervous system, which is said to have thoracolumbar outflow.

Dysfunction or imbalance of the autonomic nervous system is believed to be a potential underlying mechanism for various chronic diseases. Autonomic dysfunction develops when the nerves of the ANS are damaged or degraded. This condition is called autonomic neuropathy or dysautonomia. Autonomic dysfunction can range from mild to life-threatening and can affect part of the ANS or the entire ANS. Sometimes the conditions that cause problems are temporary and reversible. Others are chronic, or long term, and may continue to worsen over time. Examples of chronic diseases that are associated with autonomic dysfunction include, but are not limited to, diabetes, Parkinson's disease, tremor, cardiac arrhythmias including atrial fibrillation, hypertension, overactive bladder, urinary incontinence, fecal incontinence, inflammatory bowel diseases, irritable bowel syndrome, gastroparesis, functional dyspepsia, other gastrointestinal disorders, rheumatoid arthritis, psoriasis, insomnia, migraine, depression, and anxiety.

In some embodiments, systems and methods can be configured to treat one, two, three or more non-tremor symptoms of Parkinson's disease. Such non-tremor Parkinson's symptoms can include motor symptoms such as, for example, rigidity, bradykinesia (including, for example, hypomimia or facial masking, a decreased blink rate, and micrographia), postural instability, gait difficulties (including, for example, freezing of gait, festination, and propulsion), dystonia, vocal symptoms (including, for example, stuttering, rapid speech, and softening of the voice). Non-tremor non-motor Parkinson's symptoms can also include, for example, hyposmia, anosmia, insomnia, depression, anxiety, pain, psychosis, fatigue, dementia, weight loss, GI disturbances (including, for example, constipation, abdominal pain, nausea, vomiting, and diarrhea), lightheadedness, urinary symptoms (including, for example, frequency, urgency, hesitancy, and neurogenic bladder), excessive sweating, impotence, reduced libido, and personality changes, including, for example, impulse control disorders. In some embodiments, systems and methods can be configured to treat tremor, including Parkinson's tremor.

In some embodiments, systems and methods can be configured to treat a sleep disorder, including but not limited to insomnia, such as acute or chronic insomnia, or symptoms thereof, such as, for example, difficulty falling asleep, early waking during the night, daytime tiredness or sleepiness, attention and/or memory difficulties, and the like. Other sleep disorders that can be treated with systems and methods as disclosed herein can include, for example, sleep apnea, narcolepsy, restless legs syndrome, and REM sleep behavior disorder, sleepwalking, hypersomnia, somniphobia, catathrenia, bruxism, sleep apnea, sleep paralysis, and night terrors.

External activation of peripheral nerves can modulate the central neural networks that affect the autonomic nervous system. Various ways to active nerves include mechanical stimulation, such as with skin stretch or pressure, electrical stimulation, electromechanical stimulation, ultrasonic stimulation, acupuncture or electroacupuncture, auditory stimulation, chemical agents, and optogenetic techniques.

The vagus nerve is an example of a particular nerve of interest for stimulation, as is a major nerve in the human body that modulates the autonomic nervous system. The vagus nerve, named after the Latin word vagus (because the nerve controls such a broad range of target tissues - vagus in Latin literally means "wandering"), has parasympathetic that originate in the dorsal nucleus of the vagus nerve and the nucleus ambiguus in the CNS. The vagus has an autonomic ganglion associated with it at approximately the level of C1 vertebra. The vagus nerve is hard to track definitively due to its ubiquitous nature in the thorax and abdomen so the major contributions will be discussed. Several parasympathetic nerves come off the vagus nerve as it enters the thorax. One nerve is the recurrent laryngeal nerve, which becomes the inferior laryngeal nerve. From the left vagus nerve the recurrent laryngeal nerve hooks around the aorta to travel back up to the larynx and proximal esophagus while, from the right vagus nerve, the recurrent laryngeal nerve hooks around the right subclavian artery to travel back up to the same location as its counterpart. These different paths are a direct result of embryological development of the circulatory system. Each recurrent laryngeal nerve supplies the trachea and the esophagus with parasympathetic secretomotor innervation for glands associated with them (and other fibers that are not peripheral nerves).

Another nerve that comes off the vagus nerves approximately at the level of entering the thorax are the cardiac nerves. These cardiac nerves go on to form cardiac and pulmonary plexuses around the heart and lungs. As the main vagus nerves continue into the thorax they become intimately linked with the esophagus and sympathetic nerves from the sympathetic trunks to form the esophageal plexus, which is efficient as the major function of the vagus nerve from there on will be control of the gut smooth muscles and glands. As the esophageal plexus enter the abdomen through the esophageal hiatus anterior and posterior vagus trunks form. The vagus trunks then join with preaortic sympathetic ganglion around the aorta to disperse with the blood vessels and sympathetic nerves throughout the abdomen. The extent of the parasympathetic in the abdomen include innervation with the pancreas, kidneys, liver, gall bladder, stomach and gut tube. The vagus contribution of parasympathetic continues down the gut tube until the end of the midgut. The midgut ends two thirds of the way across the transverse colon near the splenic flexure.

The median nerve is a major peripheral nerve of the upper arm. For motor functions, the median nerve innervates the flexor and pronator muscles in the anterior compartment of the forearm (except the flexor carpi ulnaris and part of the flexor digitorum profundus, innervated by the ulnar nerve). The median nerve also supplies innervation to the thenar muscles and lateral two lumbricals in the hand. For sensory functions, the median nerve gives rise to the palmar cutaneous branch, which innervates the lateral aspect of the palm, and the digital cutaneous branch, which innervates the lateral three and a half fingers on the anterior (palmar) surface of the hand. The median nerve is derived from the medial and lateral cords of the brachial plexus. It contains fibers from roots C6-T1, and can contain fibers from C5 in some individuals. After originating from the brachial plexus in the axilla, the median nerve descends down the arm, initially lateral to the brachial artery. Halfway down the arm, the nerve crosses over the brachial artery, and becomes situated medially. The median nerve enters the anterior compartment of the forearm via the cubital fossa. In the forearm, the nerve travels between the flexor digitorum profundus and flexor digitorum superficialis muscles. The median nerve gives off two major branches in the forearm, the anterior interosseous nerve and the palmar cutaneous nerve. After giving off the anterior interosseous and palmar cutaneous branches, the median nerve enters the hand via the carpal tunnel - where it terminates by dividing into two branches, the recurrent branch and the palmar digital branch. The median nerve is responsible for the cutaneous innervation of part of the hand. This is achieved via two branches, the palmar cutaneous branch and the palmar digital cutaneous branch.

The tibial nerve is a branch of the sciatic nerve, and arises at the apex of the popliteal fossa. It travels through the popliteal fossa, giving off branches to muscles in the superficial posterior compartment of the leg. Here, the tibial nerve also gives rise to branches that contribute towards the sural nerve, which innervates the posterolateral aspect of the leg. The tibial nerve continues its course down the leg, posterior to the tibia. During its descent, it supplies the deep muscles of the posterior leg. At the foot, the nerve passes posteriorly and inferiorly to the medial malleolus, through a structure known as the tarsal tunnel. This tunnel is covered superiorly by the flexor retinaculum. Within this tunnel, branches arise from the tibial nerve to supply cutaneous innervation to the heel. Immediately distal to the tarsal tunnel, the tibial nerve terminates by dividing into sensory branches, which innervate the sole of the foot. The tibial nerve innervates all the muscles in the posterior compartment of the leg. They are divided into deep and superficial compartments, including popliteus, flexor hallucis longus, flexor digitorum longus, tibialis posterior, plantaris, soleus, and gastrocnemius. In the popliteal fossa, the tibial nerve gives off cutaneous branches. These combine with branches from the common fibular nerve to form the sural nerve. This sensory nerve innervates the skin of the posterolateral side of the leg and the lateral side of the foot. The tibial nerve also supplies all the sole of the foot via three branches, the medial calcaneal branches, the medial plantar nerve, and the lateral plantar nerve.

### SUMMARY

Some embodiments relate generally to the treatment of diseases associated with autonomic dysfunction, autonomic imbalance or dysautonomia, more specifically to systems and methods of treating chronic stress, diabetes, Parkinson's disease, tremor, cardiac arrhythmias including atrial fibrillation, hypertension, heart failure, overactive bladder, urinary incontinence, fecal incontinence, inflammatory bowel diseases including Crohn's disease and ulcerative colitis, irritable bowel syndrome, gastrointestinal disorders, rheumatoid arthritis, migraine, pain, clinical depression, post-traumatic stress disorder, generalized anxiety disorder, neurocardiogenic syncope, and mast cell disorders, through noninvasive peripheral nerve neuromodulation (e.g., neurostimulation) regulated by one or more measured biological signals.

In some embodiments, a system can comprise, not comprise, consist essentially of, or consist of any number of features as disclosed herein.

In some embodiments, a method can comprise, not comprise, consist essentially of, or consist of any number of features as disclosed herein.

In several embodiments, the embodiments described herein that selectively target one or more peripheral nerves and/or that coordinate neuromodulation (e.g., neurostimulation) of multiple peripheral nerves with one or more features of a rhythmic biological signal can include one or more of the following advantages: greater therapeutic benefit with less discomfort; less current use (e.g., less power and improved battery life) with non-continuous stimulation; and increased likelihood of patient compliance due to the foregoing. In several embodiments, utilization of a neurostimulation system with a user signaling device integrated within, or separate from other components of the neurostimulation system and configured to provide instructional feedback to a user to guide voluntarily controlled biological rhythmic processes can be advantageous in that they do not necessarily require (in other words, do not comprise) any sensors/device configured to measure respiratory activity. In several embodiments, the embodiments described herein that include multiple peripheral nerve stimulation can promote sympathovagal balance with at least one peripheral nerve modulating the sympathetic nervous system and at least one peripheral nerve modulating the parasympathetic nervous system can advantageously have the ability to selectively modulate either sympathetic and/or parasympathetic arms of the autonomic nervous system in response to detected sympathetic and/or parasympathetic overactivity. In several embodiments, peripheral nerve stimulation can advantageously have synergistic effects when combined with pharmacotherapy, including but not limited to anti-depressants including tricylic antidepressants, selective serotonin reuptake inhibitors, and MAO inhibitors. The effects can include enhanced response to therapy, a lesser dose of tricylic antidepressants, selective serotonin reuptake inhibitors, and MAO inhibitors needed to achieve the effects and thus lower adverse reactions, and the like. Combination therapy can be beneficial in some embodiments to reduce the time it takes to achieve a therapeutic effect (e.g., by at least 10%, 25%, 50% or more, or overlapping ranges therein) or lengthens the therapeutic effect (by e.g., by at least 10%, 20%, 40% or more, or overlapping ranges therein), or improve the overall benefit (e.g., larger reduction in mood disorder symptom magnitude or frequency).

In some embodiments, systems and methods are not configured to be placed within, or stimulate the inner ear/ear canal. In some embodiments, a system is not configured to stimulate the trigeminal nerve (and methods do not stimulate the trigeminal nerve). In some embodiments, a system is not configured to be placed on the forehead, other parts of the head, and/or the neck (and methods do not involve placement of a stimulation component on the forehead, other parts of the head, and/or the neck). In some embodiments, a system does not include self-adhesive electrodes. In some embodiments, electrodes of the system are not magnetically connected to the stimulation device. In some embodiments, systems and methods deliver only electrical energy, such as only transcutaneous electrical energy, and do not involve any one or more of: magnetic energy (e.g., including transcranial magnetic stimulation (TMS)), ultrasound energy, RF energy, microwave energy, and/or thermal energy). In some embodiments, a system does not include any implanted components. In some embodiments, systems are not configured for placement on the upper arm (and methods do not involve placement of a stimulation component on the upper arm). In some embodiments, systems and methods can modulate one or more nerves that are not associated with the vagus nerve, either alone or together with one or more nerves that are associated with the vagus nerve. However, some embodiments can include any number of the foregoing features of this paragraph.

In some embodiments, the systems and methods are configured to treat mood disorder symptoms, but are not configured to, or do not treat tremors or a movement disorder, including but not limited to Parkinson's disease. In some of the embodiments described herein, one, several or all of the following features are not included: (i) sensors configured to assess patient motion and/or collect motion data, (ii) accelerometers, gyroscopes, magnetometers, inertial measurement units. and (iii) EMG or other muscle sensors. In some embodiments, systems and methods are not configured for, or are not placed on the upper arm and/or are not configured for neuromodulation on the skin surface of the forehead. In some embodiments, systems and methods are not configured to, or do not modulate descending (e.g., efferent) nerve pathways, and only modulate ascending (e.g., afferent) nerve pathways. In some embodiments, systems and methods are not configured to, or do not modulate nerves only on the ventral side of the wrist. In some embodiments, systems and methods do not include any implantable components. In some embodiments, systems and methods are not configured for percutaneous or subcutaneous stimulation, and are only configured for transcutaneous neuromodulation. In some embodiments, systems and methods are not configured for, or do not treat cardiac disorders, including but not limited to hypertension, angina pectoris, ischemia, myocardial infarction, congestive heart failure, and/or arrhythmias. In some embodiments, systems and methods are not configured for only neuromodulating, e.g., stimulating the ventral side of the wrist, rather some configurations may neuromodulate, e.g., deliver stimulation between two or more of the ventral, dorsal, and/or lateral sides of the wrist to target the medial nerve.

In some embodiments, systems and methods are not configured to, or do not stimulate any number of the following acupuncture points: the top of the head (Du 20 Baihui), the forehead (GB.14 Yangbai), behind the ear (GB.20 Fengchi), above the ear (GB.8 Shuaigu), dorsal-side of lower arm (SJ.5 Waiguan), top of the hand between the thumb and index finger (LI.4 Hegu), the toe (GB.41 Linqi), the ankle (GB.37 Guangming), top of the foot (St.44 Neiting), the foot (Liv.3 Taichong), (St.8 Touwei), the temple (GB.4 Hanyan), top of the hand between the thumb and index finger (LI.4 Hegu), the elbow (LI.11 Quchi), and the shin (St.36 Zusanli).

Biological rhythms of the human body can regulate changes or modulate activity in the brain, the nervous system, the immune system, cardiac function, sleep activity, and cognitive function. For example, the release of hormones such as melatonin, cortisol, thyroid stimulating hormone (TSH), and prolactin (PRL) are highly regulated by the circadian and sleep-wake cycles. An additional example, a consistent increase in sympathetic tone of the autonomic nervous systems has be observed in the early morning hours or later stages of the sleep cycle, which is regulated by endogenous circadian and diurnal rhythm patterns. Biological rhythm patterns can include, but are not limited to, circadian and diurnal rhythms, ultradian rhythms, infradian rhythms, brain or neuronal rhythms (i.e., brain oscillations), cardiac rhythms, breathing rhythms, and movement rhythms. Circadian and diurnal rhythms are endogenous rhythm pattern that cycles on a daily (approximately 24 hour) basis under normal circumstance. Examples of measurable biological signals include, waking/sleep cycle, body temperature, blood pressure, reaction time, levels of alertness, patterns of hormone secretion, and digestive functions. Ultradian rhythms are endogenous rhythm pattern that occurs on a shorter time scale than circadian rhythms. Examples include feeding and digestion patterns. Infradian rhythms are endogenous rhythm pattern that has a cycle duration longer than circadian rhythms, that is more than 24 hours per cycle. An example includes the female menstrual cycle. Brain or neuronal rhythms are oscillatory signals from neurons in the cortex that are characterized by frequency, amplitude, and location of source. Cardiac rhythms repeated, regular patterns of electromechanical activity associated with the heart that drives heart beat and blood flow. Breathing rhythms are the repeated pattern of inhalation and exhalation to drive air to the lungs for oxygen/carbon dioxide exchange in the blood. Voluntary control of breathing rhythm has effects on cardiac rhythm and brain rhythms. Movement rhythms are voluntarily and involuntarily controlled rhythmic motions such as walking, cycling, running in healthy individuals, and tremor in movement disorders, which can be driven by exogenous factors, such as caffeine or stress. In some embodiments, systems and methods do not affect or synchronize with one, two, or more of any of the aforementioned biological rhythms.

In some embodiments, disclosed herein are devices and methods for transcutaneously stimulating a plurality of peripheral nerves of a patient. The methods can include one or more of: positioning a first peripheral nerve effector proximate a first location on an extremity of the patient to stimulate a first peripheral nerve of the patient; positioning a second peripheral nerve effector proximate a second location of the patient to stimulate a second peripheral of the patient; assessing activity associated with respiration of the patient and determining the phase of a respiratory cycle of the patient from the activity associated with respiration in real-time; delivering a first electrical nerve stimulation signal transcutaneously to the first peripheral nerve effector to stimulate the first peripheral nerve sufficient to modify at least one brain or spinal cord autonomic feedback loop to modulate sympathetic nervous activity of the patient if the phase of the respiratory cycle is determined to be a predetermined portion of an inspiratory phase of the respiratory cycle; and delivering a second electrical nerve stimulation signal transcutaneously to the second peripheral nerve effector to stimulate the second peripheral nerve sufficient to modify at least one brain or spinal cord autonomic feedback loop sufficient to modulate parasympathetic activity of the patient if the phase of the respiratory cycle is determined to be a predetermined portion of an expiratory phase of the respiratory cycle. In some embodiments, the first electrical nerve stimulation signal and the second electrical nerve stimulation signal are configured to balance parasympathetic and sympathetic nervous system activity of the patient. Systems to perform these methods are also provided, including components such as a first peripheral nerve effector, a second peripheral nerve effector, and a controller configured to receive data regarding a rhythmic biological signal of the patient and determine one or more features of the rhythmic biological signal in real time. Sensors may or may not be used to for example, measure respiratory activity/cycles. In some embodiments, a vagal nerve and one or more non-vagal nerves are stimulated.

In some embodiments, a method can also include monitoring sympathetic and parasympathetic activity in the patient. Monitoring sympathetic and parasympathetic activity can include receiving data from a sensor measuring heart rate variability of the patient. Measuring heart rate variability can include determining a ratio of absolute low frequency to absolute high frequency of heart rate variability of the patient. Monitoring sympathetic and parasympathetic activity can also include receiving data from a sensor that measures at least one of electrodermal activity, thermometry, and ECG information of the patient.

A method can also include adjusting the first electrical nerve stimulation signal upon identifying abnormal sympathetic activity in the patient, and/or adjusting the second electrical nerve stimulation signal upon identifying abnormal parasympathetic activity in the patient.

In some embodiments, at least one of the first electrical nerve stimulation signal and the second electrical nerve stimulation signal comprises burst stimulation, including but not limited to theta burst stimulation.

In some embodiments, the first peripheral nerve is on an upper extremity of the patient. The first peripheral nerve could be, for example, the median nerve, the radial nerve, the ulnar nerve, the medial cutaneous nerve, the lateral cutaneous nerve, and the musculocutaneous nerve.

In some embodiments, the second peripheral nerve is on a lower extremity of the patient. The second peripheral nerve could be, for example, the tibial nerve, the saphenous nerve, the common peroneal nerve, the femoral nerve, the sacral nerve, the sciatic nerve, and the sural nerve.

Assessing activity associated with breathing could include, for example, assessing chest wall movement of the patient, assessing abdominal wall movement of the patient, assessing carbon dioxide levels associated with the patient's respiration., assessing oxygen levels associated with the patient's respiration, assessing temperature levels associated with the patient's respiration, and/or assessing sounds associated with the patient's respiration via a microphone.

In some embodiments, disclosed herein is a wearable system for transcutaneously stimulating a plurality of nerves of a patient. The system can include one or more of a first peripheral nerve effector configured to be positioned proximate a skin surface on an extremity of the patient; a second peripheral nerve effector configured to be positioned about a location of the patient; a respiratory sensor configured to sense activity relating to respiration of the patient and convert the sensed activity into a corresponding respiration sensed signal; a biomedical sensor configured to detect autonomic nervous system activity of the patient and convert the detected activity into a corresponding autonomic detection signal; and/or a controller in operable communication with the respiratory sensor and the first biomedical sensor, the controller configured to analyze the respiration detection signal and recognize a phase of a respiratory cycle of the patient in real-time. The controller can be configured to analyze the autonomic detection signal and recognize at least one of sympathetic and parasympathetic activity of the patient. The controller can also be configured to generate a first electrical nerve stimulation signal transcutaneously to the first peripheral nerve effector to stimulate a first peripheral nerve sufficient to modify at least one brain or spinal cord autonomic feedback loop upon detection of a predetermined portion of a first phase of the respiratory cycle. The controller can also be configured to generate a second electrical nerve stimulation signal transcutaneously to the second peripheral nerve effector to stimulate a second peripheral nerve associated with a parasympathetic nervous pathway of the patient to modify at least one brain or spinal cord autonomic feedback loop upon detection of a predetermined portion of a second phase of the respiratory cycle. The controller can be configured to adjust the first electrical nerve stimulation signal and the second electrical nerve stimulation signal to balance parasympathetic and sympathetic nervous system activity of the patient. Sensors may or may not be used to for example, measure respiratory activity/cycles. In some embodiments, a vagal nerve and one or more non-vagal nerves are stimulated.

In some embodiments, the first predetermined phase is an inspiratory phase of the respiratory cycle. In some embodiments, the second predetermined phase is an expiratory phase of the respiratory cycle. A respiratory sensor can be operably attached to a respiratory belt configured to be placed around the rib-cage portion of the thorax to detect respiratory movements.

In some embodiments, the respiratory sensor comprises electrodes to evaluate changes in electrical impedance across the thoracic region over the respiratory cycle.

In some embodiments, the biomedical sensor comprises pulse sensors on the extremities to measure blood pressure and derive waveforms to calculate cardiac performance.

In some embodiments, the respiratory sensor is operably attached to a nasal or oral patient interface to evaluate pulmonary activity by measuring respiratory flow and continuous expired concentration of carbon dioxide.

In some embodiments, the respiratory sensor comprises an optical transcutaneous sampling cell for measuring carbon dioxide or oxygen concentration within a blood vessel.

In some embodiments, the controller is configured to adjust the first electrical nerve stimulation signal upon identifying abnormal sympathetic activity in the patient.

In some embodiments, the controller is configured to adjust the first electrical nerve stimulation signal upon identifying abnormal parasympathetic activity in the patient.

In some embodiments, the controller is configured to adjust at least one of the first electrical stimulation signal and the second electrical nerve stimulation signal based on feedback received regarding the autonomic balance of the patient.

In some embodiments, the feedback comprises at least one of EKG, heart rate, and heart rate variability of the patient.

In some embodiments, the feedback comprises a ratio of absolute low frequency to absolute high frequency of heart rate variability of the patient.

In some embodiments, the feedback comprises electrodermal activity of the patient.

In some embodiments, disclosed herein is a method for stimulating one or more nerves of a patient, comprising one or more of assessing respiration of a patient and determining the phase of a respiratory cycle of the patient in real-time; stimulating a first peripheral nerve of the patient sufficient to modulate sympathetic nervous activity of the patient if the phase of the respiratory cycle is determined to be an inspiratory phase of the respiratory cycle; and stimulating a branch of the vagus nerve sufficient to modulate parasympathetic activity of the patient if the phase of the respiratory cycle is determined to be an expiratory phase of the respiratory cycle.

In some embodiments, the first peripheral nerve is not a branch of the vagus nerve.

In some embodiments, the first peripheral nerve is on an upper extremity of the patient.

In some embodiments, the first peripheral nerve is the median nerve.

In some embodiments, the first peripheral nerve is on a lower extremity of the patient.

In some embodiments, the first peripheral nerve is the saphenous nerve of the patient.

In some embodiments, the branch of the vagus nerve is the auricular branch of the vagus nerve, or the cervical branch of the vagus nerve.

In some embodiments, the stimulation comprises only transcutaneous stimulation, percutaneous stimulation, or implantable stimulation.

In some embodiments, the stimulation comprises electrical stimulation, vibrational stimulation, acoustic stimulation, and/or magnetic stimulation.

In some embodiments, the method is sufficient to have a therapeutic effect on cardiac dysfunction of the patient, including but not limited to one or more of hypertension, congestive heart failure, angina pectoris, an arrhythmia, atrial fibrillation, and/or supraventricular tachycardia.

In some embodiments, the method is sufficient to have a therapeutic effect on a mental health condition of the patient, including but not limited to depression, anxiety, bipolar disorder, or a psychotic disorder, such as schizophrenia for example.

In some embodiments, the method is sufficient to have a therapeutic effect to upregulate or downregulate immune function of the patient.

In some embodiments, the method is sufficient to have a therapeutic effect to improve signs or symptoms of an autoimmune disease in the patient; reduce inflammation in the patient; improve signs or symptoms of inflammatory bowel disease in the patient; reduce pain in the patient; and/or reduce signs or symptoms of migraine in the patient.

In some embodiments, disclosed herein are systems for stimulating a plurality of nerves of a patient, including one or more of: a first peripheral nerve effector configured to be positioned on a patient's skin on an extremity of the patient; a second peripheral nerve effector configured to be positioned to stimulate a branch of the vagus nerve of the patient; a respiratory sensor configured to detect activity relating to respiration of the patient and convert the detected activity into a corresponding respiration detection signal; a controller in operable communication with the respiratory sensor, the controller configured to analyze the respiration detection signal and recognize a portion of a phase of a respiratory cycle of the patient in real-time.

In some embodiments, the controller is configured to generate a first nerve stimulation signal transcutaneously to the first peripheral nerve effector to stimulate a first peripheral nerve sufficient to modify at least one brain or spinal cord autonomic feedback loop upon detection of a predetermined portion of an inspiratory phase of the respiratory cycle.

In some embodiments, the controller is configured to generate a second nerve stimulation signal transcutaneously to the second peripheral nerve effector to stimulate a second peripheral nerve associated with a parasympathetic nervous pathway of the patient to modify at least one brain or spinal cord autonomic feedback loop upon detection of a predetermined portion of an expiratory phase of the respiratory cycle.

In some embodiments, the respiratory sensor is operably attached to a respiratory belt configured to be placed around the rib-cage portion of the thorax to detect respiratory movements.

In some embodiments, the respiratory sensor comprises electrodes to evaluate changes in electrical impedance across the thoracic region over the respiratory cycle.

In some embodiments, the biomedical sensor comprises pulse sensors on the extremities to measure blood pressure and derive waveforms to calculate cardiac performance.

In some embodiments, the respiratory sensor is operably attached to a nasal cannula to evaluate pulmonary activity by measuring respiratory flow and continuous expired concentration of carbon dioxide.

In some embodiments, the respiratory sensor comprises an optical transcutaneous sampling cell for measuring carbon dioxide concentration within a blood vessel.

In some embodiments, the controller is configured to adjust the first nerve stimulation signal upon identifying abnormal sympathetic activity in the patient.

In some embodiments, the controller is configured to adjust the first nerve stimulation signal upon identifying abnormal parasympathetic activity in the patient.

In some embodiments, the controller is configured to adjust at least one of the first nerve stimulation signal and the second nerve stimulation signal based on feedback received regarding the autonomic balance of the patient.

In some embodiments, the feedback comprises measured at least one of EKG, heart rate, and heart rate variability of the patient.

In some embodiments, the feedback comprises a ratio of absolute low frequency to absolute high frequency of heart rate variability of the patient.

In some embodiments, the feedback comprises electrodermal activity of the patient.

In some embodiments, disclosed herein is a method of transcutaneously stimulating one or more peripheral nerves of a patient with a wearable neurostimulation device regulated by a measured rhythmic biological signal to treat a patient, comprising any number of: positioning a first peripheral nerve effector proximate a skin surface on a first ear of the patient to stimulate a first peripheral nerve of the patient; positioning a second peripheral nerve effector proximate a skin surface proximate a first or a second ear of the patient to stimulate a second peripheral nerve of the patient; measuring a rhythmic biological signal of the patient and determining one or more features of the measured rhythmic biological signal of the patient in real-time; delivering a first electrical nerve stimulation signal transcutaneously to the first peripheral nerve effector to stimulate the first peripheral nerve sufficient to modify a first neural circuit to modulate nervous system activity of the patient at least in part based on the measured rhythmic biological signal; and/or delivering a second electrical nerve stimulation signal transcutaneously to the second peripheral nerve effector to stimulate the second peripheral nerve sufficient to modify the first or a second neural feedback loop to modulate nervous system activity of the patient at least in part based on the measured rhythmic biological signal.

In some embodiments, the first peripheral nerve is the auricular vagus nerve, and the second peripheral nerve is a nerve other than the auricular vagus nerve, and/or the second electrical nerve stimulation signal is offset at a preselected time interval from the first electrical stimulation signal.

In some embodiments, the features of the measured rhythmic biological signal comprise the phase, frequency, magnitude, or timing of the measured rhythmic biological signal.

In some embodiments, the method also includes adjusting one or more of the first electrical stimulation signal and the second electrical stimulation signal based on the one or more features of the measured rhythmic biological signal.

In some embodiments, the method also includes adjusting the first electrical stimulation signal and the second electrical stimulation signal based on the one or more features of the measured rhythmic biological signal.

In some embodiments, the method also includes adjusting the first electrical stimulation signal based on a first feature of the measured rhythmic biological signal, and adjusting the second electrical stimulation signal based on a second feature of the measured rhythmic biological signal.

In some embodiments, the first peripheral nerve and the second peripheral nerve are located on the first ear of the patient.

In some embodiments, the first peripheral nerve is located on the first ear of the patient and the second peripheral nerve is located on the second ear of the patient.

In some embodiments, the method also includes monitoring sympathetic and parasympathetic activity in the patient.

In some embodiments, monitoring sympathetic and parasympathetic activity comprises receiving data from a sensor that measures heart rate variability.

In some embodiments, monitoring sympathetic and parasympathetic activity comprises receiving data from a sensor that electrodermal activity.

In some embodiments, measuring heart rate variability also comprises determining a ratio of absolute low frequency to absolute high frequency of heart rate variability of the patient.

In some embodiments, monitoring sympathetic and parasympathetic activity comprises receiving data from a sensor that measures at least one of electrodermal activity, heart rate variability, thermometry, pupillometry and electrocardiogram information of the patient.

In some embodiments, the method further comprises adjusting the first electrical nerve stimulation signal upon identifying abnormal sympathetic activity in the patient.

In some embodiments, the method further comprises adjusting the second electrical nerve stimulation signal upon identifying abnormal parasympathetic activity in the patient.

In some embodiments, at least one of the first electrical nerve stimulation signal and the second electrical nerve stimulation signal comprises burst stimulation.

In some embodiments, the burst stimulation comprises theta burst stimulation.

In some embodiments, the theta burst stimulation is preceded by a priming stimulation with a waiting period between priming and theta burst stimulation between 1 - 30 minutes.

In some embodiments, the measured rhythmic biological signal is one selected from the group consisting of a breathing cycle, cardiac rhythm cycle, sympathetic tonic rhythmic discharge, sleep-wake cycle, menstrual cycle, and cortical brain rhythm patterns.

In some embodiments, the method further comprises neuromodulating one or more additional nerves selected from the group consisting of the trigeminal nerve, greater auricular nerve, auriculotemporal nerve, and the lesser occipital nerve.

In some embodiments, measuring the breathing cycle comprises assessing at least one of chest wall and abdominal wall movement of the patient.

In some embodiments, measuring the breathing cycle comprises assessing carbon dioxide levels associated with the patient's breathing.

In some embodiments, measuring the breathing cycle comprises assessing oxygen levels associated with the patient's breathing.

In some embodiments, measuring the breathing cycle comprises assessing temperature levels associated with the patient's breathing.

In some embodiments, measuring the breathing cycle comprises assessing sounds associated with the patient's breathing via a microphone.

In some embodiments, measuring the cardiac rhythm cycle comprises assessing heart rate.

In some embodiments, measuring the cardiac rhythm cycle comprises assessing electrocardiogram data.

In some embodiments, measuring the cardiac rhythm cycle comprises assessing photoplethysmography.

In some embodiments, measuring cortical brain rhythm patterns comprises assessing electroencephalogram data.

In some embodiments, the method includes treating a mood disorder, such as, for example, one or more of depression, bipolar disorder, anxiety disorder, mania, and schizoaffective disorder.

In some embodiments, the method includes treating a cardiovascular disorder, such as, for example, one or more of hypertension, coronary artery disease, angina pectoris, congestive heart failure, a cardiac dysrhythmia, and a cardiac dyssynchrony.

In some embodiments, the method includes treating an inflammatory disorder.

In some embodiments, the method includes treating a neurologic disorder, such as, for example, one or more of Parkinson's disease, a sleep disorder, and another neurologic disorder.

In some embodiments, the method includes treating a urologic disorder.

In some embodiments, the method also includes stimulating the first peripheral nerve during a first phase of the rhythmic biologic signal, and stimulating the second peripheral nerve during a second phase of the rhythmic biological signal.

In some embodiments, the first peripheral nerve is associated with the parasympathetic nervous system, and the second peripheral nerve is associated with the sympathetic nervous system.

In some embodiments, the first phase is expiration, and the second phase is inspiration.

In some embodiments, disclosed herein is a wearable neurostimulation system for transcutaneously stimulating peripheral nerves of a patient regulated by a rhythmic biological signal, comprising any number of: a first peripheral nerve effector configured to be positioned proximate a first location on the skin surface on a first ear of the patient; a second peripheral nerve effector configured to be positioned at a second location of the patient proximate the first ear or a second ear of the patient; a first biomedical sensor configured to detect activity relating to a rhythmic biological signal of the patient and convert the detected activity into a corresponding rhythmic detection signal; and/or a controller in operable communication with the first biomedical sensor, the controller configured to analyze a rhythmic biological signal and recognize one or more features of the rhythmic biological signal of the patient in real-time.

In some embodiments, the controller is configured to generate a first electrical nerve stimulation signal transcutaneously to the first peripheral nerve effector to stimulate a first peripheral nerve sufficient to modify a first neural feedback loop upon detection of a predetermined feature of the rhythmic biological signal, wherein the first peripheral nerve is the auricular branch of the vagus nerve.

In some embodiments, the controller is configured to generate a second electrical nerve stimulation signal transcutaneously to the second peripheral nerve effector to stimulate a second peripheral nerve other than the auricular branch of the vagus nerve and associated to modify the first or a second neural feedback loop upon detection of the first or a second predetermined feature of the rhythmic biological signal,

wherein the second electrical nerve stimulation signal is offset at a preselected time interval from the first electrical stimulation signal.

In some embodiments, the one or more features of the rhythmic biological signal comprise the phase, frequency, magnitude, or timing of the measured rhythmic biological signal.

In some embodiments, the controller is further configured to adjust one or more of the first electrical stimulation signal and the second electrical stimulation signal based on the one or more features of the measured rhythmic biological signal.

In some embodiments, the controller is further configured to adjust the first electrical stimulation signal and the second electrical stimulation signal based on the one or more features of the measured rhythmic biological signal.

In some embodiments, the controller is further configured to adjust the first electrical stimulation signal based on a first feature of the measured rhythmic biological signal, and adjust the second electrical stimulation signal based on a second feature of the measured rhythmic biological signal.

In some embodiments, the first peripheral nerve is associated with a sympathetic nervous pathway of the patient and the second peripheral nerve is associated with a parasympathetic nervous pathway of the patient.

In some embodiments, the rhythmic biological signal is a breathing cycle, cardiac rhythm cycle, sympathetic tonic rhythmic discharge, sleep-wake cycle, menstrual cycle, or cortical brain rhythm patterns.

In some embodiments, the rhythmic biological signal is the breathing cycle and the first predetermined phase is an exhalation phase of the breathing cycle.

In some embodiments, the rhythmic biological signal is the breathing cycle and the second predetermined phase is an inhalation phase of the breathing cycle.

In some embodiments, the rhythmic biological signal is the breathing cycle and the first biomedical sensor is operably attached to an effort belt configured to be placed around the rib-cage portion of the torso to detect breathing movements.

In some embodiments, the rhythmic biological signal is the breathing cycle and the first biomedical sensor comprises electrodes to evaluate changes in electrical impedance across the torso region over the breathing cycle.

In some embodiments, the rhythmic biological signal is the breathing cycle and the first biomedical sensor is operably attached to a nasal or oral patient interface to evaluate pulmonary activity by measuring respiratory flow and continuous expired concentration of carbon dioxide.

In some embodiments, the rhythmic biological signal is the breathing cycle and the first biomedical sensor comprises an optical transcutaneous sampling cell for measuring carbon dioxide or oxygen concentration within a blood vessel.

In some embodiments, the system further comprises a second biomedical sensor.

In some embodiments, the second biomedical sensor comprises pulse sensors on the extremities to measure blood pressure and derive waveforms to calculate cardiac performance.

In some embodiments, the second biomedical sensor comprises electrically conductive sensors on the chest, torso, upper extremities, or in the ear to detect electrocardiogram, cardiac activity, heart rate, or heart rate variability.

In some embodiments, the second biomedical sensor comprises photoplethysmography sensors on the upper extremities or in the ear to detect cardiac activity, heart rate, or heart rate variability.

In some embodiments, the controller is configured to adjust the first electrical nerve stimulation signal upon identifying abnormal sympathetic activity in the patient.

In some embodiments, the controller is configured to adjust the first electrical nerve stimulation signal upon identifying abnormal parasympathetic activity in the patient.

In some embodiments, the controller is configured to adjust at least one of the first electrical stimulation signal and the second electrical nerve stimulation signal based on feedback received regarding the autonomic balance of the patient.

In some embodiments, disclosed herein is a method of transcutaneously stimulating a one or more peripheral nerves of a patient with a wearable neurostimulation device regulated by a measured rhythmic biological signal to treat a patient, comprising any number of: positioning a first peripheral nerve effector proximate a skin surface on a first ear of the patient to stimulate a first peripheral nerve of the patient; signaling the patient to perform a first voluntary activity associated with a biological process for a first specified duration; signaling the patient to perform a second voluntary activity associated with the biological process for a second specified duration; and/or delivering a first electrical nerve stimulation signal transcutaneously to the first peripheral nerve effector to stimulate the first peripheral nerve sufficient to modify a first neural circuit to modulate nervous system activity of the patient during only at least part of the first specified duration,

In some embodiments, the first peripheral nerve is the auricular vagus nerve.

In some embodiments, the method further comprises: positioning a second peripheral nerve effector proximate a skin surface of the patient to stimulate a second peripheral nerve of the patient; and/or delivering a second a first electrical nerve stimulation signal transcutaneously to the first peripheral nerve effector to stimulate the second peripheral nerve sufficient to modify a first neural circuit to modulate nervous system activity of the patient during only at least part of the second specified duration.

In some embodiments, the second peripheral nerve is a nerve on an ear of the patient.

In some embodiments, the second peripheral nerve is on a contralateral side of the patient with respect to the first peripheral nerve.

In some embodiments, the second peripheral nerve is on an ipsilateral side of the patient with respect to the first peripheral nerve.

In some embodiments, the method does not comprise sensing any features of the biological process.

In some embodiments, signaling the patient to perform a first voluntary activity comprises displaying information on a screen.

In some embodiments, signaling the patient to perform a first voluntary activity comprises providing audio instructions.

In some embodiments, signaling the patient to perform a first voluntary activity comprises providing tactile cues.

In some embodiments, the biological process comprises respiration.

In some embodiments, the first voluntary activity comprises a first phase of respiration.

In some embodiments, the first voluntary activity comprises a second phase of respiration.

In some embodiments, disclosed herein is a system for transcutaneously stimulating a one or more peripheral nerves of a patient with a wearable neurostimulation device regulated by a measured rhythmic biological signal to treat a patient, comprising any number of: a first peripheral nerve effector configured to be positioned proximate a skin surface on a first ear of the patient to stimulate a first peripheral nerve of the patient; a controller configured to signal the patient to perform a first voluntary activity associated with a biological process for a first specified duration; and to perform a second voluntary activity associated with the biological process for a second specified duration; and/or deliver a first electrical nerve stimulation signal transcutaneously to the first peripheral nerve effector to stimulate the first peripheral nerve sufficient to modify a first neural circuit to modulate nervous system activity of the patient during only at least part of the first specified duration,

In some embodiments, the first peripheral nerve is the auricular vagus nerve.

In some embodiments, the system further comprises a second peripheral nerve effector proximate a skin surface of the patient to stimulate a second peripheral nerve of the patient; and the controller is further configured to deliver a second a first electrical nerve stimulation signal transcutaneously to the first peripheral nerve effector to stimulate the second peripheral nerve sufficient to modify a first neural circuit to modulate nervous system activity of the patient during only at least part of the second specified duration.

In some embodiments, the second peripheral nerve is a nerve on an ear of the patient.

In some embodiments, the second peripheral nerve is on a contralateral side of the patient with respect to the first peripheral nerve.

In some embodiments, the second peripheral nerve is on an ipsilateral side of the patient with respect to the first peripheral nerve.

In some embodiments, the system does not comprise any sensors configured to sense any features of the biological process.

In some embodiments, the controller is configured to signal the patient to perform the first voluntary activity by activating a display.

In some embodiments, the controller is configured to signal the patient to perform the first voluntary activity by sending audio instructions.

In some embodiments, the controller is configured to signal the patient to perform the first voluntary activity by sending tactile cues.

In some embodiments, the biological process comprises respiration.

In some embodiments, the first voluntary activity comprises a first phase of respiration.

In some embodiments, the first voluntary activity comprises a second phase of respiration.

In some embodiments, disclosed herein is a method of transcutaneously neuromodulating a one or more peripheral nerves of a patient with a wearable neuromodulation device regulated by a measured rhythmic biological signal to treat a patient, comprising any number of: positioning a first peripheral nerve effector proximate a skin surface on a first location of the patient to neuromodulate a first peripheral nerve of the patient; positioning a second peripheral nerve effector proximate a skin surface on a second location of the patient to neuromodulate a second peripheral nerve of the patient; measuring a rhythmic biological signal of the patient and determining one or more features of the measured rhythmic biological signal of the patient in real-time; delivering a first electrical nerve neuromodulation signal transcutaneously to the first peripheral nerve effector to neuromodulate the first peripheral nerve sufficient to modify a first neural circuit to modulate nervous system activity of the patient at least in part based on the measured rhythmic biological signal; and/or delivering a second electrical nerve stimulation signal transcutaneously to the second peripheral nerve effector to neuromodulate the second peripheral nerve sufficient to modify the first or a second neural feedback loop to modulate nervous system activity of the patient at least in part based on the measured rhythmic biological signal.

In some embodiments, the features of the measured rhythmic biological signal comprise the phase, frequency, magnitude, or timing of the measured rhythmic biological signal.

In some embodiments, the method further comprises adjusting one or more of the first electrical neuromodulation signal and the second electrical neuromodulation signal based on the one or more features of the measured rhythmic biological signal.

In some embodiments, the method further comprises adjusting the first electrical neuromodulation signal and the second electrical neuromodulation signal based on the one or more features of the measured rhythmic biological signal.

In some embodiments, the method further comprises adjusting the first electrical neuromodulation signal based on a first feature of the measured rhythmic biological signal, and adjusting the second electrical neuromodulation signal based on a second feature of the measured rhythmic biological signal.

In some embodiments, the first location is on a first ear of the patient.

In some embodiments, the second location is on the first ear of the patient.

In some embodiments, the second location is on a second ear of the patient.

In some embodiments, the second location is not on the first ear of the patient.

In some embodiments, disclosed herein is a wearable neuromodulation system for transcutaneously stimulating peripheral nerves of a patient regulated by a rhythmic biological signal, comprising any number of: a first peripheral nerve effector configured to be positioned proximate a first location on the skin surface on a first ear of the patient; a second peripheral nerve effector configured to be positioned at a second location of the patient; a first biomedical sensor configured to detect activity relating to a rhythmic biological signal of the patient and convert the detected activity into a corresponding rhythmic detection signal; and/or a controller in operable communication with the first biomedical sensor, the controller configured to analyze the rhythmic detection signal and recognize one or more features of the rhythmic biological signal of the patient in real-time.

In some embodiments, the controller is configured to generate a first electrical nerve neuromodulation signal transcutaneously to the first peripheral nerve effector to stimulate a first peripheral nerve sufficient to modify a first neural feedback loop upon detection of a predetermined feature of the rhythmic biological signal, and the controller is configured to generate a second electrical nerve stimulation signal transcutaneously to the second peripheral nerve effector to neuromodulation a second peripheral nerve associated to modify the first or a second neural feedback loop.

In some embodiments, the one or more features of the rhythmic biological signal comprise the phase, frequency, magnitude, or timing of the measured rhythmic biological signal.

In some embodiments, the controller is further configured to adjust one or more of the first electrical neuromodulation signal and the second electrical neuromodulation signal based on the one or more features of the measured rhythmic biological signal.

In some embodiments, the controller is further configured to adjust the first electrical neuromodulation signal and the second electrical neuromodulation signal based on the one or more features of the measured rhythmic biological signal.

In some embodiments, the controller is further configured to adjust the first electrical neuromodulation signal based on a first feature of the measured rhythmic biological signal, and adjust the second electrical neuromodulation signal based on a second feature of the measured rhythmic biological signal.

In some embodiments, a method of transcutaneously neuromodulating one or more nerves of a patient with a neuromodulation device, comprises any number of: positioning a first nerve effector proximate a first skin surface of the patient to neuromodulate a first nerve of the patient; positioning a second nerve effector proximate a second skin surface of the patient to neuromodulate a second peripheral nerve of the patient; measuring a rhythmic biological signal of the patient and determining one or more features of the measured rhythmic biological signal of the patient in real-time; delivering a first electromagnetic neuromodulation signal transcutaneously to the first peripheral nerve effector to neuromodulate the first peripheral nerve sufficient to modify a first neural circuit to modulate nervous system activity of the patient at least in part based on the measured rhythmic biological signal; and/or delivering a second electromagnetic neuromodulation signal transcutaneously to the second peripheral nerve effector to neuromodulate the second peripheral nerve sufficient to modify the first or a second neural feedback loop to modulate nervous system activity of the patient at least in part based on the measured rhythmic biological signal, wherein the first peripheral nerve is a vagus nerve, and the second peripheral nerve is a nerve other than the vagus nerve, wherein the second electromagnetic nerve neuromodulation signal is offset at a preselected time interval from the first electromagnetic neuromodulation signal.

In some embodiments, the first electromagnetic neuromodulation signal comprises an electrical neuromodulation signal.

In some embodiments, the first electromagnetic neuromodulation signal comprises a vibrational neuromodulation signal.

In some embodiments, disclosed herein is a wearable neuromodulation system for transcutaneously neuromodulating peripheral nerves of a patient regulated by a rhythmic biological signal, comprising any number of: a first peripheral nerve effector configured to be positioned proximate a first location on the skin surface on a first ear of the patient; a second peripheral nerve effector configured to be positioned at a second location of the patient; a first biomedical sensor configured to detect activity relating to a rhythmic biological signal of the patient and convert the detected activity into a corresponding rhythmic detection signal; a controller in operable communication with the first biomedical sensor, the controller configured to analyze the rhythmic detection signal and recognize one or more features of the rhythmic biological signal of the patient in real-time, wherein the controller is configured to generate a first electromagnetic nerve neuromodulation signal transcutaneously to the first peripheral nerve effector to stimulate a first peripheral nerve sufficient to modify a first neural feedback loop upon detection of a predetermined feature of the rhythmic biological signal, wherein the controller is configured to generate a second electrical nerve stimulation signal transcutaneously to the second peripheral nerve effector to neuromodulation a second peripheral nerve associated to modify the first or a second neural feedback loop.

In some embodiments, the one or more features of the rhythmic biological signal comprise the phase, frequency, magnitude, or timing of the measured rhythmic biological signal.

In some embodiments, the controller is further configured to adjust one or more of the first neuromodulation signal and the second neuromodulation signal based on the one or more features of the measured rhythmic biological signal.

In some embodiments, disclosed herein is a method of transcutaneously stimulating one or more peripheral nerves of a patient with a wearable neurostimulation device regulated by a measured rhythmic biological signal to treat a patient, comprising any number of: positioning a first peripheral nerve effector proximate a skin surface on a first ear of the patient to stimulate a first peripheral nerve of the patient; positioning a second peripheral nerve effector proximate a skin surface proximate a first or a second ear of the patient to stimulate a second peripheral nerve of the patient; measuring a rhythmic biological signal of the patient and determining one or more features of the measured rhythmic biological signal of the patient in real-time; delivering a first electrical nerve stimulation signal transcutaneously to the first peripheral nerve effector to stimulate the first peripheral nerve sufficient to modify a first neural circuit to modulate nervous system activity of the patient at least in part based on the measured rhythmic biological signal; and/or delivering a second electrical nerve stimulation signal transcutaneously to the second peripheral nerve effector to stimulate the second peripheral nerve sufficient to modify the first or a second neural feedback loop to modulate nervous system activity of the patient at least in part based on the measured rhythmic biological signal, and adjusting one or more of the first electrical stimulation signal and the second electrical stimulation signal based on the one or more features of the measured rhythmic biological signal, wherein the first peripheral nerve is the auricular vagus nerve, and the second peripheral nerve is a nerve other than the auricular vagus nerve and selected from the group consisting of the trigeminal nerve, greater auricular nerve, auriculotemporal nerve, and the lesser occipital nerve, wherein the second electrical nerve stimulation signal is offset at a preselected time interval from the first electrical stimulation signal, wherein the rhythmic biological signal is a respiratory signal, wherein the features of the measured rhythmic biological signal comprise the phase, frequency, magnitude, or timing of the measured rhythmic biological signal, and wherein at least one of the first electrical nerve stimulation signal and the second electrical nerve stimulation signal comprises burst stimulation.

In some embodiments, the method also comprises stimulating the first peripheral nerve during a first phase of the rhythmic biologic signal, and stimulating the second peripheral nerve during a second phase of the rhythmic biological signal.

In some embodiments, the first peripheral nerve is associated with the parasympathetic nervous system, and the second peripheral nerve is associated with the sympathetic nervous system.

In some embodiments, the first phase is expiration, and the second phase is inspiration.

In some embodiments, disclosed herein is a wearable neurostimulation system for transcutaneously stimulating peripheral nerves of a patient regulated by a rhythmic biological signal, comprising: a first peripheral nerve effector configured to be positioned proximate a first location on the skin surface on a first ear of the patient; a second peripheral nerve effector configured to be positioned at a second location of the patient proximate the first ear or a second ear of the patient; a first biomedical sensor configured to detect activity relating to a rhythmic biological signal of the patient and convert the detected activity into a corresponding rhythmic detection signal; a controller in operable communication with the first biomedical sensor, the controller configured to analyze the rhythmic biological signal and recognize one or more features of the rhythmic biological signal of the patient in real-time, wherein the controller is configured to generate a first electrical nerve stimulation signal transcutaneously to the first peripheral nerve effector to stimulate a first peripheral nerve sufficient to modify a first neural feedback loop upon detection of a predetermined feature of the rhythmic biological signal, wherein the first peripheral nerve is the auricular branch of the vagus nerve, wherein the controller is configured to generate a second electrical nerve stimulation signal transcutaneously to the second peripheral nerve effector to stimulate a second peripheral nerve other than the auricular branch of the vagus nerve and associated to modify the first or a second neural feedback loop upon detection of the first or a second predetermined feature of the rhythmic biological signal, wherein the second electrical nerve stimulation signal is offset at a preselected time interval from the first electrical stimulation signal, wherein the rhythmic biological signal is a respiratory signal, wherein the features of the measured rhythmic biological signal comprise the phase, frequency, magnitude, or timing of the measured rhythmic biological signal, and wherein at least one of the first electrical nerve stimulation signal and the second electrical nerve stimulation signal comprises burst stimulation.

In some embodiments, the rhythmic biological signal is the breathing cycle and the first predetermined phase is an exhalation phase of the breathing cycle.

In some embodiments, the rhythmic biological signal is the breathing cycle and the second predetermined phase is an inhalation phase of the breathing cycle.

In some embodiments, disclosed herein is a wearable neurostimulation system for transcutaneously stimulating peripheral nerves of a patient regulated by a rhythmic biological signal, comprising any number of: a first peripheral nerve effector configured to be positioned proximate a first location on the skin surface on a first ear of the patient; a second peripheral nerve effector configured to be positioned at a second location of the patient proximate the first ear or a second ear of the patient; a first biomedical sensor configured to detect activity relating to a rhythmic biological signal of the patient and convert the detected activity into a corresponding rhythmic detection signal; and/or a controller in operable communication with the first biomedical sensor, the controller configured to analyze the rhythmic biological signal and recognize one or more features of the rhythmic biological signal of the patient in real-time, wherein the controller is configured to generate a first electrical nerve stimulation signal transcutaneously to the first peripheral nerve effector to stimulate a first peripheral nerve sufficient to modify a first neural feedback loop upon detection of a predetermined feature of the rhythmic biological signal, wherein the first peripheral nerve is the auricular branch of the vagus nerve of a first ear, wherein the controller is configured to generate a second electrical nerve stimulation signal transcutaneously to the second peripheral nerve effector to stimulate a second peripheral nerve which is the auricular branch of the vagus nerve of a second ear and associated to modify the first or a second neural feedback loop upon detection of the first or a second predetermined feature of the rhythmic biological signal, wherein the second electrical nerve stimulation signal is offset at a preselected time interval from the first electrical stimulation signal, wherein the rhythmic biological signal is a respiratory signal, wherein the features of the measured rhythmic biological signal comprise the phase, frequency, magnitude, or timing of the measured rhythmic biological signal, and wherein at least one of the first electrical nerve stimulation signal and the second electrical nerve stimulation signal comprises burst stimulation.

In some embodiments, the rhythmic biological signal is the breathing cycle and the first predetermined phase is an exhalation phase of the breathing cycle.

In some embodiments, the rhythmic biological signal is the breathing cycle and the second predetermined phase is an inhalation phase of the breathing cycle.

### BRIEF DESCRIPTION OF THE DRAWINGS

Non-limiting novel features of the invention are set forth with particularity in the claims that follow. A better understanding of the features and advantages of some embodiments of the present invention will be obtained by reference to the following detailed description that sets forth illustrative embodiments, in which the principles of the invention are utilized, and the accompanying drawings.

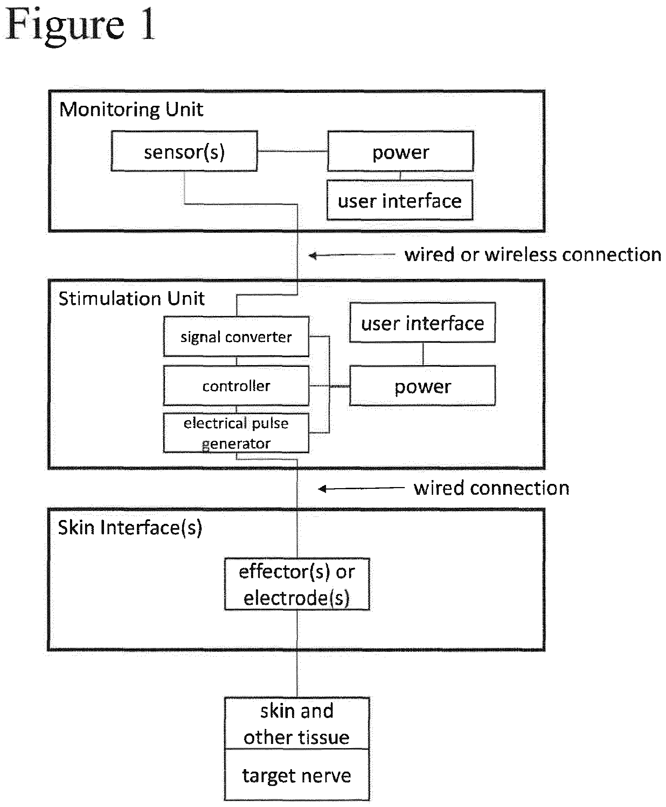

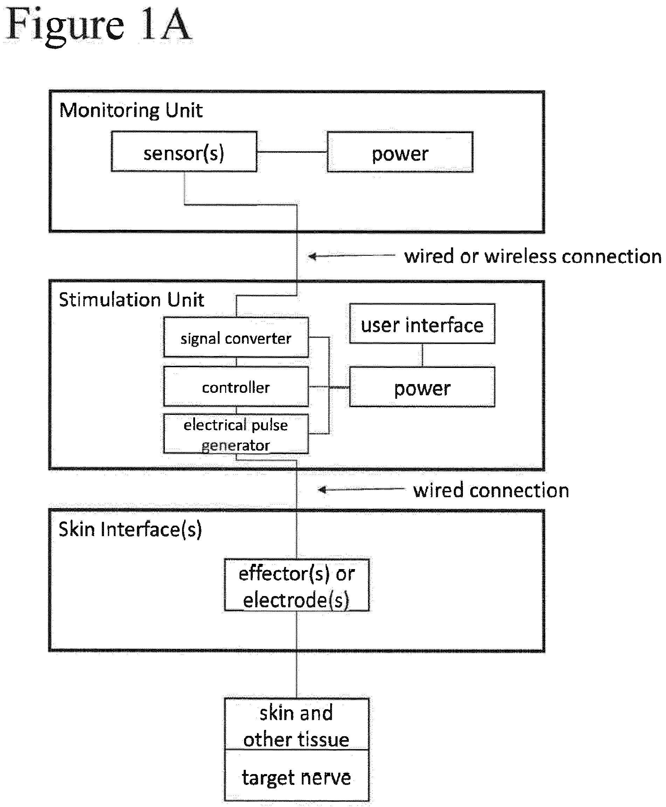

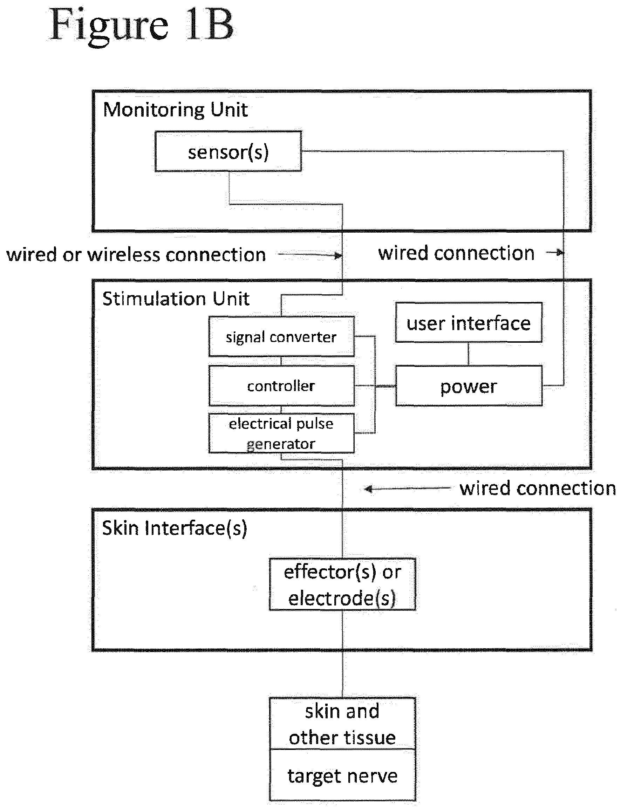

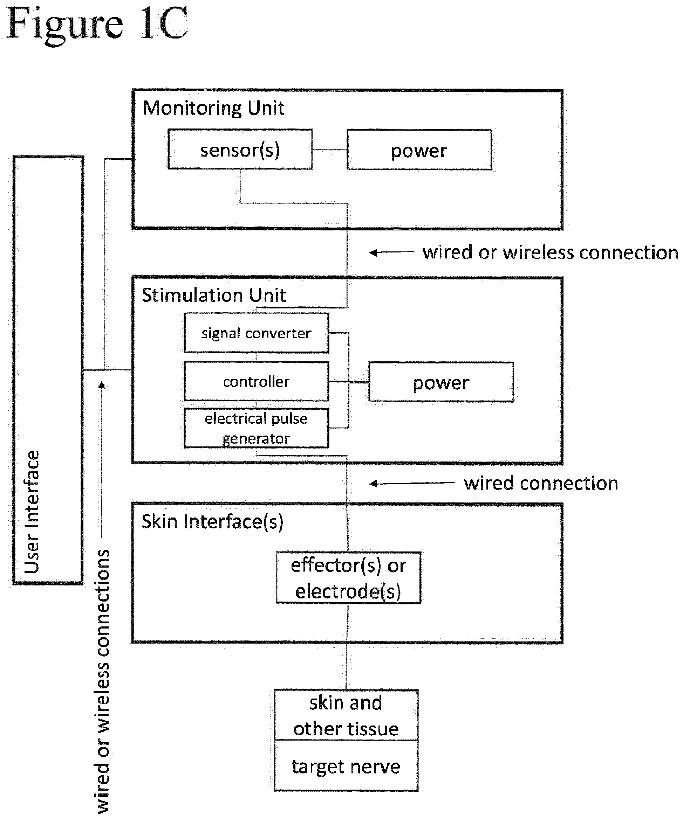

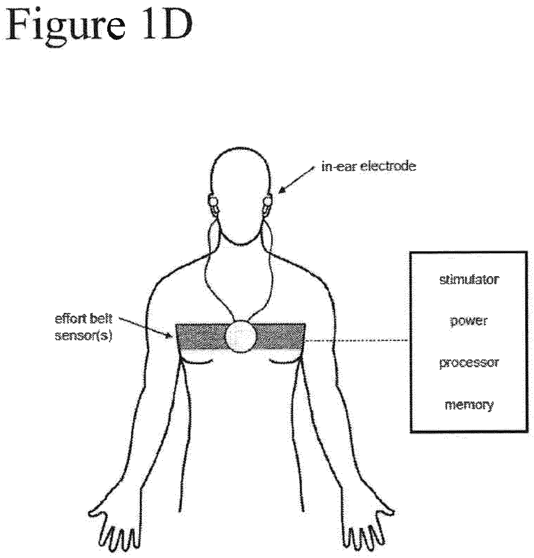

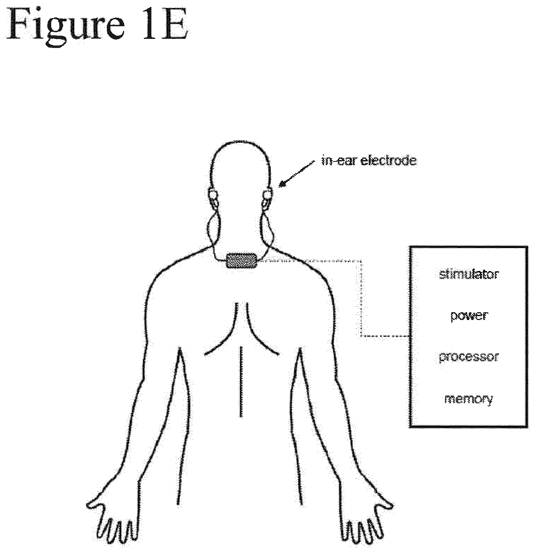

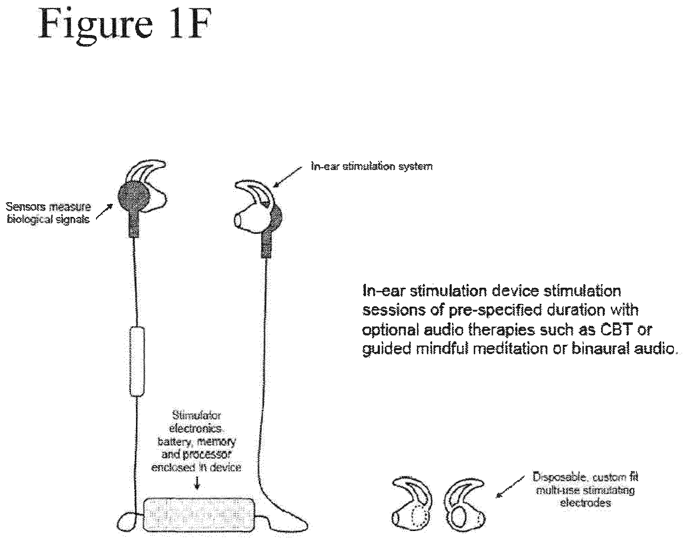

FIGS. 1-1C illustrate various block diagrams of embodiments of a device and system that provides peripheral nerve stimulation, targeting individual nerves, to a disease and uses sensors to detect a biological signal and/or assess autonomic imbalance to modify one or more parameters of the delivered stimulation or regulate when stimulation is applied. FIG. 1D schematically illustrates a stimulation system configured for bilateral in-ear stimulation including a pair of in-ear electrodes operably connected to an effort belt sensor attached to the chest or torso with a strap, along with an enclosure housing electronic components. FIG. 1E schematically illustrates a stimulation system configured for bilateral in-ear stimulation. FIG. 1F provides detailed illustration of an in-ear stimulation device comprising reversibly removable, disposable electrodes, sensors to measure biological signals, and headphone speaker for audio therapies or relaxation techniques such as positive affirmations, guided mindful mediation, or binaural audio.
FIG. 2 schematically illustrates selected anatomy relating to the ear and the auricular branch of the vagus nerve.
FIG. 3 illustrates an embodiment of an in-ear stimulation device, specifically targeting the auricular branch of the vagus nerve.
FIG. 4 illustrates a block diagram of an embodiment of a device and system that provides peripheral nerve stimulation and senses biological signals to control the delivered stimulation.
FIG. 5 illustrates various embodiments of a monitoring unit and a therapy unit that form a two-part treatment system.
FIG. 6 illustrates the regulation of stimulation to an early expiratory phase of the respiratory cycle, illustrating stimulation synchronized with certain changes in measured heart rate and tidal volume, for example.
FIGS. 7A-7D are flow charts and including potential diagnostic, treatment, and disease and patient management and tracking steps, according to some embodiments of the invention. FIGS. 7E-7F schematically illustrates a diagnosis, assessment, and prescription flow chart for a subject with imbalance in autonomic tone and an associated disease, according to some embodiments of the invention.
FIGS. 8 and 9 illustrate human body meridian points that can be used as locations for stimulation.
FIG. 10 illustrates an embodiment of the wearable therapy system that uses the cloud to receive and transmit data between the therapy system and various users, including patient and physician.
FIG. 11 is a block diagram that illustrates the individual components of the therapy unit, band, and base station shown in FIG. 10.
FIG. 12 illustrates a block diagram of a stimulator with a microprocessor and switches, according to some embodiments of the invention.
FIG. 13 illustrates an embodiment of a stimulator with electrodes that can be disposed on a wearable band, according to some embodiments of the invention.
FIGS. 14-16 illustrate various systems and methods of peripheral nerve stimulation can be provided that targets one, two, or more individual nerves.

### DETAILED DESCRIPTION

In one embodiment, a neurostimulation system, delivers stimulation in synchronization with a prespecified phase of a measured biological rhythm or biological cycle, such as the breathing cycle, tonic sympathetic rhythm, cardiac rhythm, and the like. In some embodiments, a neurostimulation system and method is not synchronized to a breathing cycle. FIGS. 1-1F illustrate multiple possible embodiments of a neurostimulation system comprising a power source, processor, memory, one or more stimulators, one or more stimulating electrodes, and one or more sensors. In some embodiments, the system has one or more communication modules for wired or wireless (e.g., Bluetooth) communication between multiple stimulators. FIG. 1 illustrates a neurostimulation system including a monitoring unit, stimulating unit, and skin interface. The monitoring unit can include one or more sensors, a power source, and a user interface. The monitoring unit can be connected via wires or wirelessly to a stimulation unit. The stimulation unit can include a signal converter, controller, electrical pulse generator, user interface, and power source, and be operably connected, such as via a wired connection, to a skin interface including one, two, or more end effectors, such as electrodes for example. The skin interface can be placed, for example, transcutaneously over skin and other tissue proximate one, two or more target nerves. FIG. 1A illustrates a neurostimulation system similar to, and including any number of features of the embodiment of FIG. 1, illustrating that the monitoring unit need not necessarily include a discrete user interface. FIG. 1B illustrates a neurostimulation system similar to, and including any number of features of the embodiment of FIG. 1 or FIG. 1A, illustrating that the monitoring unit could include one, two, or more sensors connected via wires or wirelessly to the stimulation unit, and via a wired connection to a power source of the stimulation unit. FIG. 1C illustrates a neurostimulation system similar to, and including any number of features of the embodiment of FIGS. 1-1B, illustrating that the monitoring unit and stimulation unit could be operably connected to a common user interface via wired or wireless connections.

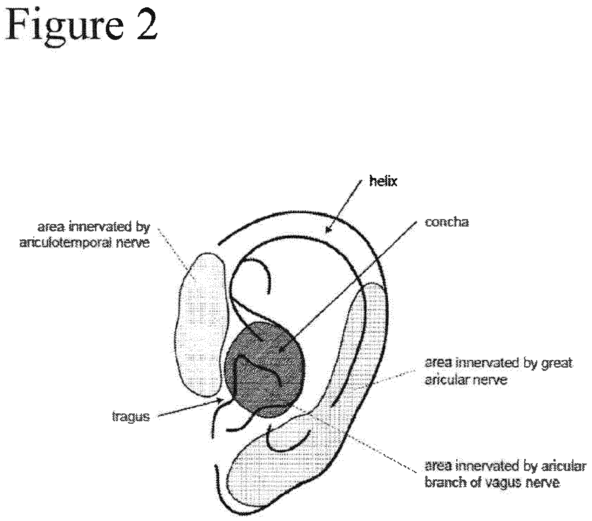

In a further embodiment, timing stimulation with breathing can take advantage of the cycle of suppression of vagal tone and parasympathetic activity by the baroreceptors during the inhalation phase of each breath. Stimulation of vagal afferents, such as via the auricular branch of the vagus nerve, as illustrated in FIG. 2, which is acting to increase vagal tone, can be enhanced by activating the stimulation only during the exhalation phase of breathing, when the baroreceptors are not acting to suppress vagal tone. Stimulation of peripheral sensory afferents that modulate sympathetic tone, such as the C and A-delta fibers in the median nerve, can be enhanced by activating the stimulation only during the inhalation phase of breathing, when the baroreceptors are acting to suppress vagal tone. In a further embodiment, respiration phase is measured using an effort belt, as illustrated in FIG. 1D, or other devices as disclosed elsewhere herein.

Not to be limited by theory, stimulation of sensory afferents in the peripheral nervous system can modulate sympathetic activity via projections to the hypothalamus. Efferents projecting from the hypothalamus descend through the brainstem and medulla to synapse with the intermediolateral cell column neurons where preganglionic fibers emerge to the chain of sympathetic ganglia which can modulate sympathetic activity on a systemic level.

In some embodiments, disclosed herein are systems for stimulation of a plurality of peripheral nerves regulated by a biological signal for the treatment of conditions associated with acute, paroxysmal, and/or chronic imbalance of autonomic activity, such as clinical depression, anxiety, hypertension, atrial fibrillation, overactive bladder, inflammatory bowel diseases, etc. Stimulation of two, three, or more nerves, such as a first nerve associated with modulation of sympathetic activity (e.g., via sensory afferents in the median, radial, and/or saphenous nerves), and a second nerve associated with parasympathetic activity (e.g., via vagal afferents of the auricular branch of the vagus nerve and/or tibial nerve via the sacral parasympathetics) can be surprisingly synergistic. Not to be limited by theory, dual nerve stimulation regulated by a biological signal can in some cases synergistically increase the effectiveness of therapy by individually modulating each limb of the autonomic nervous system to maintain autonomic homeostasis. For example, sensors in the system that measure aspects of autonomic activity, such as heart rate, heart rate variability, and/or electrodermal activity, as illustrated in FIG. 1C and 1F, can be collected and analyzed to determine the autonomic state of a patient, and stimulation can be applied to one or both nerves to modulate sympathetic or parasympathetic activity, or both. However, in some embodiments sympathetic or parasympathetic activity is not modulated.

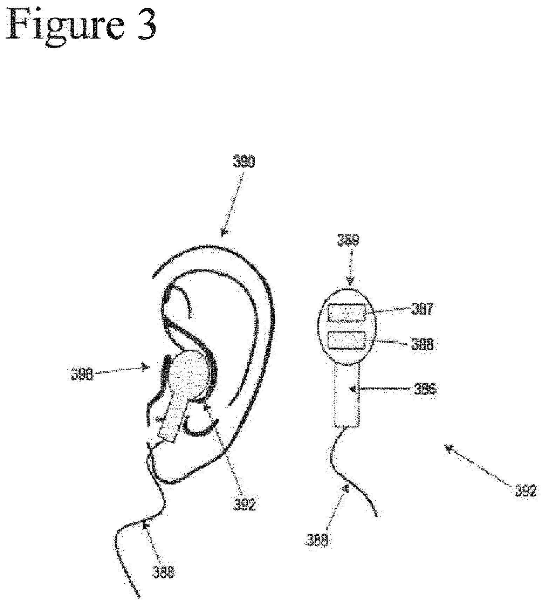

In some embodiments, disclosed herein are systems and methods for stimulation of a plurality of nerves regulated by a biological signal for the treatment of conditions including but not limited to chronic stress, clinical depression, generalized anxiety disorder, PTSD or other diseases as disclosed elsewhere herein. Stimulation of 2, 3, or more nerves, such as a first nerve associated with the wrist and a second nerve associated with the ear, e.g., the auricular vagus and median nerve could be used for the treatment of conditions such as depression. FIG. 3 illustrates an embodiment of an in-ear electrode to stimulate the auricular branch of the vagus nerve. Dual nerve stimulation regulated by a biological signal can in some cases synergistically increase the effectiveness of therapy by combining synergistically the effects of, for example, auricular vagus and median nerve stimulation, along with increased responsiveness of the nerves to stimulation during a selected phase or selected phases of a measured biological rhythmic or cyclic signals, such as the respiratory cycle, cardiac rhythm cycle, sympathetic tonic rhythmic discharge, sleep-wake cycle, or brain rhythm patterns. In some embodiments, the system can be configured to independently control stimulation of a first target nerve (including stimulation parameters such as frequency and others listed herein) and a second target nerve respectively. In some embodiments, the system can be configured to modulate a first target nerve when selected first criteria relating to the biological signal are recognized. The system can also be configured to modulate a second target nerve when selected second criteria relating to the biological signal are recognized. As one non-limiting example, a first target nerve can be modulated during an inspiratory phase of the respiratory cycle, and a second target nerve can be modulated during an expiratory phase of the respiratory cycle. The first target nerve and the second target nerve can be stimulated with either the same or different parameters, and can be stimulated simultaneously or in alternating or other fashion. In some embodiments, the stimulation systems can include a plurality of independent stimulation circuits, or a common circuit with a controller configured to switch stimulation parameters for one, two, or more nerves. In some embodiments, stimulation regulated by a measured biological signal can be unilateral only or bilateral. In some embodiments, bilateral stimulation could involve, for example, stimulation of the left auricular vagus nerve and the left median nerve, for example, alternating or concurrent with stimulation of the right auricular vagus nerve and the right median nerve. In some embodiments, the stimulation regulated by a biological signal could include ipsilateral stimulation (e.g., left auricular vagus nerve and the left median nerve, for example, alternating or concurrent with stimulation of the right auricular vagus nerve and the right median nerve) and/or contralateral stimulation (e.g., left auricular vagus nerve and the right median nerve, for example, alternating or concurrent with stimulation of the right auricular vagus nerve and the left median nerve). In some embodiments, bilateral stimulation could involve, for example, stimulation of the left auricular vagus nerve and another nerve proximate the left ear or facial region, for example, alternating or concurrent with stimulation of the right auricular vagus nerve and another nerve proximate the right ear or facial region. In some embodiments, the stimulation regulated by a biological signal could include ipsilateral stimulation (e.g., left auricular vagus nerve and another nerve proximate the left ear or facial region, for example, alternating or concurrent with stimulation of the right auricular vagus nerve and a nerve proximate the right ear or facial region) and/or contralateral stimulation (e.g., left auricular vagus nerve and another nerve proximate the left ear or facial region, for example, alternating or concurrent with stimulation of the right auricular vagus nerve and another nerve proximate the right ear or facial region). In some embodiments, bilateral stimulation could involve stimulation of the left auricular vagus nerve and the right auricular vagus nerve, with or without stimulation of additional nerves. In other words, the stimuli may be applied in sequence (e.g., serially or one after another) and/or in at least partially overlapping (e.g., parallel). Other permutations and target nerves including those disclosed elsewhere herein are also possible. Sensors may or may not be used to for example, measure respiratory activity/cycles. In some embodiments, a vagal nerve and one or more non-vagal nerves are stimulated.

Stimulation of the auricular branch of the vagus nerve can occur, for example, noninvasively via a plug, earpiece, or other device that can include electrodes for transcutaneous electrical stimulation in some cases. FIG. 3 illustrates an embodiment of a tragus stimulator 392 with an earbud configuration positioned in the tragus 398 of the ear 390. The stimulator 392 can be wired as shown, or wireless in other embodiments. The stimulator 392 can include a distal ear receptacle portion 389 that can include a cathode 387 and an anode 388, a hub 386 proximate the receptacle portion 389, and a conduit 388 to a source of electromagnetic energy, such as electrical energy. In some embodiments, the tragus stimulator 392 includes one or more sensors for measuring parameters relating to stimulation and/or physiologic function as discussed elsewhere herein. The in-ear stimulator 392 can be unilateral or bilateral (e.g., placed in both ears), also shown schematically in FIG. 1D and 1E. In some embodiments, systems and methods do not stimulate the auricular branch of the vagus nerve.

In some embodiments, a system can include a plurality of stimulators that communicate with each other wirelessly and provided a synchronized, patterned stimulation. In some embodiments, multiple stimulators may be in electrical connection with multiple electrode pairs to stimulate multiple nerves simultaneously. In one embodiment, a system can include a stimulator on the wrist to target median nerve and a stimulator in the ear to target the auricular branch of the vagus nerve. Each stimulator in the system can communicate with each other via a wired or wireless connection. Multiple stimulators can provide synchronized stimulation to the multiple nerves. Stimulation may be, for example, burst, offset, or alternating between the multiple nerves, completely offset in time, or overlapping. In some embodiments, stimulation is patterned, and not continuous stimulation.

Some embodiments of the system could centrally store biological measures from multiple wearers on a server system (e.g., the cloud), along with other relevant demographic data about each user, including age, weight, height, gender, ethnicity, etc. Data collected from multiple wearers can be analyzed using standard statistical analysis, machine learning, deep learning, or big data techniques, such as a logistic regression or Naive Bayes classifier (or other classifiers), to improve prediction of the onset of symptoms associated with the treated disease by determining correlations between biological measures, tracked symptoms, and other recorded events. These correlations can be used to set parameters of the stimulation waveform applied by the therapy unit, determine best time to apply stimulation therapy, and/or adapt the stimulation waveform applied by the therapy unit in real time.

The system can also log the patient satisfaction after each stimulation session or the end of a specified period, like a day or week or month, via an input on the device, which provides another piece of information to help feedback application of therapy. In some cases, if a person is satisfied, the therapy is maintained at the current stimulation waveforms and levels. In other cases, this may mean that the stimulation treatment may need to be optimized, for example, by changing stimulation parameters such as waveform frequency or amplitude.

In some embodiments, the wearable monitor can have a visual, auditory, tactile (e.g., squeezing band), or vibrotactile cues to notify the wearer of key events based on analysis of biological measures, including, but not limited to, prediction of cardiac dysrhythmia, cardiac dyssynchrony, blood pressure or increased blood pressure, and/or increase in stress level, heart rate, heart rate variability, or other parameters. The cuing system could also notify the wearer of other predetermined events or reminders set by the wearer. A cuing system can be used to communicate information to the wearer, such as the presence of a disease episode or episode of symptom onset such as atrial fibrillation, high blood pressure or other predetermined events, in a more discreet, personalized way, without drawing attention from others in social situations.

In some embodiments, the form of the wearable monitor and/or therapy unit could be a wrist band or watch, a ring, a glove, an arm sleeve or arm band or cuff, knee band, sock, leg sleeve or cuff, an ear piece/headphone, head band, a necklace or neck band, or a compliant patch that conforms to multiple locations on the body.

In one embodiment, the wearable monitor can have a processing unit and memory that collects, stores, processes, and analyzes the biological measures, along with other data input by the wearer.

In some embodiments, the wearable monitor can take user input about events, including diet history, medication history, caffeine intake, alcohol intake, sodium intake, etc. The monitor can use accelerometers to measure specific movements, gestures, or tapping patterns to record user inputs at specific prompts. Other touch sensors, such as resistive strips or pressure sensitive screens, could be used to measure specific gestures to record user inputs. These gesture-based measures to record user input minimize the complexity of steps required to input user data into the device. The data can be stored in memory and processed by the processing unit. In some embodiments, the data can be transmitted from the wearable monitor to an external computing device.

In one embodiment, the wearable monitor and/or the therapy unit can connect with other applications, such as calendars and activity logs, to sync and track events or a saved calendar can be saved and stored on the device. In some embodiments, the wearable monitor and/or the therapy unit can communicate with a variety of computing devices, such as a smart phone, a smart watch, a tablet, a laptop computer, or a desktop computer, for example, that have these applications. In some embodiments, the wearable monitor can include an ambulatory blood pressure monitor.

Some embodiments of the present invention relate generally to the treatment of diseases of which the underlying mechanisms are driven by dysfunction and/or imbalance of the autonomic nervous system, and more specifically to systems and methods of treating diseases or disease symptoms exacerbated by autonomic dysfunction, including but not limited to depression, anxiety, insomnia, hypertension, cardiac arrhythmia, overactive bladder, inflammatory bowel diseases (e.g., Crohn's disease, colitis, functional dyspepsia), fecal incontinence, headaches and migraine, chronic pain, vagal syncope, inflammatory diseases (e.g., rheumatoid arthritis, lupus, and other autoimmune diseases) through noninvasive or minimally invasive peripheral nerve stimulation controlled to be coordinated by one, two, or more biological or physiological signals, including but not limited to heart rate (electrocardiogram or ECG/EKG signal; photoplethysmography or PPG signal), respiratory rate and/or content (respiration rate; respiration phase; capnogram; oximetry; spirography), skin conductance (electrodermal activity or EDA signal), muscle current (electromyography or EMG signal), brain electrical activity (electroencephalography or EEG signal) or a combination (e.g., respiratory polysomnography), wherein a signal generated is generated by a biological or physiological sensor worn on or implanted in the body and said signal is sent to a controller to control the timing of delivering stimulation to one or more target nerves. The system can include a peripheral nerve stimulator including a pulse generator and at least two electrodes configured to deliver electrical stimulation to a nerve, acupressure point, or meridian in the patient's limb, torso, back, head, neck, and/or, ear. In some embodiments, the stimulation can be sufficient to reduce a number of symptoms exacerbated by autonomic dysfunction. In some embodiments, the system can also include one, two, three, or more sensors. In some embodiments, the stimulator, electrodes, and/or the sensor(s) could be implantable within a patient, minimally invasive, or non-invasive (i.e., body-worn). In some embodiments, electrodes and/or the sensor(s) could be percutaneous or transcutaneous. In some embodiments, the systems and methods do not have any implantable components. In some embodiments, electrical stimulation could be delivered bilaterally (i.e., on both sides of the body), or unilaterally.

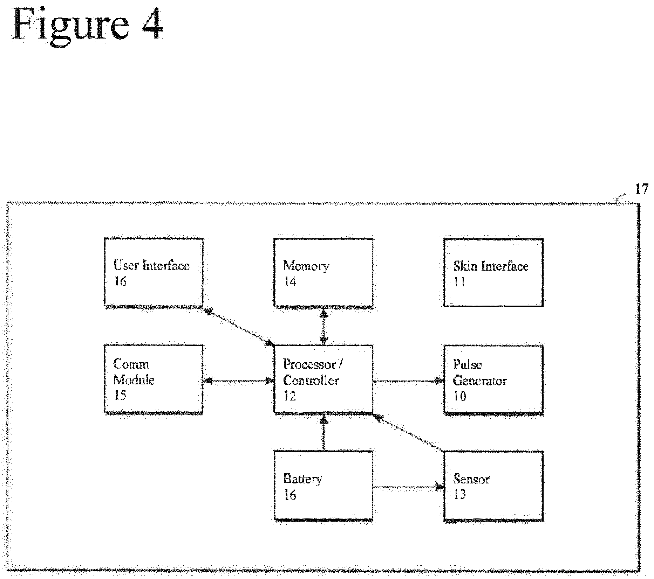

In some embodiments, as illustrated in FIG. 4, the system 17 comprises a pulse generator 10 that delivers electrical stimulation to a nerve through one or more skin interfaces 11 that sits adjacent to one or more target peripheral nerves. A processor and controller 12 receive one on more signals generated by one or more sensors 13 to control timing and parameters of stimulation. The processor 11 uses instructions stored in memory 14 to coordinate receiving signals from one or more sensors 13 and using that signal to control stimulation delivered by the pulse generator 10. Memory 14 in the system can store signal data from the sensors 13. The system has a communication module 15 to transmit data to other devices or remote server via standard wired or wireless communication protocols. The system is powered by a battery 16. The system has a user interface 17 that allows an end user to receive instructions, feedback and control aspects of the delivered stimulation, such as intensity of the stimulation.

In some embodiments, disclosed herein is a wearable device for inducing neural plasticity in a user with patterned transcutaneous electrical stimulation of afferent and/or efferent peripheral nerves regulated by one or more measured biological signals and/or biological rhythms. The biological signal can be cyclical (rhythmic) or non-cyclical (non-rhythmic) in nature. In some embodiments, patterned stimulation can be regulated by one or more techniques, including synchronizing stimulation, alternating stimulation bilaterally or across different target regions based on measurements of biological signals, including but not limited to, the respiratory cycle, neural oscillations, cardiac rhythms, circadian or diurnal rhythms, or movement rhythms, or a combination thereof. Synchronizing stimulation to phases of the respiratory cycle can take advantage on the cycle of suppression of vagal tone and parasympathetic activity by the baroreceptors during the inhalation phase of each breath. Stimulation of vagal afferents, such as via the auricular branch of the vagus nerve, which is acting to increase vagal tone, can be enhanced by activating the stimulation only during the exhalation phase of breathing, when the baroreceptors are not acting to suppress vagal tone. Stimulation of peripheral sensory afferents that modulate sympathetic tone, such as the C and A-delta fibers in the median nerve, can be enhanced by activating the stimulation only during the inhalation phase of breathing, when the baroreceptors are acting to suppress vagal tone. Synchronizing stimulation to neural oscillations can promote biofeedback for the patient by promoting and reinforcing alpha (8-13 Hz) wave activity in the brain, which has been shown to improve symptoms associated with seizures and the treatment of depression. Stimulation can also be synchronized to neural oscillation in the delta (0.5-4 Hz), theta (4-8 Hz), beta (13-32 Hz), or gamma (25-140 Hz) wave bands. In some embodiments, stimulation can be at a fixed or variable frequency. The frequency could be, for example, about 1 Hz, 2 Hz, 3 Hz, 4 Hz, 5 Hz, 6 Hz, 7 Hz, 8 Hz, 9 Hz, 10 Hz, or ranges including any two of the foregoing values. In some embodiments, the stimulation can include high-frequency alternating current such as about or at least about 1 kHz, 5 kHz, 10 kHz, 25 kHz, 50 kHz, 100 kHz, or more or less, or ranges including any two of the foregoing values. Alternating stimulation bilaterally, for example, alternating stimulation between the left and right auricular branch of the vagus nerve at a rate between 0.1-10 Hz, can improve the efficiency of accessing and processing traumatic memories or adverse experiences associated with anxiety, post-traumatic stress disorder, and other mental health disorders. Alternating stimulation bilaterally with eye movement using a technique known as Eye Movement Desensitization and Reprocessing (EMDR) can be effective in process trauma associated with post-traumatic stress disorder (PTSD). Synchronizing stimulation to phases of the cardiac rhythm can also take advantage on the cycle of increase in sympathetic tone followed by suppression of vagal tone and parasympathetic activity associated with respiratory sinus arrhythmia. Synchronizing stimulation to phases of circadian or diurnal rhythms can take advantage on natural daily and sleep cycles that modulate autonomic tone, both in the sympathetic and parasympathetic limbs. The device can include any number of a controller; a first peripheral nerve effector, comprising at least one stimulation electrode configured to be positioned to transcutaneously modulate a first afferent peripheral nerve; and at least one biomedical sensor or data input source configured to provide feedback information. The feedback information could include physiologic parameters including vital signs, measures of sympathetic or parasympathetic activity, information relating to the phase of the respiratory cycle, and other information disclosed herein. The controller can include a processor and a memory for receiving the feedback information from the sensor, that when executed by the processor, cause the device to adjust one or more parameters of a first electrical stimulus based at least in part on the feedback information; and/or deliver the first electrical stimulus to the first afferent peripheral nerve to the first peripheral nerve effector. The controller can be configured to synchronize/gate the stimulation to one or more particular phases of the respiratory cycle. The first electrical stimulus can include patterned, such as burst (e.g., theta burst) electrical stimulation configured to induce neural plasticity. The stimulation can be continuous, intermittent, or intermediate theta burst stimulation in some embodiments. The stimulation can alternate bilaterally across nerves on the left and right side of the body. The device can also be configured to deliver a priming electrical nerve stimulation signal prior to the first electrical stimulation signal, which can be a non-theta burst stimulation signal. The device can further include a second peripheral nerve effector, including at least one stimulation electrode configured to be positioned to transcutaneously modulate a second afferent peripheral nerve, and is configured to deliver a second electrical nerve stimulation signal transcutaneously to the afferent peripheral nerve of the user. The signal can include, for example, electrical theta burst stimulation.

In one embodiment, the system delivers stimulation to one or more peripheral nerves during specific phases of a rhythmic biological signal, including but not limited to breathing cycles, cardiac rhythm cycles, and/or diurnal or circadian cycles and a controller that determines when to deliver stimulation to one or more peripheral nerves based on a measured biological signal from a sensor. In a further embodiment, the system includes sensors to measure phases of the breathing cycle, such as an effort belt, video or doppler based systems that measure motion of the torso or thorax, or transthoracic impedance measurements, a processor to detect phases of the breathing cycle, and a controller to deliver stimulation during specified phases, such as expiration or inhalation. In another embodiment, the system includes sensors to measure cardiac rhythm, including but not limited to sensing electrode to measure cardiac electrical activity, such as an electrocardiogram (ECG) or photoplethysmography (PPG), a processor to detect changes in cardiac rhythm associated with sinus respiratory arrhythmia (SRA) and a controller to deliver stimulation during specified phases of SRA. In another embodiment, the system includes sensors that measure brain oscillations, such as electroencephalogram (EEG), with a process to detect a dominant frequency or multiple dominant frequency, and a controller to adjust one or more stimulation parameter based on the measure neuronal frequencies. In another embodiment, the system includes a plurality of sensors to detect sleep wake cycles, such as accelerometry, video-based, or doppler-based sensors to detect motion, EEG to measure brain state, bed pressure sensors to detect motion while lying in bed, heart rate monitors to detect changes in heart rate and heart rate variability, and skin impedance sensors to detect electrodermal activity, a processor to determine specific phases of the circadian or diurnal cycle and a controller to deliver stimulation during specific phases of the circadian or diurnal cycle, such as in the early morning hours when sympathetic tone may be most elevated.

In some embodiments, a therapy system regulated by one or more measured biological signals can include any of the following components: (1) a monitoring unit having sensors, circuitry, and optionally may have a power source and/or a microcontroller, (2) a therapy unit having a stimulator (e.g., a pulse generator), circuitry, a power source and a microcontroller, (3) a skin interface having electrodes and electrical connections for electrically connecting the electrodes to the therapy unit, and (4) a respiratory detection device as disclosed elsewhere herein. In some embodiments, all components are separate components that can be reversibly attached to each other to form a wearable therapy system. In some embodiments, any two or more of the components can be combined or integrated together to form a wearable system that can be reversibly attached to each other. It should be noted that some functions can crossover, such as the electrodes of the skin interface being used as sensors to measure electrical activity (e.g. EMG and ECG) and impedance, for example. In some embodiments, any one of the detachable components can be disposable and/or can be sent back to the manufacturer for recycling.

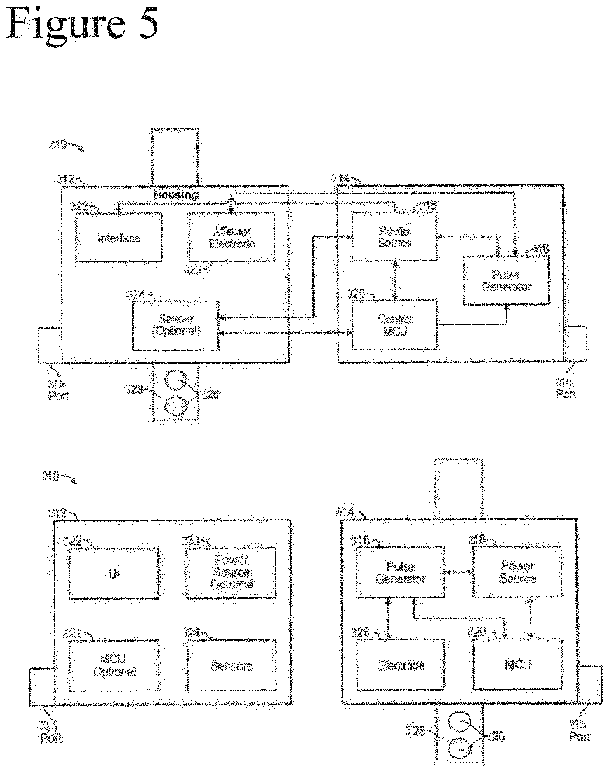

One embodiment, as shown in FIG. 5, is a system 10 including a monitor unit 12 that can be wearable in some embodiments and 2) a therapy unit 14. In some embodiments, the therapy unit 14 can be can be detachable and can be reversibly attached to the wearable monitor unit 12. The therapy unit 14 may contain an electrical stimulation signal generator 16, power source 18, and a microprocessor and/or microcontroller 20 to control the stimulation. The therapy unit 14 can reversibly connect and communicate directly and/or wirelessly to the wearable monitor 12. In some embodiments, the therapy unit 14 may remain 5 separates from the wearable monitor unit 12 and can communicate wirelessly with the wearable monitor unit 12. In some embodiments, the therapy unit 14 can have a data/power port 15, such as a USB port that allows a user to charge the power source 18, update the software and/or parameters on the microcontroller 20, and/or retrieve data from memory on the wearable monitor unit 12 and/or therapy unit 14. In some embodiments, the data/power port can be located on the 10 wearable monitor unit 12 or both the wearable monitor unit 12 and therapy unit 14. In some embodiments, the wearable monitor unit 12 and/or therapy unit 14 can communicate wirelessly with an external computing device to update the software and/or parameters and/or retrieve data. The devices can be in operable communication with a respiratory detection device (not shown) as disclosed elsewhere herein.

In some embodiments, a neuromodulation system regulated by a measure biological signal can include a plurality of stimulators that communicate with each other wirelessly and provide a synchronized continuous or patterned stimulation, and/or synchronize the timing of different stimulations. In some embodiments, multiple stimulators may be in electrical connection with multiple electrode pairs to stimulate multiple nerves simultaneously. Each stimulator in the system can communicate with each other via a wired or wireless connection. Multiple stimulators can provide synchronized stimulation to the multiple nerves, gated to either the same or a different phase of the respiratory cycle. Stimulation may be, for example, burst, offset, or alternating between the multiple nerves. In some embodiments, a stimulation system with a plurality of stimulator housings that can include or be operably connected to a patch having electrodes and a skin-contacting surface. The stimulators can be physically discrete for each other, or combined into a single housing.

In some embodiments, the stimulation involves one, two, three, or more target tissue locations. The target tissue could involve neural tissue, or non-neural tissue (e.g., muscle stimulation, such as functional electrical stimulation). The neural tissue to be modulated could include, for example, the brain including brainstem, cranial nerves, the spinal cord, sympathetic and parasympathetic nerves, and/or non-cranial peripheral afferent and efferent nerves. In some embodiments, the neural tissue to be modulated does not include one more of the brain including brainstem, cranial nerves, the spinal cord, sympathetic and parasympathetic nerves, and/or non-cranial peripheral afferent and efferent nerves.

Brain structures suitable for neuromodulation regulated by a biological signal could include, for example, deep brain structures, including one or more of the caudate nucleus, zona incerta, ventralis intermedius, subthalamic nucleus, red nucleus, substantia nigra, internal capsule, putamen, and globus pallidus externus or intemus.

Cranial nerves, and branches thereof suitable for neuromodulation regulated by a biological signal could include, for example, any number of (including one, two three, or more of): olfactory, optic, oculomotor, trochlear, trigeminal (including its branches, e.g., the ophthalmic nerve (V1) including the frontal nerve, supraorbital nerve, supratrochlear nerve, lacrimal nerve, nasociliary nerve (including the sensory root of the ciliary ganglion, posterior ethmoidal nerve, long ciliary nerve, infratrocheal nerve, and anterior ethmoidal nerve), the maxillary nerve (V2) including the middle meningeal nerve, infraorbital nerve, zygomatic nerve, posterior superior alveolar nerve, greater and lesser palatine nerves, nasopalatine nerve, sphenopalatine ganglion, pharyngeal nerve, and the mandibular nerve (V3)), abducens, facial (including the temporal, zygomatic, marginal, and cervical branches), vestibulocochlear, glossopharyngeal, vagus, meningeal vagus, accessory and hypoglossal nerves; the posterior auricular nerve, and the greater (superficial) petrosal nerve. In some embodiments, nerves suitable for neuromodulation can include one, two, or more nerves with direct pathways to the spinal trigeminal nucleus (e.g., an innervation target of the vagus from the superior ganglion). In some embodiments, systems and methods do not stimulate one, two, or more of the foregoing nerves.

In some embodiments, neuromodulation regulated by a biological signal involves a vagus nerve, including any number of the auriculotemporal nerve, the greater auricular nerve, the auricular branch of the vagus nerve (auricular branch of the vagus nerve), or the cervical branch of the vagus nerve. In some embodiments, neuromodulation regulated by a biological signal does not involve a vagus nerve (or any of the foregoing vagal nerve branches), a parasympathetic nerve, and/or a sympathetic nerve or nerve tissue. In some embodiments, neuromodulation can involve any number of nerves on the external ear, such as, for example, the greater auricular nerve, the auriculotemporal nerve, and the lesser occipital nerve.

Peripheral nerves suitable for neuromodulation regulated by a biological signal could include any number of, for example, the median, ulnar, radial, or other nerves in the hand, arm, and spinal area, or along muscle or within joints. These regions target to the nerves may include the brachial plexus, medial nerves, radial nerves, and ulnar, median cutaneous, lateral cutaneous, dermal, or joint space nerves. These regions may also target the musculature including muscles of the shoulder, muscles of the arm, and muscles of the forearm, hand, or fingers. Muscles of the shoulder may include, by non-limiting example, the deltoid, teres major and supraspinatus. Muscles of the arm may include the coracobrachialis and triceps brachii. Muscles of the forearm may include the extensor carpi radialis longus, abductor pollicis longus, extensor carpi ulnaris, and flexor carpi ulnaris. In some embodiments, one, two, three or more of these regions are stimulated (directly and/or indirectly).

In some embodiments, neuromodulation regulated by a biological signal can target, for example, a nerve on the lower extremity, such as a leg, knee, calf, ankle, and/or foot. In some embodiments, the tibial nerve or posterior tibial nerve can be targeted, which can branch into the medial and lateral plantar nerve branches, and the calcaneal nerves. The saphenous nerve is the cutaneous branch of the femoral nerve. Other nerves include, for example, the sural nerve, pudendal nerve, pelvic nerve, dorsal genital nerve, external anal sphincter nerve, and the dorsal genital nerve, or nerves in the sacral or lumbothoracic regions, for example. In some embodiments, the tibial (e.g., posterior tibial) nerve can be stimulated transcutaneously in a manner similar to percutaneous tibial nerve stimulation but noninvasively and in a more prolonged manner. The tibial nerve can provide parasympathetic modulation of the anti-inflammatory pathway. Specifically, electrical stimulation tuned to excite large myelinated fibers in the tibial nerve provides somatic afferent input to sacral plexus, mediating parasympathetic input via the pelvic nerves via release of cholinergic transmitters that bind to microphages in the blood stream to inhibit the release of TNF. In some embodiments, systems and methods include only transcutaneous elements without any implanted and/or percutaneous components. In some embodiments, the nerve(s) to be stimulated are lower extremity peripheral afferent nerves only, and are not spinal nerves. However, in some embodiments, spinal nerves can be stimulated. The stimulators at the various sites can be implanted (e.g., subcutaneous) and/or transcutaneous. In some embodiments, the nerves to be stimulated are only upper extremity afferent peripheral nerves. In some embodiments, the nerves to be stimulated are only lower extremity afferent peripheral nerves. In some embodiments, the nerves to be stimulated are only cranial afferent peripheral nerves. In some embodiments, a nerve associated with the vagus nerve is stimulated together with one, two, or more upper extremity or lower extremity peripheral nerves.

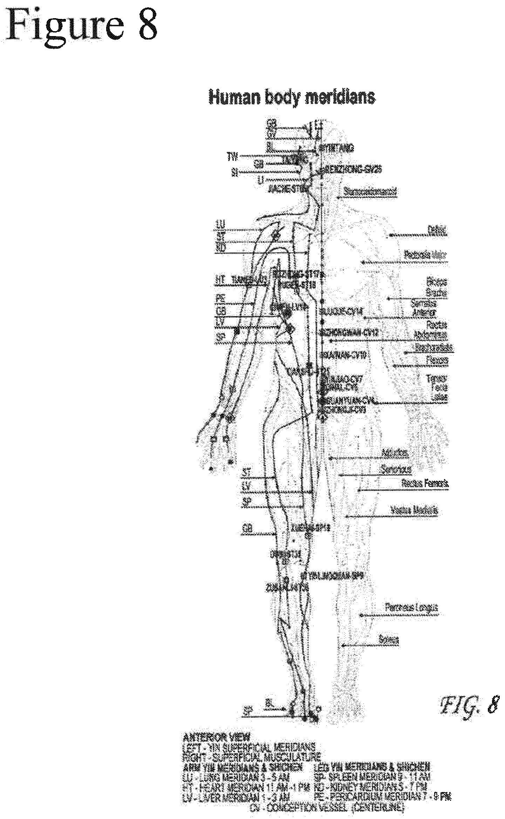

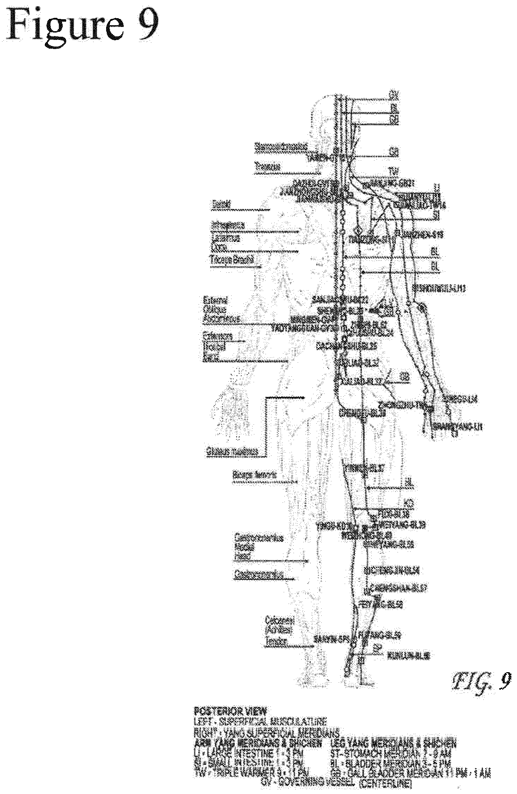

In some embodiments, any form of stimulation regulated by a biological signal as disclosed herein can be utilized to apply transcutaneous and/or percutaneous stimulation to one, two, or more acupuncture points. In some embodiments, the acupuncture points to be stimulated could include any one, two, three, four, five, six, seven, eight, nine, ten, or any other number of the following: BL18 (Ganshu), BL23 (Shenshu), BL27 (Xiaochangshu); BL28 (Pangguangshu); BL32 (Ciliao); BL33 (Zhongliao); BL53 (Baohuang); CV2 (Qugu); CV3 (Zhongji); CV4 (Guanyuan); CV5 (Shinen); CV6 (Qihai); GB34 (Yanglingquan); KI7 (Fuliu); KI10 (Yingu); LR1 (Dadun); LR2 (Xingjian); LR8 (Quan); N-BW-38 (Xiajiaoshu); SP6 (Sanyinjiao); SP9 (Yinlingquan); and/or ST28 (Shuidao). In some embodiments, the points to be stimulated include BL18, BL23, BL28, and CV2. In some embodiments, the points to be stimulated include ST28, SP6, BL23, BL28, BL32, BL33, BL53, CV3, and N-BW-38. In some embodiments, the points to be stimulated include SP6, BL23, BL27, BL28, BL33, and CV4. In some embodiments, the points to be stimulated include SP9, LR1, LR2, CV4, and CV6. In some embodiments, the points to be stimulated include SP6, SP9, BL23, CV3, and CV6. In some embodiments, the points to be stimulated include SP9 and GB34. In some embodiments, the points to be stimulated include SP9, KI7, KI10, and LR8. In some embodiments, the point to be stimulated is either CV5 alone or BL39 alone, or a combination thereof. Other permutations of stimulation points are also possible, depending on the desired clinical result. FIGS. 8-9 illustrate non-limiting examples of potential acupuncture points that can be stimulated, in accordance with some embodiments of the invention. In some embodiments, one, two, or more of the above-mentioned acupressure points, or none of the above-mentioned acupressure points are stimulated by systems and methods as disclosed herein.

In some embodiments, stimulation regulated by a biological signal can involve electrical stimulation as disclosed elsewhere herein. In some embodiments, stimulation regulated by a biological signal could also, instead or in addition, involve stimulation modalities other than or in addition to electrical stimulation. For example, at least one of the first stimulation actuator or the second stimulation actuator may comprise a vibrational or mechanical actuating actuator (such as means for vibrating, generating and transferring vibration to for example the skin, (e.g., vibrator, sonic systems, solenoids, offset motor) including for example an element, device, mechanism, component or portion affector, or the like). In some embodiments, a mechanical actuator configured to apply mechanical energy that is not vibrational may be activated to apply pressure at a specific location, such as an acupressure point. In some embodiments, a peg with a rounded end or other shaped element may be driven by a motor or solenoid to protrude into skin from a band worn around a body part to apply pressure to a nerve (e.g., the median nerve for a wrist band). In some embodiments, a band worn around a body part may be driven by a motor or solenoid to increase tension or tighten the band to apply pressure to a nerve (e.g., the median nerve for a wrist band). At least one of the first stimulation actuator or the second stimulation actuator may comprise a magnetic actuator (such as means for generating a magnetic field, (e.g., a magnet, an electromagnet) including for example an element, device, mechanism, component, portion, affector, or the like). At least one of the first stimulation actuator or the second stimulation actuator may comprise a chemical (e.g., pharmacological therapy, e.g., a local anesthetic such as lidocaine). At least one of the first stimulation actuator or the second stimulation actuator may comprise an ultrasonic (e.g., focused ultrasound) actuator (such as means for generating ultrasonic energy (e.g., a transducer, piezoelectric element, coupling fluid) including for example an element, device, mechanism, component, portion, affector, or the like). At least one of the first stimulation actuator or the second stimulation actuator may comprise a microwave actuator (such as means for generating microwave energy (e.g., a microwave generator) including for example an element, device, mechanism, component or portion affector, or the like). In some embodiments, at least one of the first stimulation actuator or the second stimulation actuator comprises an electromagnetic actuator for generating electromagnetic energy, waves, fields, etc. In addition to a second stimulation actuator, a third, fourth or additional stimulation actuator is used in some embodiments. In some embodiments, one or more of magnetic, RF, ultrasonic, vibrational, microwave, thermal, or cooling stimulation is not utilized.

The stimulation may include mechanical excitation of the proprioceptors by means including vibrotactile or haptic sensation. The mechanical stimulation might include force, vibration and/or motion. The effector induces action potentials in the target nerves by exciting the Golgi tendon organs (GTOs) or Pacinian corpuscles. Mechanical effectors can include, for example, small motors; piezoelectrics; one or more vibrotactile units comprised of a mass and an effector to move the mass such that a vibratory stimulus is applied to the body; an eccentric mass mounted on a shaft such that a vibratory stimulus is produced when the shaft is rotated; an ultrasonic motor; a magnetorheological fluid (MRF) effector or electroactive polymer (EAP) effector; and/or a speaker (e.g., a haptic speaker, a piezoelectric speaker, an electroactive polymeric transducer, a magnetic coil speaker). In some embodiments, mechanical stimulation is not utilized.

The vibratory stimulus can in some embodiments be about 250 Hz, corresponding to the optimal sensitivity of the Pacinian corpuscles (also known as lamellar corpuscles). The Pacinian corpuscles are the nerve endings in the skin that sense touch and vibration. Deformation of the corpuscle opens pressure-sensitive sodium ion channels to cause action potentials. In one embodiment, the vibration may be below 50 Hz to excite the Meissner's corpuscles (also called tactile corpuscles) in the fingers that are sensitive to light touch. In some embodiments, the vibratory stimulus can be, for example, at least about, about, or no more than about 10 Hz, 20 Hz, 30 Hz, 40 Hz, 50 Hz, 60 Hz, 70 Hz, 80 Hz, 90 Hz, 100 Hz, 110 Hz, 120 Hz, 130 Hz, 140 Hz, 150 Hz, 160 Hz, 170 Hz, 180 Hz, 190 Hz, 200 Hz, 210 Hz, 220 Hz, 230 Hz, 240 Hz, 250 Hz, 260 Hz, 270 Hz, 280 Hz, 290 Hz, 300 Hz, 350 Hz, 400 Hz, 450 Hz, 500 Hz, or more or less, or ranges including any two of the foregoing parameters.

In some embodiments, the vibratory and/or electrical stimulus comprises a combination of one or more single frequencies. For example, a first single frequency can be applied for a first duration and a second single frequency can be applied for a second duration. The second duration may be after (e.g., immediately after, after a pause duration) the first duration. The second duration may at least partially overlap the first duration. Continuing the example, a third single frequency different than the second frequency can be applied for a third duration. The third duration may be after (e.g., immediately after, after a pause duration) the second duration. The third duration may at least partially overlap the second duration. The third duration may at least partially overlap the first duration. The third duration may at least partially overlap the first duration and the second duration. The example may continue for additional durations of single frequencies. The different frequencies can be applied to the same vibratory stimulator or different vibratory stimulators (e.g., on the same body part, on different body parts). Vibratory stimulus applied to different body parts may meet at a point between the body parts and may form a standing wave. In some embodiments, the meeting point may be the target body part (e.g., elbow and finger meeting at the wrist).

In some embodiments, the vibratory and/or electrical stimulus comprises a combination of one or more single frequencies and one or more sweep frequencies (e.g., continuously changing from a lower frequency to a higher frequency). For example, a first single frequency can be applied for a first duration and a first sweep frequency can be applied for a second duration. The second duration may be after (e.g., immediately after, after a pause duration) the first duration. The second duration may at least partially overlap the first duration. Continuing the example, a second single frequency different than the second frequency can be applied for a third duration. The third duration may be after (e.g., immediately after, after a pause duration) the second duration. The third duration may at least partially overlap the second duration. Continuing the example, a second sweep frequency different than the first sweep frequency (e.g., having a different low frequency, high frequency, and/or rate of frequency change) can be applied for a fourth duration. The fourth duration may be after (e.g., immediately after, after a pause duration) the third duration. The fourth duration may at least partially overlap the third duration. The example may continue for additional durations of single frequencies and/or sweep frequencies. The different frequencies can be applied to the same vibratory stimulator or different vibratory stimulators (e.g., on the same body part, on different body parts). Vibratory stimulus applied to different body parts may meet at a point between the body parts and may form a standing wave. In some embodiments, the meeting point may be the target body part (e.g., elbow and finger meeting at the wrist). Amplitudes of the sweeps could be enveloped by other waveforms, such as sinusoid or Gaussian curves, or the parameters could be changed randomly or pseudo-randomly.

Discussions of different frequencies above can also apply to other parameters such as frequency, amplitude, pulse width, pulse spacing, phase, waveform shape, waveform symmetry, duration, duty cycle, on/off time, or bursting, combinations thereof, and the like. Discussions of vibratory stimuli above also apply, in several applicable embodiments, to other modalities and combinations thereof.

In some embodiments, the effector can be a phased array ultrasonic (e.g., focused ultrasound) effector. For example, a phased array ultrasonic effector may comprise a plurality of ultrasonic transducer elements. The elements may each have a width and a thickness. The thickness may be related to the width (e.g., thickness being a fraction (e.g., 1/2, 1/3, 1/4, 1/5, 1/10, ranges between such values, etc.) or multiple (e.g., 2×, 3×, 4×, 5×, 10×, ranges between such values, etc.) of the width). The elements may each have a width and a space between the elements may be related to the width (e.g., the same as the width, half the width, twice the width). Spacing between the elements may be adjustable. In some embodiments, the elements have a width between about 0.5 mm and about 2 mm and a spacing between about 0.1 mm and about 2 mm. The elements may be arranged in a one-dimensional array or a two-dimensional array. The elements may be cuboid, rectangular, cylindrical, prismatic, pyramidal, or any appropriate shape. Ultrasonic signals may be, for example, between about 20 kHz and about 2 GHz or more (e.g., about 20 kHz, about 50 kHz, about 100 kHz, about 500 kHz, about 1 MHz, about 1.5 MHz, about 2 MHz, ranges between such values, and the like). At least one of the elements may transmit a different frequency. Each of the elements may transmit a different frequency. Each of the elements may transmit a same frequency. In some embodiments, a dosage level applied by an ultrasonic effector is between about 0 W/cm² and about 2 W/cm² (e.g., about 0 W/cm², about 0.1 W/cm², about 0.25 W/cm², about 0.5 W/cm², about 1 W/cm², about 1.5 W/cm², about 2 W/cm², ranges between such values, etc.). One, some, or all of the ultrasonic transducer elements may be diverging, focused, scattered, flat, etc. In some embodiments, transducer elements may be arranged in a way to focus energy (e.g., energy from different elements results in constructive interference) at a location below the surface of the skin that is in proximity to a target nerve or region of tissue.

The ultrasonic transducer elements may be arranged on a printed circuit board. The ultrasonic transducer elements may be arranged on a flex circuit. A flex circuit may comprise, for example ultrasonic transducer elements and one or more other types of effectors. Certain circuit boards, flex circuits, etc. may include other combinations of effectors.

In some embodiments, the effector can be a magnet or a plurality of magnets. For example, a first magnet having a first polarity may be applied to a first site and a first magnet having a second polarity may be applied to a second site. The first polarity may be the same as the second polarity (e.g., positive-positive, negative-negative). The first polarity may be different than the second polarity (e.g., positive-negative, negative-positive). The first site may be proximate the second site (e.g., within about 10 cm, within about 8 cm, within about 6 cm, within about 5 cm, within about 4 cm, within about 3 cm, within about 2 cm, within about 1 cm, or closer). The first magnet may comprise the same material as the second magnet. The first magnet may comprise a different material than the second magnet. The first magnet may have the same dimensions (e.g., thickness, length, width diameter) as the second magnet. The first magnet may have at least one dimension (e.g., thickness, length, width diameter) different than the second magnet. The first magnet may have the same mass as the second magnet. The first magnet may have a different mass than the second magnet.

A magnet may comprise an electromagnet comprising a material that only becomes magnetic upon the application of a current to a wire wrapped around the magnet to create a magnetic field that can multiply the magnetic field strength of the material. In embodiments comprising an electromagnet, the magnetic effector can be turned on and off without moving the effector.

In some embodiments, magnetic energy can be applied using mini-TMS coils that are orthogonally oriented to the target nerve to deliver a burst of electromagnetic energy in proximity of the target nerve. A transcranial magnetic stimulation (TMS) coil comprises a toroidal shape containing multiple windings of a wire. Upon application of a current to the wire, a magnetic field is formed orthogonal to the plane of the toroid. The current can be altered over time, for example when connected to a stimulator. The magnetic field causes electric current to flow in the region of the body adjacent to the TMS. A mini-TMS could be a smaller version of a TMS configured to penetrate tissue, for example as opposed to the skull.

Several embodiments may alternatively apply other effectors including acoustic (using ultrasonic excitation for exciting sensory nerves at the fingertips), vibratory, tactile, luminescent (e.g., light exposure in optogenetically modified nerves), magnetically (e.g., by rapidly switching RF fields) or a combination of mechanisms. Some examples of modalities that may be used in combination include two, three or more of the following: electrical stimulation, magnetic stimulation, mechanical (e.g., vibrotactile) stimulation, focused ultrasonic (focused, high and/or low intensity, phased array), radiofrequency stimulation, or microwave stimulation. Such modalities may be used in the same region or on different regions. In some embodiments, different modalities are used to treat tremor or effect other neuromodulation for a single region, even though two or more body regions are modulated. For example, the wrist and the forearm may be modulated to synergistically reduce hand tremor. In other embodiments, different modalities are used to treat a disease or effect other neuromodulation for multiple regions. For example, the wrist and the ankle or leg, wrist and the ear, or ankle or leg and the ear may be modulated unilaterally or bilaterally, for example.

Some embodiments can involve stimulation patterns (e.g., bursting, pulse patterns, random, pseudo-random, or noise) selected to synergistically improve the efficiency and efficacy of stimulation regulated by a biological signal. In some embodiments, a system could include an electrode configuration to stimulate nerves in an alternating pattern that could be rhythmic or pseudorandom. For rhythmic alternating patterns, the alternating frequency can be in a range from 1-100 Hz, which has been shown to improve efficiency of therapy by promoting plasticity of corticospinal circuits. In some embodiments, a device embodiment could include an electrode configuration to alternate stimulation of nerve and adjust stimulation parameters (e.g., stimulation frequency, alternating frequency, duration of stimulation, stimulation time of day) based on an assessment of autonomic balance, for example, by measuring heart rate variability (HRV) and analyzing sympathovagal balance as a the ratio of absolute low frequency (LF) to absolute high frequency (HF) power, or LF/HF of measured HRV as noted elsewhere herein.

In some embodiments, stimulation regulated by a biological signal can be provided in a bursting pattern where the bursting can either be rhythmic (e.g., at regular intervals) or pseudorandom. In some embodiments, a stimulation waveform can be provided that combines infraslow stimulation frequency (0.01-0.1 Hz) with a higher frequency stimulation (1-200Hz), or lower frequency (1-200Hz) with very high frequencies (1000-10kHz).

Noninvasive peripheral nerve theta burst stimulation may be effective in some cases in driving cortical or spinal plasticity more efficiently than continuous stimulation to reduce symptoms and improve an individual's quality of life.

In some embodiments, the stimulation involves patterns of electromagnetic stimulation of peripheral nerves. The patterned stimulation could be a bursting stimulation, such as an on/off pattern that repeats at regular intervals (e.g., on for 10ms, off for 20ms, etc.), or non-burst patterned stimulation that can be more complex in some embodiments, such as a stochastic pattern or a sinusoidal envelope for example.

In some embodiments, the burst stimulation includes theta burst stimulation. Theta burst stimulation (TBS) is a patterned form of repetitive stimulation that uses high frequency pulses separated by varying inter-burst intervals. Originally used for the induction of long-term potentiation in hippocampal learning and memory research, theta burst stimulation in the form of repetitive magnetic stimulation (rTMS) has been demonstrated to noninvasively induce plasticity in humans in the motor, sensory and visual cortex. Depending on various parameters including the duration and continuity of stimulation, a long-term potentiation or depression (LTP/LTD) like effect can be observed, which are surrogate measures of synaptic efficacy. The number of sessions and the spacing interval between individual sessions of stimulation can also have an effect on the duration of the induced response. The level of muscle relaxation before or during stimulation can also affect the resulting direction or amplitude of plasticity induction suggesting that homeostatic mechanisms are in place that adjust the threshold for plasticity depending on prior synaptic activity. The effective modulation of nervous system plasticity demonstrated with theta burst stimulation can have great potential for the treatment of various neurologic disorders, and can have an effect on other central neural circuits.

In some embodiments, theta burst stimulation can take the form of intermittent theta burst stimulation (iTBS), continuous theta burst stimulation (cTBS), and intermediate theta burst stimulation (imTBS). Each illustrate examples of TBS including a burst of 3 stimuli at 50 Hz (20 ms between each stimulus) which was repeated at inter-burst intervals of 200 ms (5 Hz). In the iTBS example pattern, an about 2 second train of TBS is repeated about every 10 seconds for a total of 190 seconds (600 pulses). In the imTBS example pattern, an about 10 second train of TBS is repeated every 15 seconds for a total of 11 seconds (600 pulses). In the cTBS pattern, a 40 second train of uninterrupted TBS is given (600 pulses). The burst pattern (or a combination of two or more burst patterns) can be selected depending on the desired clinical result. In some cases, cTBS can be inhibitory, iTBS can be excitatory, and imTBS can be neither excitatory nor inhibitory, but this may be varied depending on the parameters. In some embodiments, inhibitory stimulation of a first nerve (e.g., the median, ulnar, or radial nerve) can be used alone or in combination with excitatory stimulation of a second nerve (e.g., the median, ulnar, or radial nerve), such as to restore or improve sympathetic and parasympathetic balance. In some embodiments, inhibitory or excitatory stimulation of a nerve can be controlled by adjusting frequency or pulse width of the stimulation waveform.

In some embodiments, a device can include a plurality of electrodes, e.g., four electrodes to where a first electrode pair stimulates at a specified first frequency, f Hz, and a second electrode pair stimulates at a second frequency slightly higher or lower than the first pair, f ± x Hz. In some embodiments, the second frequency can be different from that of, but within about ± 20%, 15%, 10%, 9%, 8%, 7%, 6%, 5%, 4%, 3%, 2%, or 1% of the first frequency, or ranges including any two of the foregoing values.

Stimulating at intensities below the sensory threshold or with high frequencies (e.g., between about 1 kHz to about 100 kHz) can avoid the discomfort (tingling, numbness, pain) that can be associated with peripheral nerve stimulation. Because the exact electrode position, size and surface contact can have a large effect on the stimulation level and the anatomical structures that receive the stimulation, the sensory threshold may need to be calibrated for each patient and even for each session. This calibration may be done by the user manually setting the stimulation parameters or otherwise indicating their sensory threshold. Another possible embodiment is for the device to automatically sweep through a range of stimulation parameters and the patient chooses the most comfortable set of parameter values. Another possible embodiment is for the patient to choose from among a set of previously chosen parameter values that provided effective and comfortable stimulation.

Also disclosed herein are systems and methods for treating various diseases, including but not limited to mental health disorders such as clinical depression and anxiety, and cardiac dysfunctions, such as hypertension and arrhythmias, that can include assessing at least one of sympathetic and parasympathetic activity of a subject and determining the presence of sympathetic or parasympathetic overactivity or underactivity in the subject; and stimulating a first peripheral nerve sufficient to have a therapeutic effect on the inflammatory response of a patient if abnormal sympathetic activity is present; and/or stimulating the a second peripheral nerve sufficient to have a therapeutic effect on a disease or symptom if abnormal parasympathetic activity is present. In some embodiments, stimulating comprises only electrical transcutaneous stimulation. The stimulation can include inhibiting or exciting nerve activity of one, two, three or more peripheral nerve targets. Sympathetic and parasympathetic activity of a subject can include measuring heart rate variability (HRV), such as via a wrist-worn device. Other parameters such as heart rate and electrodermal activity and blood pressure can be measured in addition or alternatively. HRV can be measured during a predefined period of time, such as 24 hours, either prior to and/or after the initial stimulation. Also disclosed herein in some embodiments is a method for treating various diseases, including but not limited to mental health disorders such as clinical depression and anxiety, and cardiac dysfunctions, such as hypertension and arrhythmias, that can include electrically stimulating a first nerve associated with autonomic response in a patient; assessing at least one of sympathetic and parasympathetic activity of a subject and determining the presence or absence of sympathetic or parasympathetic overactivity in the subject; assessing symptomatology of the disease; and adjusting the electrical stimulation based upon assessing the at least one of sympathetic and parasympathetic activity and the symptomatology of the disease. Adjusting the electrical stimulation can include, for example, identifying sympathetic or parasympathetic overactivity in the patient, and adjusting the frequency of stimulation of the first nerve; and/or discontinuing electrical stimulation of the first nerve associated with a physiologic parameter, sign, or symptom, including but not limited to an inflammatory response in a patient; and initiating electrical stimulation of a second nerve associated with a physiologic parameter, sign, or symptom, including but not limited to an inflammatory response in a patient. Any of the foregoing neuromodulation systems and methods can utilize synchronization with one, two, or more biological rhythms as described elsewhere herein.

Not to be limited by theory, stimulation of peripheral nerves regulated by a biological signal can synergistically enhance maintenance of homeostasis of the autonomic nervous system. For example, a change of sympathetic outflow can trigger a subsequent change of the parasympathetic outflow to maintain homeostasis of the autonomic nervous system. Thus, acute, repeated increases in sympathetic outflow by stimulation of peripheral nerves can chronically increase parasympathetic outflow, which then increases release of acetylcholine and inhibition of cytokine synthesis to reduce symptoms associated with chronic inflammation. In another example, a change in parasympathetic outflow can trigger a subsequent change of sympathetic outflow. Thus, acute, repeated, increases in parasympathetic outflow by stimulation of peripheral nerves can chronically increase sympathetic outflow. In some embodiments, stimulation of peripheral nerves may increase or decrease sympathetic and parasympathetic activity, which increases or decreases overall autonomic tone but maintains homeostasis.

The stimulation waveforms described herein can be applied continuously to target nerves, or can be provided in a manner that is adaptive in applying stimulation of various durations or by adjusting (increasing or decreasing) properties of the stimulation waveform, including but not limited to amplitude, frequency, and pulse width, in response to different inputs in the system. In some embodiments, the system could include closed loop control, using one or more signals measured by the device or feedback input into the device by the patient or physician to modulate the stimulation to improve efficacy. The signals or input could include, for example, any number of the following: sensors on-board the device or connected in the digital ecosystem; evaluation of autonomic function, reflex loop integrity, or excitability using heart rate variability, galvanic skin response, or pupil dilation, measuring muscle sympathetic nerve activity (MSNA), and/or measuring h-reflex by sending a stimulation signal and measure response with EMG. In some embodiments, the signals or input can also include sleep sensor sets, including but not limited to accelerometers, gyroscopes, infrared based motion sensors, and/or pressure sensors under a mattress, to measure night time motion as a measure of night time bowel events. For example, patients may wear a stimulator while sleeping and therapy can be triggered by night time restlessness, which is an indicator of an upcoming event. An EEG headband could be used to measure different sleep states or neural oscillations to provide biofeedback as previously described. Menstrual cycles can be measured from the date of the start or end of a period, or hormonal sampling levels, such as FSH, LH, estrogen, and/or progesterone for example, endometrial sampling, cervical mucus, and the like. Patient and/or physician input can provide feedback on the effectiveness of and/or satisfaction with the therapy into the device or into another connected device. Also, usage of the stimulation device can be tracked; and specific stimulation programs (e.g., a specified set of stimulation parameters) can be changed based on symptoms presented by the patient or outcomes of the therapy.

In one implementation, the system can include a respiratory sensing device comprising a respiratory belt placed around the rib-cage portion of the thorax to sense respiratory movements. Alternatively, electrodes on the chest may be adapted or combined with other methods to evaluate changes in electrical impedance across the thoracic and/or abdominal region over the respiratory cycle. Furthermore, the system may include pulse sensors on the extremities to measure blood pressure in peripheral arteries and derive waveforms to calculate cardiac performance. The respiratory belt may be equipped with a serial pneumatic bellow (where pressure inside the bellows may vary based on lung volume), a strain gage, or a piezoelectric device that is also in serial with the belt fabric, or a combination thereof. In another implementation, the sensing device can incorporate a nasal cannula to evaluate pulmonary activity by measuring respiratory flow and continuous exhaled concentration of carbon dioxide. A waveform signal describing the changes in carbon dioxide concentrations during the respiratory cycle can be collected. In another implementation, carbon dioxide detection can utilize an optical transcutaneous sampling cell for effective detection.

In some embodiments, the system may monitor the respiratory cycle utilizing non-contact sensing systems and methods, including but not limited to Doppler radar and/or optical sensors (e.g., infrared or video) configured to detect respiratory patterns. For example, systems and methods could incorporate features of respiratory detection from U.S. Pub. No. 2010/0152600 to Droitcour et al., which is hereby incorporated by reference in its entirety. In some embodiments, a flow, volume, gas (e.g., oxygen and/or carbon dioxide), humidity, temperature, nasal pressure sensor can be implanted in a device in fluid communication with the respiratory tract of the patient, including in a dental appliance within the patient's mouth, tracheal stent, nasal cannula, nasal or oronasal facemask, and the like. Other systems and methods could involve cameras including thermal infrared cameras (that can detect, for example, the presence of cooler room air on inspiration, and warmer exhaled air on expiration), pulse oximetry, and/or microphone sensors placed in proximity to the respiratory airways or over the throat to audibly detect phases of the respiratory cycle via the variation of sound.

The medulla oblongata contains the dorsal respiratory group (DRG) and the ventral respiratory group (VRG). The DRG is involved in maintaining a constant breathing rhythm by stimulating the diaphragm and intercostal muscles to contract, resulting in inspiration. When activity in the DRG ceases, it no longer stimulates the diaphragm and intercostals to contract, allowing them to relax, resulting in expiration. The VRG is involved in forced breathing, as the neurons in the VRG stimulate the accessory muscles involved in forced breathing to contract, resulting in forced inspiration. The VRG also stimulates the accessory muscles involved in forced expiration to contract. The second respiratory center of the brain is located within the pons, called the pontine respiratory group, and consists of the apneustic and pneumotaxic centers. The apneustic center is a double cluster of neuronal cell bodies that stimulate neurons in the DRG, controlling the depth of inspiration, particularly for deep breathing. The pneumotaxic center is a network of neurons that inhibits the activity of neurons in the DRG, allowing relaxation after inspiration, and thus controlling the overall rate. In some embodiments, EEG sensors (e.g., transcutaneous sensors) can be utilized to measure any number of the foregoing respiratory center activities in the brain and extrapolate the phase of the respiratory cycle from those EEG patterns. In some embodiments, EMG, ultrasonic or other imaging sensors can be utilized to determine the state of diaphragmatic or accessory muscle contraction or relaxation and extrapolate from that data the phase of the respiratory cycle.

Not to be limited by theory, respiration is known to cyclically modulate activity in both input and output vagal brainstem regions. Vagal afferent fibers modulate activity in the nucleus tractus solitarius (NTS), located at the upper medulla and the spinal trigeminal nucleus (SpV) by neurons located in the superior ganglion of the vagus nerve. Since the lungs are directly innervated by the vagus nerve, respiration can modulate NTS activity directly. Respiration can also indirectly affect activity in NTS, as inspiration increases arterial pressure via blood flow to the chest, which activates vagal afferent fibers and NTS via baroreceptors. The NTS then inhibits efferent vagal outflow to the heart, leading to a transient inspiratory tachycardia with every breath, which is known as respiratory sinus arrhythmia (RSA). During inhalation, intra-thoracic pressure lowers due to the contraction and downward movement of the diaphragm and the expansion of the chest cavity. Atrial pressure is also lowered as a result of this, enabling more blood to return to the heart. As more blood enters the heart, the vasculature and atria expand, triggering baroreceptors which act to suppress vagal tone. Subsequently, heart rate increases. During exhalation, the diaphragm relaxes, moving upward and decreases the size of the chest cavity, causing a subsequent increase in intra-thoracic pressure. This increased pressure inhibits venous return to the heart and thus less atrial expansion and activation of baroreceptors occurs. As these baroreceptors are no longer acting to suppress vagal tone, heart rate decreases. Based on this cycle of suppression of vagal tone by the baroreceptors, which is in phase with respiration, effects of stimulation of vagal afferents, which is acting to increase vagal tone, can be enhanced by activating the stimulation only during the exhalation phase of breathing, when the baroreceptors are not acting to suppress vagal tone. As such, in some embodiments, stimulation can be timed to changes in heart rate and/or rhythm, as a transient tachycardia arises with every breath. A heart rate sensor could detect this rhythmic tachycardia and generate a control signal to trigger stimulation. Heart rate or rhythm sensor could be multiple or single-lead ECG sensors, wrist worn optical heart rate sensor, also known as photoplethysmograms (PPG). In some embodiments, an effective heart rate change (increase or decrease) of, for example, about or at least about 2, 4, 6, 8, 10, 12, 14, 16, 18, 20 or more beats per minute over a 1, 2, 3, 4, 5, 6, 7, 8, 9, 10 or more second interval, for example, or ranges including any two of the aforementioned values, could trigger stimulation.

On an electrocardiogram, RSA can be detectable as changes in the P-P interval, or the distance between P waves. This corresponds to the initiation of depolarization of the atria and indicates the beginning of a new electrical cycle in the heart. ECG can be detected by various devices, including but not limited to a multi-lead Holter monitor, single-lead ECG patch, implantable loop recorder, and more recently, hand-held or wrist-worn sensors that measure electrical activity, such as the Kardia by AliveCor (Mountain View, CA); these hand-held or wrist-worn sensors typically require the user to make bilateral contact with the sensor (e.g., touch the sensor with both hands). Heart rate can be detected with various devices, including but not limited to, blood pressure sensors, photoplethysmography, typically in a wrist-worn device, electrical sensors typically attached to the chest via a strap, and pulse oximetry.

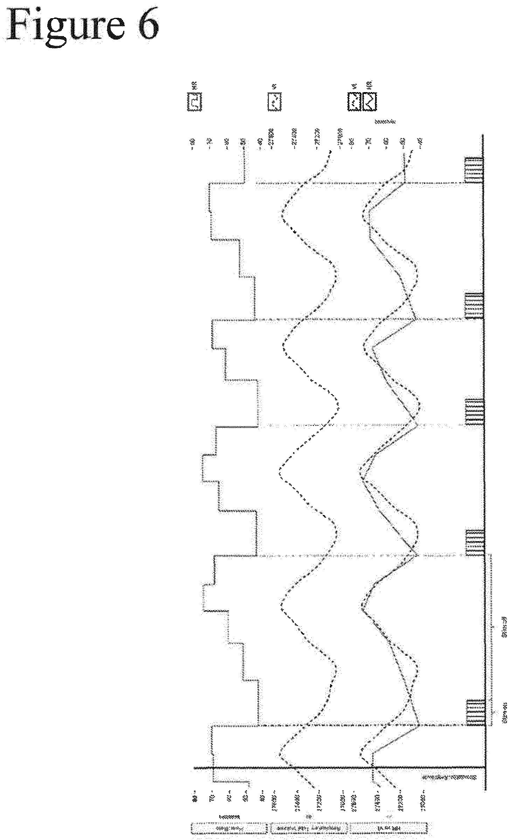

FIG. 6 schematically illustrates the regulation of stimulation to an early expiratory phase of the respiratory cycle, illustrating stimulation synchronized with certain changes in heart rate and tidal volume, for example. The stimulation can be synchronized with the exhalation portion of the respiration cycle based on a reduction in heart rate to an average threshold. Synchronization with other phases of the respiratory cycle can also be performed as described herein.

FIGS. 1-1D, and 1F illustrates schematically a stimulation system regulated by a biological signal that can include a sensing device, such as a respiratory and/or cardiac detection device, which includes a transducer, controller, and power source, in operable communication with a stimulation device including a power source that can be configured to be worn on a desired target location on the patient's anatomy, including an upper or lower extremity for example. The cardiac detection device could be a single-lead ECG chest patch in some cases.

In some embodiment, the system can include a detection device connected to a transducer that can convert the recorded electrical, electro- mechanical, or photovoltaic signals into a voltage signal. The transducer can transmit this voltage signal to a controller, which can include a circuitry capable of analyzing waveforms, generating a trigger for the stimulator, or a microchip with embedded software, as non-limiting examples. The controller can analyze the voltage signal in real-time and can detect different features of the respiratory cycle of the individual. The features detected of a biological signal waveform can include, for example, peaks, troughs, and slopes reaching, exceeding, or being less than a predetermined value, for example. Some embodiments can include time delays following a detected feature, or no time delay is utilized in some embodiments. Once these temporal landmarks are determined, the code on the controller can be further executed to produce an output signal to a high-frequency relay. The high-frequency relay may then allow the stimulator to pass current to the electrodes of the targeted anatomical location. The controller may use an algorithm to perform an analysis of biological signals received from the detection device and determine the overall pulmonary activity of the individual. The controller can identify specific points on the biological, e.g., respiratory signal where central autonomic nuclei may be more receptive to afferent input (e.g., during the expiration phase of the respiratory cycle) as illustrated schematically in FIG. 6, and produce and output signal to the high-frequency relay and the stimulation circuit. In some embodiments, while the stimulation can be synchronized to respiratory activity, the stimulation is not necessarily configured to affect or substantially affect respiratory function (e.g., one or more or respiratory rate, tidal volume, or minute ventilation). For example, dual peripheral nerve stimulation can be synchronized to two different phases of the respiratory cycle, to treat a mood disorder without affecting or substantially affecting a patient's respiratory function, and/or affecting or substantially affecting a patient's heart rate or rhythm in some embodiments.

As used herein, real-time can refer to within about 60 seconds, 45 seconds, 30 seconds, 20 seconds, 15 seconds, 10 seconds, 5 seconds, 1 second, 500 ms, 100 ms, 50 ms, or less from present, or ranges including any two of the foregoing values.

In some embodiments, the stimulation can be synchronized to early expiration, late expiration, early inspiration, and/or late inspiration. The stimulation could also be synchronized to, for example, the 1st, 2nd, 3rd, 4th, 5th, 6th, 7th, 8th, 9th, and/or 10th decile chronologically of an inspiratory and/or expiratory cycle, including ranges and/or combinations of any of the foregoing values. In some embodiments the stimulation could be synchronized continuously to the targeted phase(s) of each respiratory cycle, every other respiratory cycle, every third respiratory cycle, on for a predetermined or calculated number of targeted phases(s) of respiratory cycles and off for the same or different predetermined or calculated number of targeted phases(s) of respiratory cycles, or other patterns depending on the desired clinical result. In some embodiments, the stimulation could include a first stimulation mode during a first portion of the respiratory cycle, e.g., exhalation, and a second, different stimulation mode during a second portion of the respiratory cycle, e.g., another part of exhalation and/or inhalation. In some embodiments, a first target nerve associated with sympathetic or parasympathetic activity can be modulated during a first phase of the respiratory cycle, and a second target nerve associated with sympathetic or parasympathetic activity can be modulated during a second phase of the respiratory cycle. As one non-limiting example, a first target nerve associated with sympathetic activity can be modulated during inspiration, and a second target nerve associated with parasympathetic activity can be modulated during expiration.

In some embodiments, the stimulator can deliver various signals including electrical and other signals to target tissue, including but not limited to neural tissue including the vagus nerve, as well as other peripheral nerves as disclosed elsewhere herein. Once a trigger signal is generated from the controller, the stimulator may deliver a constant-current burst of bi-phasic square wave pulses at a frequency of about 5-200 Hz (or higher frequencies including up to about 20KHz in some embodiments), and current intensity in a range of 0.25mA to 20mA. Each pulse may have a pulse width varying from about 100-1000 microseconds. The burst timing of the burst can depend on the algorithm used to trigger the stimulation off of one or more measured biological signals and whether the burst is a fixed duration, is a percentage of one or more measured biological signals, terminates at a detected phase of a cyclical biological signal, or is based on some other algorithm implemented in the controller. For example, in some embodiments, the biological signal measured can be respiration and the burst may begin upon detection of the inspiration and/or expiration phase of the respiratory signal and continue for at least about, about, or no more than about 5%, 10%, 15%, 20%, 25%, 30%, 35%, 40%, 45%, 50%, 55%, 60%, 65%, 70%, 75%, 80%, 85%, 90%, or 95% of the average measured respiratory interval or ranges including any two of the aforementioned values.

In some embodiments, a stimulation approach regulated by a biological signal offers a 10-75% reduction in the time it takes to reach efficacy (e.g., a reduction in symptoms, such as an arrhythmia, for example) or a 10-75% increase in the length of therapeutic effect as compared to a therapeutic approach not regulated by a biological signal or no treatment. For example, in some embodiments, a stimulation approach regulated by a biological signal may reduce tremor during the stimulation and for a period of 30 minutes, 1-2 hours and 6 hours, or longer, post stimulation. Once or twice a day stimulation is provided in some embodiments. An optimized approach regulated by a biological signal in which feedback is used is provided in several embodiments.

The stimulation parameters may be adjusted automatically, or controlled by the user. The stimulation parameters may include on/off, time duration, intensity, pulse rate, pulse width, waveform shape, and the ramp of pulse on and off. In one preferred embodiment the frequency may be approximately 50 to 5000 Hz, and a preferred frequency of about 50 Hz to 300 Hz, or 150 Hz. For example, the frequency may be between about 10 Hz and about 20 kHz (e.g., about 10 Hz, about 20 Hz, about 30 Hz, about 40 Hz, about 50 Hz, about 60 Hz, about 100 Hz, about 250 Hz, about 500 Hz, about 1000 Hz, about 2500 Hz, about 5000 Hz, about 10 kHz, about 15 kHz, about 20 kHz, and ranges between such values). A preferred pulse width may range from 50 to 500 µs (micro-seconds), and a preferred pulse width may be approximately 300 µs (e.g., about 50 µs, about 100 µs, about 150 µs, about 200 µs, about 250 µs, about 300 µs, about 350 µs, about 400 µs, about 450 µs, about 500 µs, and ranges between such values). The intensity or amplitude of the electrical stimulation may vary from 0 mA to 500 mA, and a preferred current may be approximately 1 mA to 6 mA (e.g., about 0 mA, about 0.1 mA, about 1 mA, about 6 mA, about 10 mA, about 20 mA, about 30 mA, about 40 mA, about 50 mA, about 100 mA, about 200 mA, about 300 mA, about 400 mA, about 500 mA, and ranges between such values). Electrical stimulation can be adjusted in different patients and with different methods of electrical stimulation. These preferred settings are non-limiting examples. The increment of intensity adjustment may be 0.1 mA to 1.0 mA (e.g. 0.1-3 mA, 3-6 mA, 6-10 mA, and overlapping ranges therein). In one preferred embodiment, the stimulation may last for approximately 10 minutes to 1 hour (e.g. 10-20 minutes, 20-40 minutes, 40-60 minutes, and overlapping ranges therein). In some embodiments, the stimulation is below the motor threshold. In some embodiments, the stimulation is above the sensory threshold, but below the motor threshold for the target nerve. In some embodiments, the stimulation is below both the sensory threshold and the motor threshold.

In some embodiments, sympathetic and parasympathetic activity can be measured with body-worn sensors, including but not limited to pulse or heart rate, heart rate variability, electrocardiogram (ECG or PPG), electrodermal activity or galvanic skin response, blood pressure, skin temperature, and/or pupil dilation. Pulse, heart rate, or electrocardiogram sensors can measure changes in electrical activity when worn on the hand, wrist or chest, typically with multiple leads, or can use optical sensors as part of a photoplethysmogram system that measure changes in blood flow to calculate heart rate. Electrodermal activity or galvanic skin response, signal that reflects the action of sympathetic nerve traffic on eccrine sweat glands, measures changes in electrical properties of the skin, such as resistance, in the presence of a small electrical current or differences in the electrical potential between different parts of the skin. Blood pressure measurement devices measure changes in blood pressure during cardiac activity using an inflatable cuff, typically placed on the arm, wrist or fingers. Blood pressure can also be measured with other sensors placed on the skin, such as accelerometers or strain sensors that measure displacement of blood vessels. Skin temperature can be measured with a thermistor, thermocouple, or temperature sensor placed on the skin. Pupil dilation can be measured optically with image processing of video data, where for example, the video camera is disposed in a wearable set of eye glasses, contact lenses, or other form factors.

Embodiments of the invention can include a device and system and method to measure and collect biological data (e.g., heart rate, heart rate variability, ECG, galvanic skin response, temperature, and blood pressure), analyze the data as to interpret how these measures may influence a disease state, and provide peripheral nerve stimulation that targets one or more individual nerves to treat, prevent, or reduce symptoms associated with a disease, where the stimulation applied may or may not be modified based on the measured data. The peripheral nerve stimulation can be synchronized to one or more specific parts of a biological cycle for synergistically enhanced effect. The method can also include receiving an input relating to autonomic nervous system activity of the patient, including, for example, receiving data from a sensor that measures heart rate variability of the patient; and/or receiving data from a sensor that measures at least one of electrodermal activity, thermometry, and ECG information of the patient.

In some embodiments, the monitor unit can be a wearable monitor having a housing with a user interface. The housing can use a plurality of sensors to collect, store, and analyze biological measures about the wearer including, but not limited to, blood pressure, motion (e.g., accelerometers, gyroscopes, magnetometer, bend sensors), muscle activity (e.g., EMG using electrodes), cardiovascular rhythm measures (e.g., heart rate, heart rate variability, or ventricular and/or atrial dyssynchrony using electrodes to measure ECG, heart rhythm abnormalities), skin conductance (e.g., skin conductance response, galvanic skin response, using electrodes), respiratory rate, skin temperature, pupil diameter, and sleep state (e.g., awake, light sleep, deep sleep, REM). Heart rhythm measures can be recorded with optical, electrical, and/or accelerometry-based sensors. In particular, studies have shown that increased stress levels can increase blood pressure. Activities such as exercise, can also affect cardiac rate and/or rhythm, and/or affect blood pressure - measuring accelerometry (motion), heart rate, etc. could help identify these activities and normalize the measurements by similar activities. Additionally, hypertension has been correlated with heart failure - measuring ventricle dyssynchrony with ECG sensors could help identify the effectiveness of the stimulation to chronically reduce hypertension. Thus, using standard statistical analysis, machine learning, deep learning, or big data techniques, such as a logistical regression or Naive Bayes classifier, these biological measures can be analyzed to assess a person's state, such as level of stress, which in turn, can serve as a predictor for increases in cardiac dysrhythmia, cardiac dyssynchrony, and/or blood pressure. In some embodiments, the device can provide stimulation based on measurements of one or more biological measures, a determination of a person's state, and/or a prediction of cardiac dysrhythmia, cardiac dyssynchrony, and/or a change in blood pressure.

Sympathetic and parasympathetic activity can be measured through several methods, including microneurography (MSNA), catecholamine tests, heart rate, HRV, or galvanic skin response. HRV can provide a quick and effective approximation of autonomic activity in the body. HRV can be determined by analyzing the time intervals between heartbeats, also known as RR intervals. Heart rate can be accurately captured, for example, through recording devices such as chest straps or finger sensors. The differences between successive RR intervals can provide a picture of one's heart health and autonomic activity. Generally speaking, healthier hearts have more variability between successive RR intervals. This interbeat data can also be used to denote an individual's sympathetic and parasympathetic activity levels. Through frequency-domain analysis, heartbeat frequencies can be separated into distinct bands. High-frequency signals (~0.15-0.4 Hz) can almost exclusively reflect parasympathetic activity, and low-frequency signals (~0.04-0.15 Hz) can represent a mixture of sympathetic and parasympathetic activity. Therefore, taking the ratio of high frequency (HF) to low frequency (LF) signals can yield an approximation of one's sympathetic tone. In some embodiments, HRV can be analyzed, for example, under time domain, geometric domain methods in addition to frequency domain methods. In some embodiments, increased heart rate variability can signify increased parasympathetic response and/or decreased sympathetic response. Decreased heart rate variability can signify decreased parasympathetic response and/or increased sympathetic response. In some embodiments, a system can sense an increase or decrease in HRV of about or more than about 5%, 10%, 15%, 20%, 25%, 30%, 35%, 40%, 45%, 50%, 75%, 100%, or more over a baseline value (or target desired HRV value) and institute a change in one, two, or more stimulation modality parameters accordingly. In some embodiments, the one, two, or more stimulation modalities can be configured to modulate, such as increase or decrease stimulation to one or more nerves (e.g., peripheral nerves) associated with the sympathetic and/or parasympathetic nervous system, and a response to therapy can be confirmed by sensing an increase or decrease in parasympathetic or sympathetic tone, including but not limited to increase or decrease in HRV, changes in high frequency content of HRV, and changes in the ratio of high frequency and low frequency content of HRV. In some embodiments, balance of parasympathetic and sympathetic activity of the inflammatory response reflex loop can be assessed with frequency analysis of heart rate variability measured with pulsed plethysmography with an LED light source and optical sensor disposed in the device that measures fluctuations in light level due to blood flow that target one of the major blood vessels around the knee, which could include one or more of the following, femoral, popliteal, tibial, posterior tibial, anterior tibial, and/or descending genicular arteries or veins, or vessels around the wrist.

A large source of error in optical measurements of heart rate is motion artifacts due to relative motion between the optical sensor and the blood vessel being measured. In some embodiments, the optical heart rate sensor has an adhesive on the side of housing that contacts the wearer's skin to reduce relative motion between the sensor and the target blood vessel. In some embodiments, one, two, or more additional sensors are disposed in the device, including electrical sensors in contact with the wearer's skin to measure cardiac activity or pressure sensors to measure changes in blood vessels, to be used in combination with an optical sensor to improve the fidelity of heart rate measurement. In some embodiments, the system and device have memory and a processor to extract RR intervals from sensor data, calculate variability of RR intervals, transform data into frequency domain, and calculate high frequency signals, low frequency signals, and the ration of the high frequency and low frequency signals. In some embodiments, the heart rate sensor can store collected data for specified time periods to gather adequate data for heart rate variability calculation. Specified time period can range in some cases from 1 - 60 seconds, and may extend to 10 minutes or more.

In some embodiments, electrodermal activity, also known as galvanic skin response or skin conductance response, for example, can be measured using sensors, such as electrodes. Galvanic skin response is the change of the electrical resistance of the skin caused by emotional stress, and measurable with, e.g., a sensitive galvanometer. Not to be limited by theory, skin resistance varies with the state of sweat glands in the skin. Sweating is controlled by the sympathetic nervous system, and skin conductance can be an indication of psychological or physiological arousal. If the sympathetic nervous system is highly aroused, then sweat gland activity also increases, which in turn increases skin conductance. In this way, skin conductance can be a measure of emotional and sympathetic responses, which can be measured, and the feedback data can be sent to the controller, which will in turn modulate stimulation to, for example, decrease sympathetic nervous system activity. Other nonlimiting parameters associated with sympathetic and/or parasympathetic nervous system activity that can be sensed include, for example, sweating during particular times of the day and/or night, sleep states as detected, for example, by an EEG headband (to determine when sympathetic and/or parasympathetic activity is particularly high or low, and potentially correlating a sleep state such as stage 1, 2, 3, 4, or REM with nocturia), and/or motion. In some embodiments, a diagnostic and/or combination diagnostic/stimulation device can be configured to measure a person's heart rate and galvanic skin response for improved estimation of the person's autonomic activity. In some embodiments, a wearable device, such as a wrist-worn device can include both electrodermal activity (EDA) sensors and optical heart rate sensors. This combination of data can in some embodiments advantageously and synergistically provide improved estimation of sympathetic and parasympathetic activity than a single measure alone. In some embodiments, the system can include multiple sensors to measure electrodermal activity in conjunction with heart rate and HRV. Data from the multiple sensors can be analyzed by a hardware or software processor and combined to provide a more accurate estimation of sympathetic and/or parasympathetic activity. In some embodiments, the EDA and HR sensors can be disposed in a wrist-worn device that communicates via a wired or wireless connection to the stimulator or to send data to a centralized remote server (e.g., the cloud). Stimulation parameters, nerve target locations (e.g., tibial and/or saphenous nerves, median and/or auricular vagus, or others for example) or dosing regimen (e.g., duration or frequency of stimulation sessions) could be adjusted based on estimations of sympathetic and/or parasympathetic activity. Adjustments could be made in real-time, or in subsequent stimulation sessions. In some embodiments, stimulation frequency can be adjusted to either increase or decrease autonomic activity modulated by a single specific nerve, or multiple nerves. For example, in some embodiments, relatively low frequency stimulation of a target nerve (e.g., below a threshold value, e.g., about 5 Hz) can potentially inhibit the nerve and thus decreases sympathetic activity, while higher frequency stimulation (e.g., above a threshold value, e.g., about 5 Hz) can potentially excite the nerve and thus increases sympathetic activity. The same effect can occur with the same or other target nerves to regulate parasympathetic activity. In other words, in some embodiments, relatively low frequency stimulation of the target nerve (e.g., below a threshold value, e.g., about 5 Hz) can potentially inhibit the nerve and thus decreases parasympathetic activity, while higher frequency stimulation (e.g., above a threshold value, e.g., about 5 Hz) can potentially excite the nerve and thus increases parasympathetic activity. Not to be limited by theory, depending on the stimulation parameters for example, in some cases stimulating the target nerve can increase or decrease either sympathetic activity, parasympathetic activity, or both. In some embodiments, stimulation of the saphenous nerve can affect sympathetic activity, and stimulation of the tibial nerve can affect parasympathetic activity. The stimulation can be gated with respiratory activity for a synergistic effect as disclosed elsewhere herein.

In some embodiments, a neurostimulation system regulated by a biological signal can assesses cognitive state and symptomology associated with mental health disorders or mood disorders monitoring and recording the occurrence and timing of user events on a smart phone or mobile device, including but not limited to usage of applications, communications such as text, voice calls, and emails, activity data, such as walking, running, cycling, internet usage, social media engagement, where such user event data is stored in memory and used by a processor and controller to activate and/or modify stimulation, or notify a patient to start therapy, or notify a physician, therapist, healthcare professional, or caregiver. In some embodiments, initial data can be collected over a prespecified period to establish a baseline, and deviations from baseline activity or usage data can trigger notification or activation and/or modification of therapy. Other features can be used or modified for use from PCT Pub. No. WO 2015/060933 to Dagum, which is hereby incorporated by reference in its entirety. In some embodiments, data can be collected for a cohort of patients diagnosed with a specific disease. The data can correspond to sensors or usage of mobile computing devices associated with the cohort of patients. A first patient subgroup can be selected for a first patient from the cohort of patients based on first patient data from the mobile computing device indicating a first behavior associated with the first patient subgroup. A second patient subgroup can be selected for a second patient from the cohort of patients based on second patient data from the mobility dataset indicating a second behavior associated with the second patient subgroup. First medical symptom characteristics can be determined for the first patient subgroup; a relationship between the first medical symptom characteristics and first communication behaviors associated with the first patient subgroup can be identified; and a first treatment efficacy model associated with the first patient subgroup based on the relationship can be identified. The first treatment efficacy model can be operable to trigger notification or activation and/or modification of therapy. Other features can be used or modified for use from U.S. Pat. Pub. No. 2018/0068085 to Madan et al., which is hereby incorporated by reference in its entirety.

As part of a neurostimulation system regulated by a biological signal, in some embodiments, therapy can be applied and/or modified based on patient reported symptoms or patient responses to a clinically validated or prespecified survey that assess cognitive state or mood.

As part of a neurostimulation system regulated by a biological signal, in some embodiments therapy is applied and/or modified or patient is notified to start therapy based on assessment of mood or cognitive state derived from analysis of facial expression captured on a smart phone, tablet, or other device.

In some embodiments, a stimulator regulated by a biological signal can be part of a system with sensors to assess the state of sleep and modulate stimulation based on the wearer's sleep state. Sensors could include motion sensors (e.g., body worn accelerometers and gyroscopes, or wireless motion tracking via video or infrared), temperature sensors to measure body temperature, pressure sensor under the mattress to measure movement, heart rate sensors to measure HRV, other sensors to measure sympathetic and parasympathetic activity, and/or EEG sensors to measure brain activity to assess the wearer's sleep state. For example, if night time events occur during slow wave sleep when parasympathetic activity can be elevated, stimulation parameters are modulated to affect parasympathetic activity, and vice-versa for sympathetic activity. In some embodiments, stimulation regulated by a biological signal is applied while a patient is sedentary and/or asleep, which can be advantageous as activities of daily living including ambulating, talking, eating, and drinking can make it more challenging for certain respiratory detection devices to measure the real-time phase of the respiratory cycle.

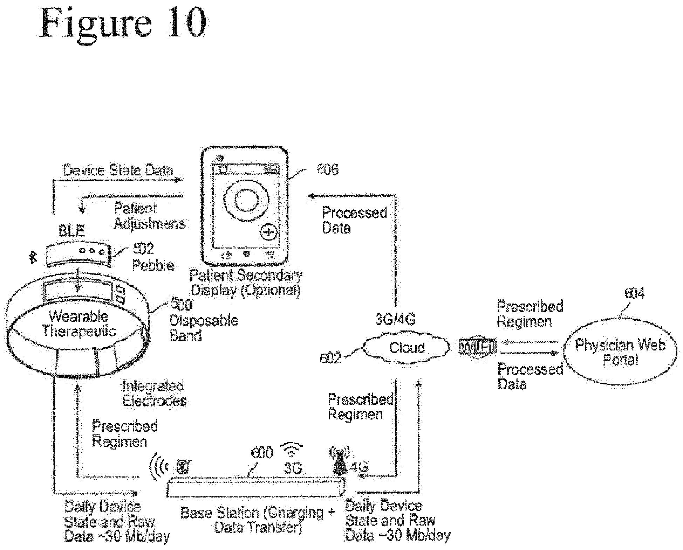

FIG. 10 illustrates an embodiment of a neurostimulation system regulated by a biological signal for treating a disease associated with imbalance of the autonomic nervous system, including but not limited to tremor, a cardiac disorder, a mental health disorder, or another disease or condition such as those disclosed elsewhere herein using a wearable therapy device. As described above, the therapy device may have two parts, a band 500 and therapy unit 502. A base station 600, which may replace the charger in the kit described above, can be used to both charge the therapy device and to receive and transmit data to the therapy device and to the cloud 602. Communication between the base station 600 and the therapy device can be wireless, such as through Bluetooth and/or Wi-Fi, and communication between the base station 600 and the cloud 602 can be through a cellular network, using a 3G or 4G connection, or through a wired connection to the internet, using DSL or cable or Ethernet, for example. A physician, patient, or other user can view and/or retrieve data stored on the cloud 602 using an online portal or a web portal 604. In addition, the physician can prescribe and/or modify a treatment regimen on the therapy unit 502 through the cloud 602 and base station 600 using the web portal 604.

As described above, the therapy device may include a removable skin interface and therapy unit. The system can also be in operable communication with a biological signal detection device, such as respiration, as described elsewhere herein. A base station, which may replace the charger in the kit described above, can be used to both charge the therapy device and to receive and transmit data to the therapy device and to the cloud. Communication between the base station and the therapy device can be wireless, such as through Bluetooth and/or WiFi, and communication between the base station and the cloud can be through a cellular network, using a 3G, 4G, or 5G connection, or through a wired connection to the internet, using DSL or cable or ethernet, for example. In an alternative embodiment, the device can communicate with a secondary device, such as a smart phone, tablet, or computer, which transmits data to the cloud via a cellular network or WiFi connection instead of the previously described base station. A physician or other user can view and/or retrieve data stored on the cloud using an online portal or a physician web portal. In addition, the physician can prescribe and/or modify a treatment regimen on the therapy unit through the cloud and base station using the web portal.

In some embodiments, the base station is used to receive and transmit relatively large amounts of data that may require a high bandwidth, such as the transmission of raw data from the therapy device, which may be about 10 to 100 Mb/day, or about 10, 20, 30, 40, or 50 Mb/day. In some embodiments, the data may be stored in memory in the base station and transmitted at another interval, such as weekly or twice weekly, with a scaling up of the bandwidth of transmission. The high bandwidth transmission of the raw data can occur daily while the therapy device is being charged, such as at night during a regular charging period. In some embodiments, the raw data can be processed by the cloud and/or the physician into processed data and sent back to the therapy device.

In some embodiments, the system may optionally include a portable computing device, such as a smart phone or tablet, to provide a secondary display and user interface for the patient and to run applications to more easily control the therapy device and view the raw and processed data. The portable computing device can be used to make patient or physician adjustments to the therapy device, such as adjusting the stimulation parameters and dosing, and can receive device state data from the therapy device, which includes data relating to the device, such as when the device was used, errors, therapy parameters such as amplitude and when they were set and delivered. In some embodiments, the portable computing device can receive processed data from the cloud through a cellular network and/or through an internet connection using WiFi, for example.

In some embodiments, various components of the neurostimulation system can include a therapy unit, reversibly removable skin interface, and base station. These components are described in detail above and also below as one particular embodiment. For example, the therapy unit can include one or more indicators, which can be LEDs, and a user interface, which can be push buttons, for example. The therapy unit can also have a stimulator with stimulation electronics and may include the capability to measure current and voltage. The therapy unit can also have a battery, which may be rechargeable and can be recharged using charging circuitry, which may be inductive. The therapy unit may further include a processor and memory to store and execute programs and instructions to accomplish the functions IO described herein. The therapy unit may also include sensors, such as motion sensors, and a communications module, which may be wireless and can communicate with the base station and/or a secondary display/computing device, as well as a respiratory detection device (not shown). The band can have electrodes and may also include memory to store identification information or may include some other form of identifier as described herein. The base station can include charging circuitry, which may also be inductive and can transmit power to the complementary charging circuitry on the therapy unit. The base station can also have a processor and memory for storing and executing instructions and programs. The base station can further include a communication module, which may be cellular, to communicate with the cloud, and another communication module, which may be wireless and used to communicate with the therapy unit and/or respiratory detection device.

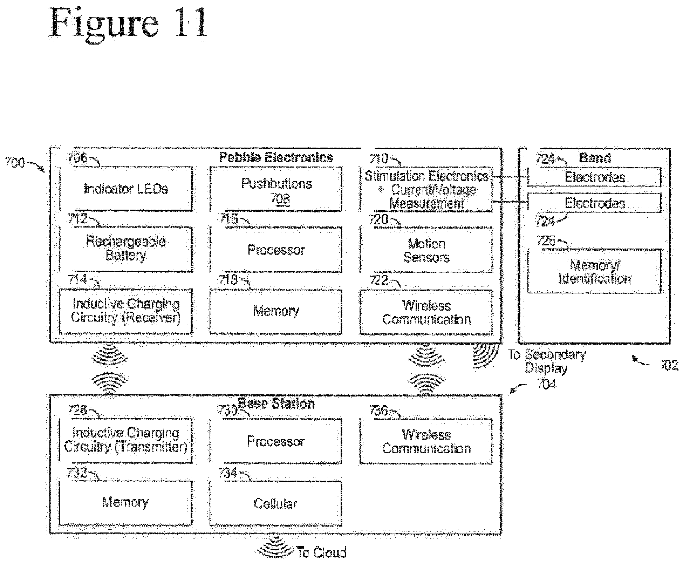

FIG. 11 illustrates the various components that can be included in a therapy unit 700, band 702, and base station 704. These components are described in detail above and also below as one particular embodiment. For example, the therapy unit 700 includes one or more indicators 706, which can be LEDs, and a user interface 708, which can be push buttons, for example. The therapy unit 700 can also have a stimulator 710 with stimulation electronics and may include the capability to measure current and voltage. The therapy unit 700 can also have a battery 712, which may be rechargeable and can be recharged using charging circuitry 714, which may be inductive. The therapy unit 710 may further include a processor 716 and memory 718 to store and execute programs and instructions to accomplish the functions described herein. The therapy unit 710 may also include sensors 720, such as blood pressure, respiratory, heart rate, electrodermal activity, or EEG sensors, and a communications module 722, which may be wireless and can communicate with the base station 704 and/or a secondary display/computing device.

The band 702 can have electrodes 724 and may also include memory to store identification information or may include some other form of identifier 726 as described herein.

The base station 704 can include charging circuitry 728, which may also be inductive and can transmit power to the complementary charging circuitry 714 on the therapy unit 700. The base station 704 can also have a processor and memory for storing and executing instructions and programs. The base station 704 can further include a communication module 732, which may be cellular, to communicate with the cloud, and another communication module 734, which may be wireless and used to communicate with the therapy unit.

In some embodiments, the system can include a heart rate monitor, respiratory sensor, ECG monitor, EEG sensors, or other cardiac monitor worn on the body, or a blood pressure cuff, each which could include an integrated nerve stimulator. In some embodiments, the nerve stimulator and cardiac monitor and/or blood pressure device can be separate devices that communicate wirelessly. In some embodiments, the device can measure cardiac rhythm and/or blood pressure over the course of minutes, hours, days, weeks and/or months to determine whether the patient's cardiac dysrhythmia, cardiac dyssynchrony, and/or blood pressure is increasing, decreasing, or staying the same. In some embodiments, the cardiac rhythm, rate, and/or blood pressure measurements are time averaged over a window, which can be days, weeks, or months. In some embodiments, a sensor, such as a motion sensor, IMU, or GPS, can be used to detect patient activity, which can affect cardiac rhythm, rate, and/or blood pressure measurements. In some embodiments, cardiac rhythm and/or blood pressure measurements are not taken when the patient is active. In some embodiments, cardiac rhythm, rate, and/or blood pressure measurements are only taken when the patient activity sensors determine that the patient is at rest. In some embodiments, the sensor can be an electrode that measures galvanic skin response, which can be correlated to stress, and changes in cardiac rhythm, rate, blood pressure, and/or sympathetic activity. In some embodiments, cardiac rhythm, rate, and/or blood pressure is measured at the same time each day with the same conditions to improve measurement consistency and to reduce variability. In some embodiments, a first stimulator is applied to one wrist or arm to stimulate one peripheral nerve in the arm, such as the median nerve, or specific nerve location, such as an acupressure point or meridians and a second stimulator is applied to one ear to stimulate one peripheral nerve in the ear, such as the auricular branch of the vagus nerve, or specific nerve location, such as an acupressure point or meridians.

In some embodiments, specific fiber types within a nerve or nerves can be selectively activated (e.g., create action potentials in such specific fiber types) to restore autonomic balance by specifically modulating sympathetic and parasympathetic limbs of the autonomic nervous system (e.g., selectively only one, or more than one of A-alpha, A-beta, A-delta, B, and/or C fibers). In some embodiments, systems and methods do not stimulate or substantially stimulate A-alpha, A-beta, A-delta, B fibers, or C fibers.

In general, stimulation of superficial and/or cutaneous afferent and/or efferent nerves can modulate parasympathetic activity by inhibiting the nucleus of the solitary tract and vagal nuclei. Stimulation of deep afferent and/or efferent nerves can modulate sympathetic activity by exciting the arcuate nucleus-ventral periaqueductal gray-nuclei raphe pathway, inhibiting the rostral ventrolateral medulla (rVLM) and thereby the sympathetic outflow. Superficial fibers are finer (e.g., smaller diameter) afferents that relay sensory information to the superficial dorsal horn, which is a distinct region of the dorsal horn and spinal gray matter; deep fibers are thicker (e.g., larger diameter) afferents that relay sensory information to the deep dorsal horn.

In some embodiments, a neurostimulation system regulated by a biological signal can include in-ear electrodes combined or incorporated with an auditory source, such as headphones, to provide auditory guidance to properly use the device, or to provide other forms of therapy or guided activities that can provide a positive therapeutic benefit, including but not limited to guided meditation, calming music, binaural beats, guided breathing exercises, and/or positive affirmations.

In some embodiments, a plurality of neurostimulation systems regulated by a biological signal that provide auditory guidance can be synchronized via wireless communication to coordinate breathing patterns and/or neural oscillations of multiple users in physical proximity or distributed across geographical locations. Synchronizing the breathing patterns and/or neural oscillations of multiple users can enhance the therapeutic benefit or increase the neuroplastic efficiency of the neurostimulation system regulated by a biological signal.

In some embodiments, a neurostimulation system is synchronized, and/or is in communication with a user signaling device configured to provide instructional feedback to a user to guide voluntarily controlled biological rhythmic processes such as breathing or movement such that the phases of the rhythmic process are synchronized with the timing of stimulation delivered to one or more peripheral nerves. The user signaling device could include, for example, a visual display presented on a screen, such as a computer monitor, tablet computer, smart phone, or smart watch; audio instructions; discrete tactile cues on when the user needs to inspire and/or expire, and the like. For example, a computer screen can instruct a user to perform a desired first activity, such as, for example, inhale for a specified duration and then perform a desired second activity, such as, for example, exhale for a specified duration. In some embodiments, a desired third, fourth, etc. activity can be instructed for a third, fourth, and subsequent duration. In one embodiment, the system delivers stimulation to a peripheral nerve only when the user is being instructed to perform the first activity, but not the second activity, such as, for example, exhale or inhale. In an alternative embodiment, the system delivers stimulation to a first peripheral nerve when the user is being instructed to inhale, and the system delivers stimulation to a second peripheral nerve when the user is being instructed to exhale. The activity, e.g., inhalation and exhalation instructions can be present on a display, either as text prompts, or graphics (such as displaying a first color to prompt inspiration, and a second color to prompt expiration as one non-limiting example). In some embodiments, inhalation and exhalation instructions can be presented as audio, either as spoken instructions or changing pitch of a tone (e.g., rising pitch to prompt inhalation and lowering pitch to prompt exhalation). In some embodiments, the stimulation can occur during all, substantially all, or only a portion of the duration of one or more of the activities, such as about, at least about, or no more than about 80%, 60%, 40%, 20%, or more or less of the activity including ranges incorporating any one or more of the foregoing values. In some embodiments, such systems and methods can be advantageous in that they do not necessarily require (in other words, do not comprise) any sensors/device configured to measure respiratory activity. In some embodiments, the system can be configured such that the patient can be prompted by the instructional feedback to perform the first activity for a duration that is the same or different than the second activity, such as inhale or exhale more slowly than normal to enhance an effect, such as, for example, at a respiration rate of about or less than about 16, 14, 12, 10, 8, 6, or less breaths per minute. In some embodiments, the patient can be instructed to perform the first activity for a duration that is about, at least about, or no more than about 10x, 9x, 8x, 7x, 6x, 5x, 4x, 3x, 2.5x, 2x, 1.5x, 1.4x, 1.3x, 1.2x, 1.1x, 1x, 0.9x, 0.8x, 0.7x, 0.6x, 0.5x, 0.4x, 0.3x, 0.2x, 0.1x, or less of the duration the patient is instructed to perform the second activity, or ranges including any two of the foregoing values. The instructional feedback can in some cases instruct the patient to perform biofeedback activities that can, for example, modulate (increase or decrease) sympathetic and/or parasympathetic activity, including coughing, the Valsalva maneuver, carotid sinus massage, holding one's knees against one's chest, and the like. Other instructional feedback activities may include, for example, holding one's breath, hyperventilating, closing one's eyes, opening one's eyes, stretching, lying supine, sitting, standing up, exercising, and the like.

In several embodiments, stimulation regulated by one or more measured biological signals can advantageously have synergistic effects when combined with pharmacotherapy, including pharmacotherapy for mental health disorders, cardiac disorders, pain, and other diseases. The effects can include enhanced response to therapy, a lesser dose of the pharmacotherapy needed to achieve the effects and thus lower adverse reactions, and the like. This is beneficial in some embodiments to reduce the time it takes to achieve a therapeutic effect (e.g., by at least 10%, 25%, 50% or more, or overlapping ranges therein) or lengthens the therapeutic effect (by e.g., by at least 10%, 20%, 40% or more, or overlapping ranges therein), or improve the overall benefit (e.g., larger reduction in pain, blood pressure, heart rate, arrhythmia frequency, and the like).

In some embodiments, therapy can have an unexpectedly synergistic effect when combined with one, two, or more pharmacologic agents such as a rate-control agent (e.g., a beta-blocker such as for example atenolol, metoprolol, propranolol, carvedilol; a calcium-channel blocker such as for example nifedipine, amlodipine, diltiazem, or verapamil; or a cardiac glycoside such as digoxin) and/or an anti-arrhythmic agent (e.g., quinidine, procainamide, disopyramide, lidocaine, mexiletine, flecainide, propafenone, sotalol, ibutilide, dofetilide, amiodarone or dronedarone). In some embodiments, a cardiac glycoside such as digoxin can be administered orally, intravenously, or another route along with peripheral nerve stimulation protocols such as described herein for an unexpected synergistically beneficial effect in treating cardiac arrhythmias, cardiac dyssynchrony, and/or hypertension. Not to be limited by theory, digitalis glycosides and cardiac glycosides, sometimes referred to as digoxin or deacetyllanatoside C., can modulate arterial baroreflex mechanisms in humans. A diminishing of the baroreceptor reflex can lead to continuous and excessive sympathetic activity, which in turn can lead to an increase in heart rate, blood pressure, and the initiation and maintenance of cardiac dysrhythmias. Abnormal baroreceptor function can be related to elevated activation of the sodium-potassium ATPase pump; digitalis glycosides and cardiac glycosides act to decrease this elevated activation, which leads to increased sensitivity of the baroreceptors, including sensitivity to stimulation. Thus, electrical stimulation of peripheral nerves that modulates the baroreceptors, e.g., median, radial, ulnar or cutaneous fibers of the arm, can have an unexpectedly synergistic effect with digitalis glycosides and cardiac glycosides to inhibit elevated sympathetic activity; the glycosides increase sensitivity of the baroreceptor reflex, and stimulation activates the baroreceptor reflex. This synergistic effect can be advantageous by reducing the required dosage of the glycosides to treat cardiac dysfunction, such as hypertension or cardiac dysrhythmias, as the therapeutic index of digoxin is very narrow and severe toxic effects may occur at plasma concentrations only twice the therapeutic plasma concentration range. In some embodiments, the dose of cardiac glycoside, such as digoxin, administered to the patient can be much less than conventionally prescribed, such as about or less than about 3, 2.8, 2.6, 2.4, 2.2, 2.0, 1.8, 1.6, 1.4, 1.2, 1.0, 0.8, 0.6, 0.4 or 0.2 mcg/kg per day. In some embodiments, the dose of cardiac glycoside can be titrated to a blood level that is less than therapeutic, for example, about or less than about 1.5, 1.4, 1.3, 1.2, 1.1, 1.0, 0.9, 0.8, 0.7, 0.6, 0.5, 0.4, 0.3, 0.2, or 0.1 ng/ml. In some embodiments, the digoxin is provided in single-dose forms of administration (e.g., a tablet) of about or less than about 250mcg, 125mcg, 62.5mcg, 31.25mcg, 16mcg, 8mcg, 4mcg, 2mcg, 1mcg, or less.

In some embodiments, therapy can have an unexpectedly synergistic effect when combined with one, two, or more pharmacologic agents related to mental health improvement such as, for example, anti-depressants, mood-stabilizing agents, anti-anxiety medications, anti-psychotic medications, pain control medications, and anti-migraine medications, for example.

The anti-depressant pharmacologic agent could include, for example, a tricyclic or tetracyclic antidepressant (TCA), a selective serotonin reuptake inhibitor (SSRI), and/or a monoamine oxidase inhibitor. A TCA could include, for example, one or more of: amitriptyline, amoxapine, desipramine, doxepin, imipramine, nortriptyline, protriptyline, trimipramine, and maprotiline. A SSRI could include, for example, one or more of citalopram, escitalopram, fluoxetine, paroxetine, sertraline, vilazodone, and/or fluvoxamine. A MAO inhibitor could include, for example, a non-selective MAO inhibitor (e.g., a hydrazine including iscocarboxazid, nialamide, phenelzine, hydracarbazine; a non-hydrazine including tranylcypromine; a selective MAO-A inhibitor including bifemelane, moclobemide, pirlindole, and toloxatone; and/or a selective MAO-B inhibitor such as rasagiline, selgiline, and safinamide.

An anti-anxiety pharmacologic agent could include, for example, a benzodiazepine, a non-benzodiazepine hypnotic agent, a barbiturate, an anti-epileptic agent, and/or the like. A benzodiazepine could include, for example, one or more of: alprazolam, flunitrazepam, chlordiazepoxide, clonazepam, diazepam, lorazepam, midazolam, oxazepam, and prazepam. Other anti-anxiety agents could include buspirone and others.

A non-benzodiazepine hypnotic agent could include, for example, one or more of zolpidem, zaleplon, eszopiclone, zopiclone, ramelteon, and suvorexant. A barbiturate could include, for example, pentobarbital, phenobarbital, secobarbital, allobarbital, amobarbital, aprobarbital, alphenal, barbital, and brallobarbital. Other sleep-inducing agents such as melatonin could also be utilized.

A mood-stabilizing agent and/or anti-convulsant agent could include any of lithium, valproic acid, carbamazepine, levetiracetam, phenytoin, oxcarbamazepine, and lamotrignine.

An anti-psychotic agent could include one or more of a phenothiazine, a thioxanthene, an atypical or other antipsychotic agent, including but not limited to: prochlorperazine, chlorpromazine, fluphenazine, trifluroperazine, perphenazine, thioridazine, mesoridazine, thiothixene, clozapine, olanzapine, aripiprazole, pimavanserin, asenapine, quetiapine, risperidone, lurasidone, cariprazine, paliperidone, brexpiprazole, iloperidone, ziprasidone, loxapine, haloperidol, molindone, and pimozide.

A pain-control agent could include opiate and opioid and non-opiate agents. An opiate or opioid agent could include, for example, morphine, hydromorphone, thebaine, codeine, fentanyl, tramadol, oxycodone, hydrocodone, meperidine, pethidine, levorphanol, tapentadol, and dextropropxyphene. Non-opiate agents could include gabapentin and pregabalin.

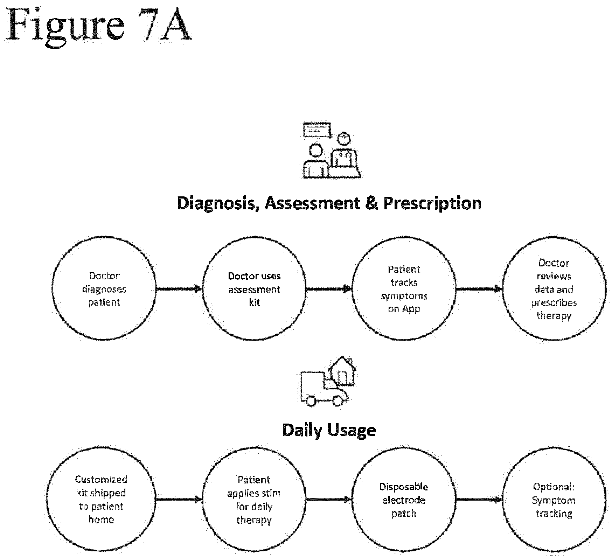

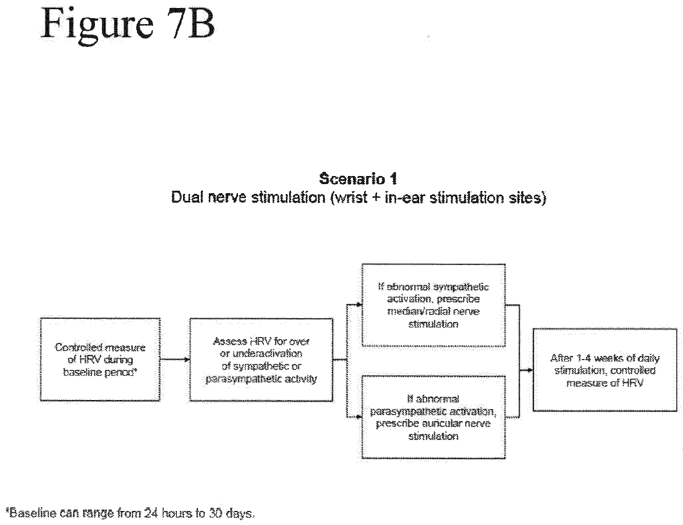

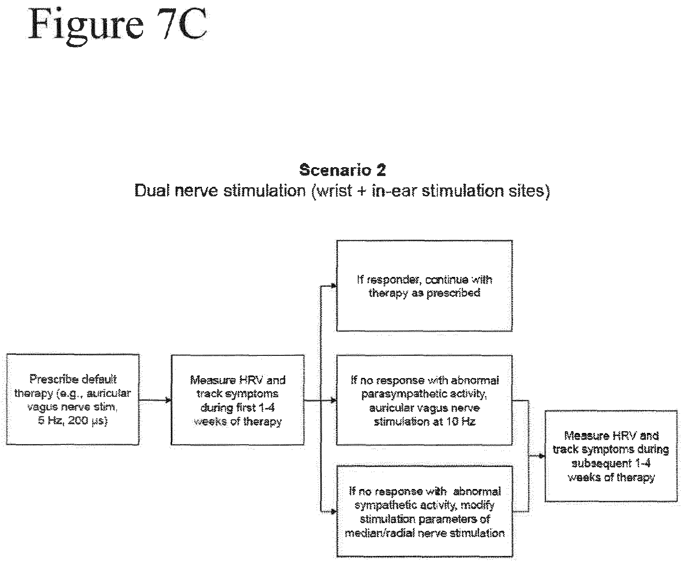

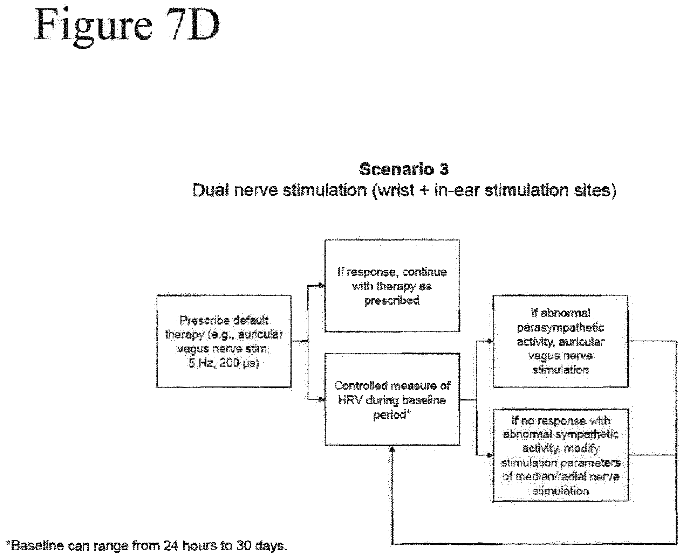

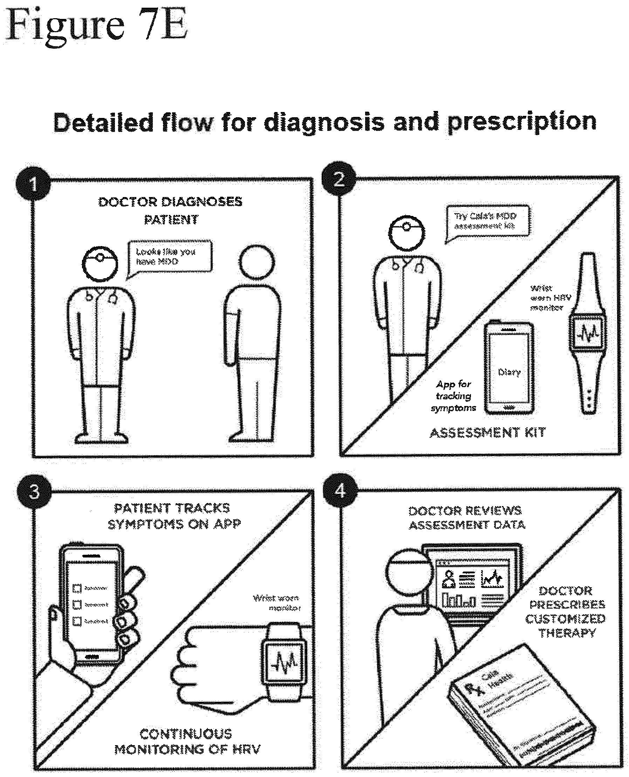

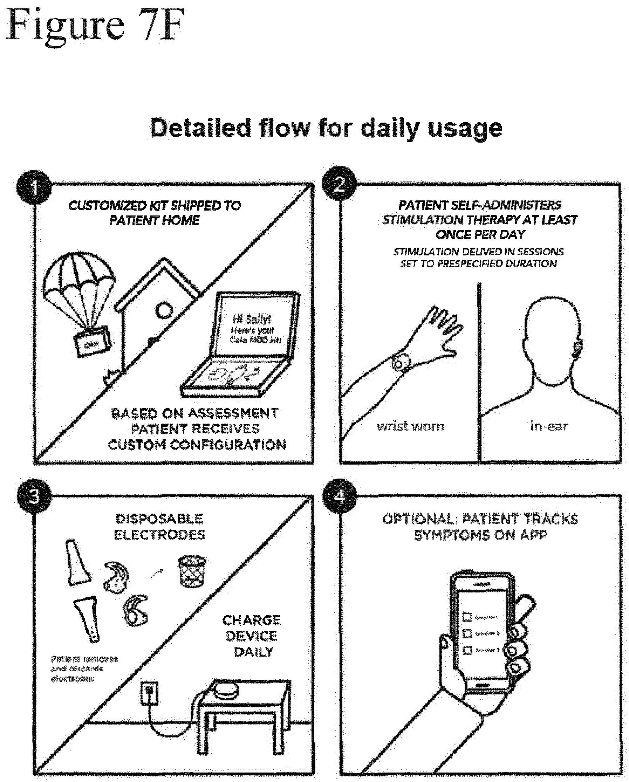

FIGS. 7A-7F schematically illustrate flow charts and detailed flow scenarios incorporating a stimulation protocol, according to some embodiments of the invention, including a sample diagnosis, prescription, and usage workflow. A physician can diagnose a patient with a disorder, such as clinical depression or another disease as disclosed elsewhere herein for example. The physician can utilize an assessment kit; and patient can track symptoms on a software app or other log, as well as via sensors, e.g., HRV or others as disclosed herein. The physician can then review the data and prescribe an appropriate therapy. A customized or individualized kit can then be provided to the patient, who can apply the neuromodulation device, which can include a device in the form on a disposable electrode patch in some cases. The indicators of symptoms, including mood and/or behavior of the patient over the day, such as smart phone usage or physical activity, can be recorded manually or electronically, such as in a software application. These indicators can be used to predict when a person is having a depressive episode and the amount of treatment can be applied accordingly. This processing and consolidation of data to anticipate the amount and timing of treatment necessary can be done within a single device or utilizing another separate device, for instance a mobile phone. In this manner, stimulation can be applied accordingly based on the number of episodes a person experiences. One method of recording indicators of mood and/or behavior or other symptoms associated with depression or mental health disorders is through a journal or diary, for example on a smartphone, tablet, or other device.

In some embodiments, the systems and methods use one or more sensor devices to measure or detect breathing activity, heart rate, or blood flow pulsatility over time, then based on a predetermined relation of the measured activity, a stimulator is instructed to provide neurostimulation to at least one, two, or more of the nerve targets described. In some embodiments, the stimulated nerve targets may selectively activate the parasympathetic nervous system, the sympathetic nervous system, or both.

FIG. 11 illustrates a flow chart of an example of a therapeutic protocol for treating a disorder, according to some embodiments of the invention. In some embodiments, sympathetic and parasympathetic activity can be assessed using sensors that measure heart rate and heart rate variability. Heart rate and HRV can be measured in various ways, including an optical sensor in a wrist worn device, a chest strap or patch that measures changes in electrical activity, a pulse oximeter worn on the finger, and the like. Sympathetic and parasympathetic activity can also be measured using electrodermal activity sensors as described elsewhere herein. In some embodiments, a single device can include both an optical heart rate sensor and electrodermal activity sensors to improve the estimation of sympathetic and parasympathetic activity. If sympathetic overactivation is identified (e.g., from HRV and/or other autonomic measurements), median nerve stimulation can be initiated (e.g., median nerve stimulation alone without auricular branch of the vagus nerve stimulation). If parasympathetic overactivation is identified, auricular branch of the vagus nerve stimulation can be initiated (e.g., auricular branch of the vagus nerve stimulation alone without median nerve stimulation).

In some embodiments, sympathetic and parasympathetic activity are assessed prior to initial stimulation to select specific nerve targets, stimulation waveforms, stimulator parameters, or dosing of stimulation (e.g., time of day, duration of stimulation, number of times per day or week). In other embodiments, a default stimulation is applied in a trial fashion, and only if a person does not respond to treatment is sympathetic and parasympathetic activity assessed. In some embodiments, sympathetic and parasympathetic activity are assessed over a single day or over multiple days during an initial period of treatment to measure any changes in autonomic activity. In some embodiments, depression or other symptoms may be tracked by the patient, either manually or on paper, onboard the stimulation device, or on an external computing device such as a smartphone, tablet, laptop, etc. to be correlated with parameters, such as HRV and changes in autonomic activity, for example.

In some embodiments, if a person does not respond to therapy, a number of parameters can be altered to modify therapy, including but not limited to increasing or decreasing, or otherwise changing any number of the following: duration of session (e.g., 20-120 minutes); number of sessions per day or week (e.g., 2 times per day to 3 times per week); time of day or night of stimulation; stimulation frequency; bursting or other stimulation pattern (including bursting frequency); nerve target (e.g., saphenous or tibial); synchronization with respect to the respiratory cycle, and/or stimulation amplitude.

The following are only examples of disorders that may be treated by any of the methods disclosed herein. Psychiatric conditions with a neurological component (such as neurotransmitter dysfunction) may be treated in some embodiments. Migraine may also be treated. In some embodiments, psychiatric disorders that can be treated include mood disorders, anxiety disorders, schizophrenia and other psychotic disorders, substance-related disorders, sleep disorders, somatoform disorders, and eating disorders. Mood disorders may include depressive disorders and bipolar disorders. They can further be characterized as major depressive disorders, dysthymic disorder, bipolar I disorder, bipolar II disorder, cyclothymic disorder, bipolar disorder not otherwise specified, mood disorders due to a medical condition, substance-induced mood disorder, or mood disorder not otherwise specified. Anxiety disorders can include panic disorder, specific phobia, social phobia, obsessive-compulsive disorder, posttraumatic stress disorder, acute stress disorder, generalized anxiety disorder, anxiety disorder due to a medical condition, substance induced anxiety disorder and anxiety disorder not otherwise specified. Substance-related disorders include substance dependence, substance addiction, substance-induced anxiety disorder, and substance-induced mood disorder. Substance dependence and addiction can occur with a variety of substances, including but not limited to, alcohol, nicotine, cocaine, opioids, narcotics, hallucinogens, amphetamines, phencyclidines, phencyclidine-like substances, inhalants, and sedatives. Substance-induced anxiety disorder can occur in response to substances which include, but not limited to, caffeine, cannabis, cocaine, hallucinogens, amphetamines, phencyclidines, phencyclidine-like substances, and inhalants. Substance-induced mood disorder can occur in response to substances which include, but not limited to cocaine, hallucinogens, opioids, amphetamines, phencyclidines, phencyclidine-like substances, and inhalants. Substance-related disorders can occur in response to one substance or to a combination of substances, such as in polysubstance-related disorder. Eating disorders can include anorexia nervosa, bulimia nervosa, and eating disorders not otherwise specified. These eating disorders may include binge eating. In some embodiments, methods provided can be used to treat subjects experiencing intermittent excessive behaviors (IEB). IEB characterize a variety of disorders including, binge eating, substance abuse, alcoholism, aberrant sexual conduct, and compulsive gambling. In certain embodiments, methods provided may not include the treatment of somatoform disorders. In certain embodiments, methods provided may include somatoform disorders but do not include the treatment of physical pain. In still other embodiments, methods provided may include the treatment of the psychiatric illness associated with pain.

In some embodiments, a systems and methods to use a neurostimulation regulated by a measured biological signal can be utilized for treating cardiac disorders including dysrhythmia, including but not limited to atrial fibrillation (such as chronic or paroxysmal atrial fibrillation) and other arrhythmias, and/or reducing cardiac dyssynchrony and/or blood pressure abnormalities (e.g., hypertension or hypotension, such as autonomic or orthostatic hypotension). Other non-limiting examples of arrhythmias that can be treated using systems and methods as disclosed herein can include, for example, long QT syndrome, torsades de pointes, premature atrial contractions, wandering atrial pacemaker, multifocal atrial tachycardia, atrial flutter, supraventricular tachycardia (including PSVT), AV nodal reentrant tachycardia, junctional rhythm, junctional tachycardia, premature junctional complex, premature ventricular contractions, accelerated idioventricular rhythm, monomorphic ventricular tachycardia, polymorphic ventricular tachycardia, and ventricular fibrillation. Targeting those specific nerves and utilizing appropriately customized stimulation results in more effective therapy (e.g., reduced arrhythmia episodes such as fibrillations or fibrillation episodes and/or shorter duration of fibrillation episodes; reduced palpitations/sensation of arrhythmias; improved rate control of arrhythmias such as a decrease in heart rate of about or at least about 10%, 20%, 30%, 40%, or more compared to pre-treatment (with or without cessation of the arrhythmia); prevention or reduction in the rate of embolic events such as stroke associated with atrial fibrillation; and/or modulation, e.g., decreasing systolic, diastolic, and/or mean blood pressure). In some embodiments, therapy can prevent or reduce the recurrence rate of fibrillation in people with persistent atrial fibrillation (AF) after pharmaco- or electro-cardioversion; or the number and duration of fibrillation episodes in paroxysmal AF patients, including but not limited to reducing the number of arrhythmia recurrent episodes after an ablation procedure. In some embodiments, therapy can reduce or eliminate the number, dose, and/or frequency of medications that a patient may need to take for their underlying arrhythmia, advantageously reducing side effects/potential toxicities.

In some embodiments, systems and methods to use a neurostimulation regulated by a measured biological signal systems and methods can be utilized for treating bladder disorders, including, urinary dysfunction (including overactive bladder, nocturia, and/or stress and urge incontinence) as well as fecal incontinence.

In some embodiments, systems and methods to use a neurostimulation regulated by a measured biological signal systems and methods can be utilized to treat inflammatory diseases, autoimmune diseases, and/or diseases involving the gastrointestinal tract, and in some cases specifically to systems and methods of treating inflammatory bowel diseases, including but not limited to ulcerative colitis, Crohn's disease, and microscopic colitis, through neuromodulation (such as noninvasive peripheral nerve stimulation). Microscopic colitis includes, for example, collagenous colitis and lymphocytic colitis.

In some embodiments, other gastrointestinal-related diseases including irritable bowel syndrome, autoimmune gastrointestinal dysmotility (AGID), esophagitis, gastritis, duodenitis, ischemic colitis, celiac sprue, Whipple's disease, peptic ulcer disease, pancreatitis, hepatitis including autoimmune hepatitis, non-alcoholic steatohepatitis (NASH), cholecystitis, primary biliary cirrhosis, primary sclerosing cholangitis, hemochromatosis, and Wilson's disease can be treated using systems and methods as disclosed herein.

Inflammation of the gastrointestinal tract (e.g., colon and/or small intestine, including the duodenum, jejunum, and ileum; stomach; and/or the esophagus) is treated in several embodiments. In some embodiments, gastric acid and/or bile secretion and/or absorption is regulated via neuromodulation to, for example, beneficially affect intestinal inflammation and/or motility. In some embodiments, pancreatic enzyme secretion is altered to treat IBD, IBS and other GI conditions such as disclosed herein. In some embodiments neuromodulation as disclosed herein can modulate (increase or decrease) the release or effects of a GI-related hormone, peptide, chemical, or other agent including but not limited to bile acid, pancreatic enzyme, gastrin, secretin, cholecystokinin (CCK), vasointestinal peptide (VIP), enterocrinin, motilin, vilikinin, somatostatin, and/or gastric acid, by about or at least about 5%, 10%, 15%, 20%, 25%, 30%, 35%, 40%, 45%, 50%, 60%, 70%, 80%, 90%, 100%, or more, or incorporating two of the aforementioned values. In some embodiments, neuromodulation can treat or prevent fecal incontinence, or increase or decrease GI motility to treat, for example, gastroparesis, ileus, large or small bowel obstruction, constipation, or diarrhea.

In some embodiments, systems and methods as disclosed herein can treat systemic autoimmune diseases, including but not limited to, rheumatoid arthritis, systemic lupus erythematosus, alopecia areata, ankylosing spondylitis, antiphospholipid syndrome, autoimmune Addison's disease, autoimmune hemolytic anemia, autoimmune hepatitis, autoimmune inner ear disease, autoimmune lymphoproliferative syndrome (alps), autoimmune thrombocytopenic purpura (ATP), Behcet's disease, bullous pemphigoid, cardiomyopathy, celiac sprue-dermatitis, chronic fatigue syndrome immune deficiency, syndrome (CFIDS), chronic inflammatory demyelinating polyneuropathy, cicatricial pemphigoid, cold agglutinin disease, Crest syndrome, Crohn's disease, Degos disease, dermatomyositis, juvenile dermatomyositis, discoid lupus, essential mixed cryoglobulinemia, fibromyalgia-fibromyositis, Graves' disease, Guillain-Barre syndrome, Hashimoto's thyroiditis, idiopathic pulmonary fibrosis, idiopathic thrombocytopenia purpura (ITP), IgA nephropathy, insulin dependent diabetes (Type I), juvenile rheumatoid arthritis (JRA), Meniere's disease, mixed connective tissue disease, multiple sclerosis, myasthenia gravis, pemphigus vulgaris, pernicious anemia, polyarteritis nodosa, polychondritis, polyglandular syndromes, polymyalgia rheumatica, polymyositis and dermatomyositis, primary agammaglobulinemia, primary biliary cirrhosis, psoriasis, Raynaud's phenomenon, Reiter's syndrome, rheumatic fever, sarcoidosis, scleroderma, Sjogren's syndrome, stiff-man syndrome, Takayasu arteritis, temporal arteritis/giant cell arteritis, ulcerative colitis, uveitis, vasculitis, vitiligo, and Wegener's granulomatosis.

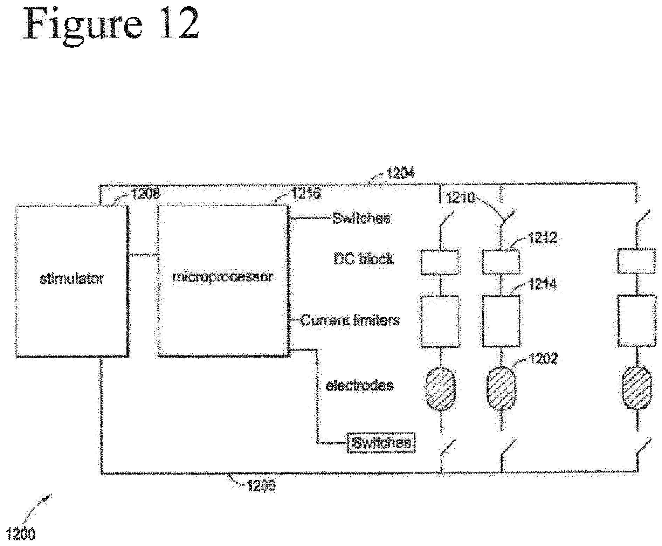

In some embodiments, stimulating three or more electrodes can be used to stimulate two or more nerves or dermatomes. In some embodiments as shown in FIG. 12 the electronics and electrical circuit 1200 used to drive the array can include an adaptable switch that allows each individual electrode 1202 to be connected to either one of the two contacts 1204, 1206 of the stimulator 1208 at a given time by opening or closing switches 1210 in each channel. Each channel can include a DC blocking circuit 1212, as charge balance can be important to prevent skin irritation and burns, and also be individually current limited by current IO limiters 1214 in order to prevent current surges that could cause injury or discomfort. This current limitation can be set to a predetermined tolerability threshold for a particular patient or group of patients.

There are many transistor circuits or components like polyfuses to limit or shutdown the current to a particular node. These circuits and its components, such as the stimulator, switches, and current limiters, can be controlled and/or be programmable by a microprocessor 1216 in real-time. The 15 switch matrix allows multiple electrodes to be connected to the same stimulator contacts at a given time for maximum flexibility. In addition, electrodes can be switched between the positive and negative contacts of the stimulator to produce a bipolar pulse.

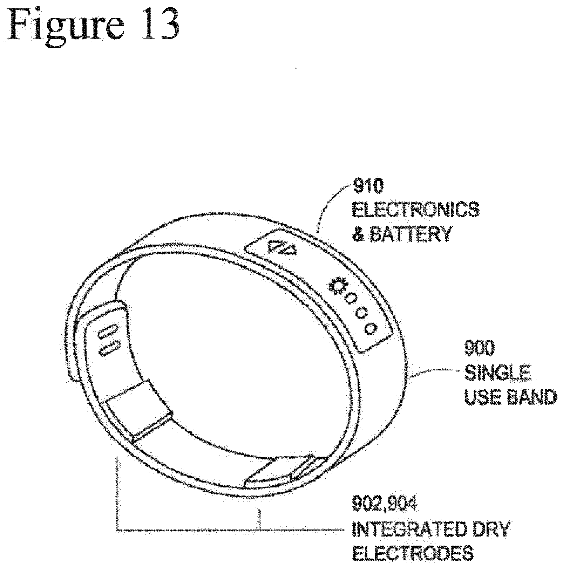

FIG. 13 shows an embodiment of a wearable band 900 with integrated electrodes 902, 904. The integrated electrodes 902, 904 can be dry electrodes in electrical communication with a detachable controller 910 through a flexible circuit embedded in the band. In some cases, dry electrodes may be more suitable for longer term use electrodes that can be used for months, such as at least 1, 2, or 3 months, before the band needs to be replaced. In some embodiments, the band may be a single use band that can be used for a relatively long period of time before replacement.

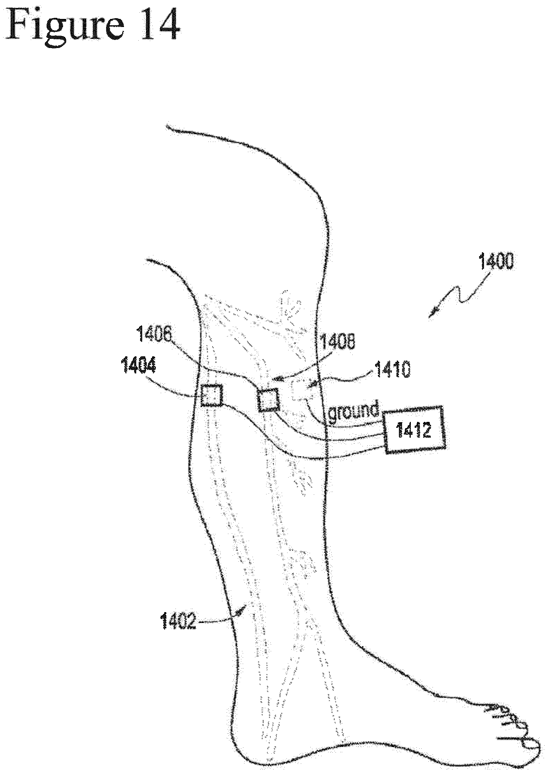

In some embodiments, disclosed herein are systems and methods for stimulating a plurality of nerves for the treatment of a disease associated with imbalance of the autonomic nervous system, or another disease described herein. Stimulation of 2, 3, or more nerves or dermatomes, such as the median, median cutaneous, radial, and/or ulnar nerve could be used for the treatment of conditions such as cardiac dysrhythmia. Dual nerve stimulation can in some cases synergistically increase the effectiveness of therapy by an effect at a nerve plexus or nerve ganglia, such as the brachial plexus, sympathetic ganglia, or a proximal location where individual nerves converge near the spinal cord. For example, in one embodiment, the devices disclosed herein are used to stimulate two nerves (including but not limited to the median, radial, ulnar, or median cutaneous nerve) located at a distance from the brachia! plexus at two different times, wherein, ultimately, the brachial plexus is stimulated by both signals from two or more nerves substantially simultaneously (e.g., less than about 2 ms, 1 ms, 0.5 ms, 0.4 ms, 0.3 ms, 0.2 ms, 0.1 ms, 0.09 ms, 0.08 ms, 0.07 ms, 0.06 ms, 0.05 ms, 0.04 ms, 0.03 ms, 0.02 ms, 0.01 ms, or less), but could be higher in some cases. In one embodiment, the two nerves are offset (in terms of timing of stimulation) by 0.1-3.0 ms. In one embodiment, two, three, four or more nerves located at a distance from a target (including but not limited to the brachial plexus) are stimulated at different times in order to hit the target at substantially the same time. In some embodiments, including those disclosed in connection with FIG. 14 below, the system can be configured to independently control stimulation of a first target nerve (including stimulation parameters such as frequency and others listed herein) and a second target nerve respectively. In other words, the first target nerve and the second target nerve can be stimulated with either the same or different parameters, and can be stimulated simultaneously or in alternating or other fashion. In some embodiments, the stimulation systems can include a plurality of independent stimulation circuits, or a common circuit with a controller configured to switch stimulation parameters for one, two, or more nerves.

In some embodiments, as illustrated schematically in FIG. 14, a system 1400 can utilize three electrodes: a first electrode 1404 positioned over a first nerve, e.g., the tibial nerve 1402, a second electrode 1406 positioned over a second nerve, e.g., the saphenous nerve 1408, and a third electrode 1410 positioned, for example, on the outer side of the leg, opposite to the first two electrodes 1404, 1406. This third electrode 1410 would serve as a common cathode for the other two electrodes 1404, 1406. The three electrodes 1404, 1406, 1410 can be oriented in such a way that the electric fields between each of the first two electrodes 1404, 1406 and the common cathode 1410 pass through the tibial nerve 1402 and saphenous nerve 1408, respectively. In some embodiments, other nerves innervating the leg including, for example, the common peroneal nerve, the femoral nerve, the sacral nerve, the sciatic nerve, and the sural nerve can also be stimulated.

Embodiments of the invention can include a device and system and method to measure and collect biological data (e.g., heart rate, heart rate variability, ECG, galvanic skin response, temperature, and blood pressure), analyze the data as to interpret how these measures may influence cardiac rhythm and/or blood pressure, and provide peripheral nerve stimulation that targets one or more individual nerves, such as the median, ulnar, and/or radial nerve, to treat or prevent cardiac dysrhythmias, reduce cardiac dyssynchrony, and/or reduce blood pressure, where the stimulation applied may or may not be modified based on the measured data.

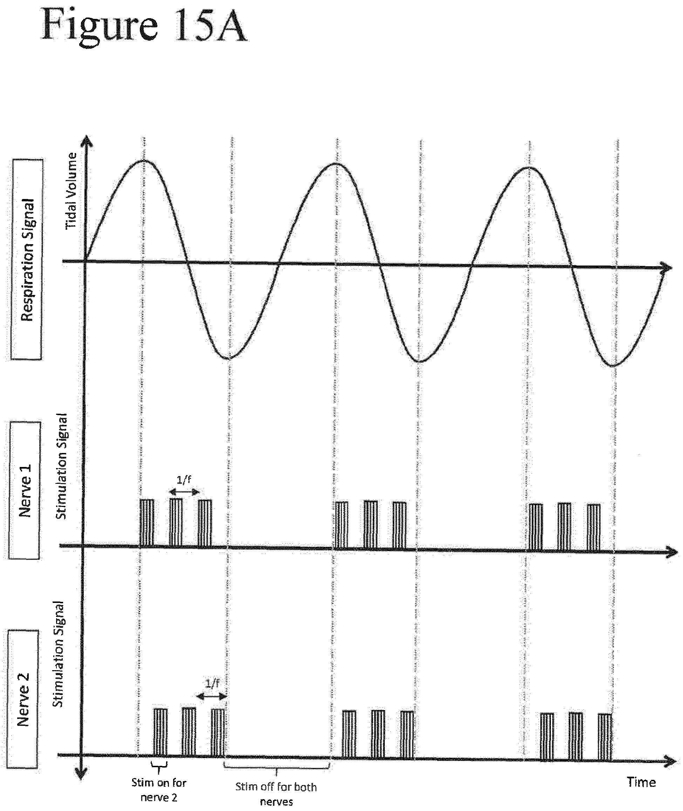

FIG. 15A illustrates an embodiment of a system including a controller configured for the regulation of alternating stimulation on two or more nerves based on the rhythmic biological signals measured, such as, e.g., with two nerves being stimulated at a constant, fixed burst frequency (e.g., *f* = 3 Hz or other frequencies, including those disclosed elsewhere herein) but at alternating times during the expiration phase of respiration (as measured through tidal volume for example). In some embodiments, the nerves are stimulated with the same stimulation parameters (including but not limited to pulse frequency, pulse width and burst frequency) without any offsetting between the two nerves. In other embodiments, the regulation of stimulation is based on other features of the measured rhythmic biological signal, such as at the start of inhalation during respiration.

In some embodiments, the stimulation can be delayed or offset by a predetermined fraction or multiple of a parameter of the measured rhythmic biological signal, such as a dominant frequency for example. The offset or delay between stimulation of different nerves could be, for example, time 1/N wherein N is the number of nerves to be modulated. In some embodiments, the offset or delay is by time T/2, 3T/4, T/4, T/5, T/6, T7, T/8, or other fractions or multiples of a measured parameter, for example.

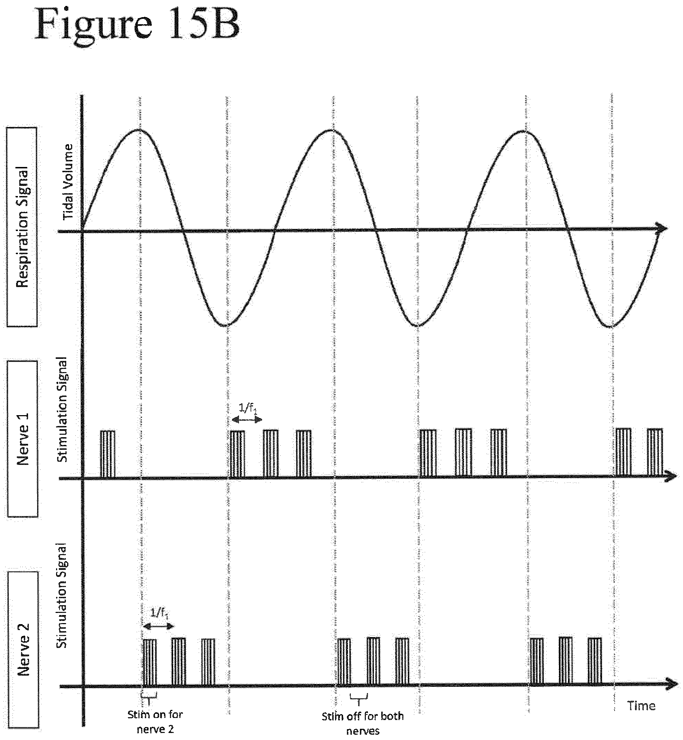

FIG. 15B illustrates an embodiment of a system including a controller configured for the regulation of alternating stimulation on more than one nerve based on different features of the rhythmic biological signal measured, such as with one nerve stimulating at the start of inhalation and another nerve stimulated at the start of expiration as measured through a respiratory signal such as tidal volume, for example.

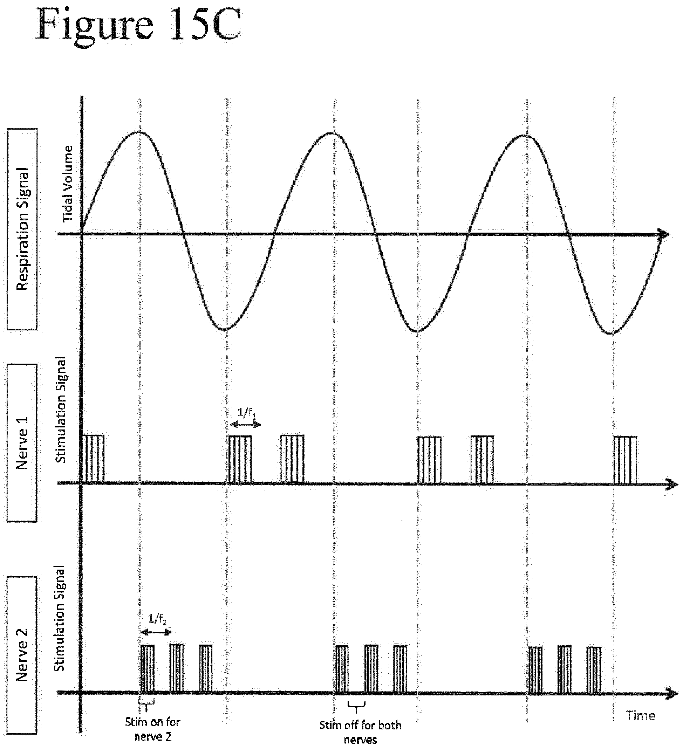

FIG. 15C illustrates an embodiment of a system including a controller configured for the regulation of alternating types of stimulation on more than one nerve based on different features of the rhythmic biological signal measured, such as with one nerve stimulating with at a set burst frequency at the start of inhalation and another nerve stimulated at different burst frequency delivered at the start of expiration as measured through a respiratory signal such as tidal volume, for example.

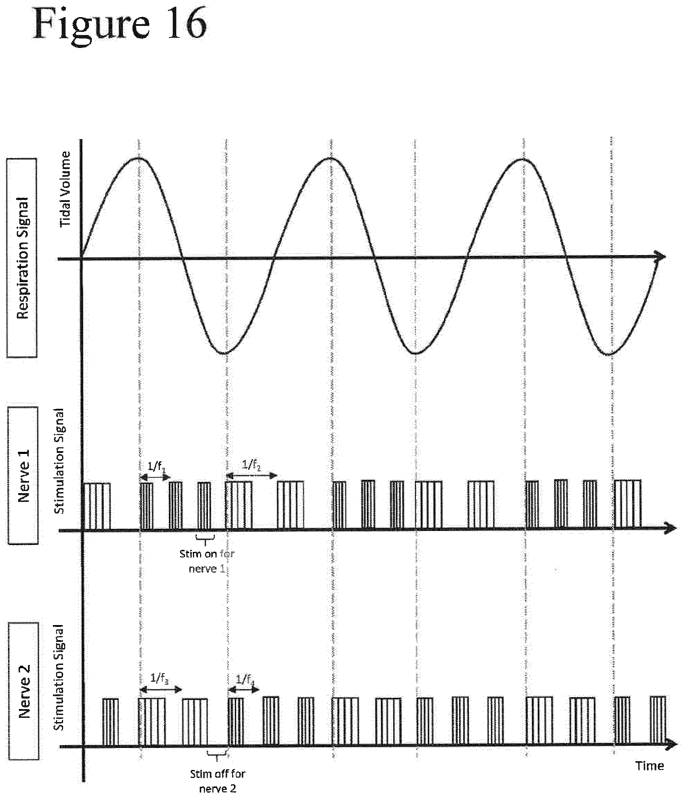

FIG. 16 illustrates an embodiment of a system including a controller configured for determining stimulation parameters (including but not limited to pulse width, pulse frequency, amplitude, intensity, burst duration, and burst frequency) that can be varied on each target nerve based on features of the rhythmic biological signal. For example, stimulation can be applied to each nerve with a unique stimulation parameter, such as burst frequency at the start of inhalation and exhalation based on the respiration.

Various embodiments of various disease altering devices and methods, have been disclosed above. These various embodiments may be used alone or in combination, and various changes to individual features of the embodiments may be altered, without departing from the scope of the invention. For example, the order of various method steps may in some instances be changed, and/or one or more optional features may be added to or eliminated from a described device. Therefore, the description of the embodiments provided above should not be interpreted as unduly limiting the scope of the invention as it is set forth in the claims.

Certain features that are described in this specification in the context of separate embodiments also can be implemented in combination in a single embodiment. Conversely, various features that are described in the context of a single embodiment also can be implemented in multiple embodiments separately or in any suitable subcombination. Moreover, although features may be described above as acting in certain combinations and even initially claimed as such, one or more features from a claimed combination can in some cases be excised from the combination, and the claimed combination may be directed to a subcombination or variation of a subcombination.

The foregoing description and examples have been set forth to illustrate the disclosure according to various embodiments and are not intended as being unduly limiting. The headings provided herein are for organizational purposes only and should not be used to limit embodiments. Each of the disclosed aspects and examples of the present disclosure may be considered individually or in combination with other aspects, examples, and variations of the disclosure. In addition, unless otherwise specified, none of the steps of the methods of the present disclosure are confined to any particular order of performance. References cited herein are incorporated by reference in their entirety. The description of an embodiment as "preferred" does not limit the use or scope of alternative embodiments.

While the methods and devices described herein may be susceptible to various modifications and alternative forms, specific examples thereof have been shown in the drawings and are herein described in detail. It should be understood, however, that the embodiments disclosed should cover modifications, equivalents, and alternatives falling within the spirit and scope of the various embodiments described herein and the appended claims.

Depending on the embodiment, one or more acts, events, or functions of any of the algorithms, methods, or processes described herein can be performed in a different sequence, can be added, merged, or left out altogether (e.g., not all described acts or events are necessary for the practice of the algorithm). In some examples, acts or events can be performed concurrently, e.g., through multi-threaded processing, interrupt processing, or multiple processors or processor cores or on other parallel architectures, rather than sequentially.

The use of sequential, or time-ordered language, such as "then," "next," "after," "subsequently," and the like, unless specifically stated otherwise, or otherwise understood within the context as used, is generally intended to facilitate the flow of the text and is not intended to limit the sequence of operations performed.

The various illustrative logical blocks, modules, processes, methods, and algorithms described in connection with the embodiments disclosed herein can be implemented as electronic hardware, computer software, or combinations of both. To clearly illustrate this interchangeability of hardware and software, various illustrative components, blocks, modules, operations, and steps have been described above generally in terms of their functionality. Whether such functionality is implemented as hardware or software depends upon the particular application and design constraints imposed on the overall system. The described functionality can be implemented in varying ways for each particular application, but such implementation decisions should not be interpreted as causing a departure from the scope of the disclosure.

The various illustrative logical blocks and modules described in connection with the embodiments disclosed herein can be implemented or performed by a machine, such as a general purpose processor, a digital signal processor (DSP), an application specific integrated circuit (ASIC), a field programmable gate array (FPGA) or other programmable logic device, discrete gate or transistor logic, discrete hardware components, or any combination thereof designed to perform the functions described herein. A general-purpose processor can be a microprocessor, but in the alternative, the processor can be a controller, microcontroller, or state machine, combinations of the same, or the like. A processor can also be implemented as a combination of computing devices, e.g., a combination of a DSP and a microprocessor, a plurality of microprocessors, one or more microprocessors in conjunction with a DSP core, or any other such configuration.

The blocks, operations, or steps of a method, process, or algorithm described in connection with the embodiments disclosed herein can be embodied directly in hardware, in a software module executed by a processor, or in a combination of the two. A software module can reside in RAM memory, flash memory, ROM memory, EPROM memory, EEPROM memory, registers, hard disk, a removable disk, an optical disc (e.g., CD-ROM or DVD), or any other form of volatile or non-volatile computer-readable storage medium known in the art. A storage medium can be coupled to the processor such that the processor can read information from, and write information to, the storage medium. In the alternative, the storage medium can be integral to the processor. The processor and the storage medium can reside in an ASIC. The ASIC can reside in a user terminal. In the alternative, the processor and the storage medium can reside as discrete components in a user terminal.

Conditional language used herein, such as, among others, "can," "might," "may," "e.g.," and the like, unless specifically stated otherwise, or otherwise understood within the context as used, is generally intended to convey that some examples include, while other examples do not include, certain features, elements, and/or states. Thus, such conditional language is not generally intended to imply that features, elements, blocks, and/or states are in any way required for one or more examples or that one or more examples necessarily include logic for deciding, with or without author input or prompting, whether these features, elements and/or states are included or are to be performed in any particular embodiment.

The methods disclosed herein may include certain actions taken by a practitioner; however, the methods can also include any third-party instruction of those actions, either expressly or by implication. For example, actions such as "positioning an electrode" include "instructing positioning of an electrode."

The ranges disclosed herein also encompass any and all overlap, sub-ranges, and combinations thereof. Language such as "up to," "at least," "greater than," "less than," "between," and the like includes the number recited. Numbers preceded by a term such as "about" or "approximately" include the recited numbers and should be interpreted based on the circumstances (e.g., as accurate as reasonably possible under the circumstances, for example ±5%, ±10%, ±15%, etc.). For example, "about 1 hour" includes "1 hour." Phrases preceded by a term such as "substantially" include the recited phrase and should be interpreted based on the circumstances (e.g., as much as reasonably possible under the circumstances). For example, "substantially perpendicular" includes "perpendicular." Unless stated otherwise, all measurements are at standard conditions including temperature and pressure. The phrase "at least one of" is intended to require at least one item from the subsequent listing, not one type of each item from each item in the subsequent listing. For example, "at least one of A, B, and C" can include A, B, C, A and B, A and C, B and C, or A, B, and C.
Furthermore, the following Aspects 1-112 are explicitly disclosed in connection with the foregoing disclosure:

### Aspects:

1. A method of transcutaneously stimulating one or more peripheral nerves of a patient with a wearable neurostimulation device regulated by a measured rhythmic biological signal to treat a patient, comprising:
   positioning a first peripheral nerve effector proximate a skin surface on a first ear of the patient to stimulate a first peripheral nerve of the patient;
   positioning a second peripheral nerve effector proximate a skin surface proximate a first or a second ear of the patient to stimulate a second peripheral nerve of the patient;
   measuring a rhythmic biological signal of the patient and determining one or more features of the measured rhythmic biological signal of the patient in real-time;
   delivering a first electrical nerve stimulation signal transcutaneously to the first peripheral nerve effector to stimulate the first peripheral nerve sufficient to modify a first neural circuit to modulate nervous system activity of the patient at least in part based on the measured rhythmic biological signal; and
   delivering a second electrical nerve stimulation signal transcutaneously to the second peripheral nerve effector to stimulate the second peripheral nerve sufficient to modify the first or a second neural feedback loop to modulate nervous system activity of the patient at least in part based on the measured rhythmic biological signal,
   wherein the first peripheral nerve is the auricular vagus nerve, and the second peripheral nerve is a nerve other than the auricular vagus nerve,
   wherein the second electrical nerve stimulation signal is offset at a preselected time interval from the first electrical stimulation signal.
2. The method of Aspect 1, wherein the features of the measured rhythmic biological signal comprise the phase, frequency, magnitude, or timing of the measured rhythmic biological signal.
3. The method of Aspects 1 or 2, further comprising adjusting one or more of the first electrical stimulation signal and the second electrical stimulation signal based on the one or more features of the measured rhythmic biological signal.
4. The method of Aspects 1 or 2, further comprising adjusting the first electrical stimulation signal and the second electrical stimulation signal based on the one or more features of the measured rhythmic biological signal.
5. The method of Aspects 1 or 2, further comprising adjusting the first electrical stimulation signal based on a first feature of the measured rhythmic biological signal, and adjusting the second electrical stimulation signal based on a second feature of the measured rhythmic biological signal.
6. The method of Aspects 1 or 2, wherein the first peripheral nerve and the second peripheral nerve are located on the first ear of the patient.
7. The method of Aspects 1 or 2, wherein the first peripheral nerve is located on the first ear of the patient and the second peripheral nerve is located on the second ear of the patient.
8. The method of Aspect 1, further comprising monitoring sympathetic and parasympathetic activity in the patient.
9. The method of Aspect 8, wherein monitoring sympathetic and parasympathetic activity comprises receiving data from a sensor that measures heart rate variability.
10. The method of Aspect 9, wherein monitoring sympathetic and parasympathetic activity comprises receiving data from a sensor that electrodermal activity.
11. The method of Aspect 9, wherein measuring heart rate variability also comprises determining a ratio of absolute low frequency to absolute high frequency of heart rate variability of the patient.
12. The method of Aspect 8, wherein monitoring sympathetic and parasympathetic activity comprises receiving data from a sensor that measures at least one of electrodermal activity, heart rate variability, thermometry, pupillometry and electrocardiogram information of the patient.
13. The method of Aspect 8, further comprising adjusting the first electrical nerve stimulation signal upon identifying abnormal sympathetic activity in the patient.
14. The method of Aspect 8, further comprising adjusting the second electrical nerve stimulation signal upon identifying abnormal parasympathetic activity in the patient.
15. The method of Aspect 1, wherein at least one of the first electrical nerve stimulation signal and the second electrical nerve stimulation signal comprises burst stimulation.
16. The method of Aspect 15, wherein the burst stimulation comprises theta burst stimulation.
17. The method of Aspect 16, wherein the theta burst stimulation is preceded by a priming stimulation with a waiting period between priming and theta burst stimulation between 1 - 30 minutes.
18. The method of Aspect 1, wherein the measured rhythmic biological signal is one selected from the group consisting of a breathing cycle, cardiac rhythm cycle, sympathetic tonic rhythmic discharge, sleep-wake cycle, menstrual cycle, and cortical brain rhythm patterns.
19. The method of Aspect **1,** further comprising neuromodulating one or more additional nerves selected from the group consisting of the trigeminal nerve, greater auricular nerve, auriculotemporal nerve, and the lesser occipital nerve.
20. The method of Aspect 18, wherein measuring the breathing cycle comprises assessing at least one of chest wall and abdominal wall movement of the patient.
21. The method of Aspect 18, wherein measuring the breathing cycle comprises assessing carbon dioxide levels associated with the patient's breathing.
22. The method of Aspect 18, wherein measuring the breathing cycle comprises assessing oxygen levels associated with the patient's breathing.
23. The method of Aspect 18, wherein measuring the breathing cycle comprises assessing temperature levels associated with the patient's breathing.
24. The method of Aspect 18, wherein measuring the breathing cycle comprises assessing sounds associated with the patient's breathing via a microphone.
25. The method of Aspect 18, wherein measuring the cardiac rhythm cycle comprises assessing heart rate.
26. The method of Aspect 18, wherein measuring the cardiac rhythm cycle comprises assessing electrocardiogram data.
27. The method of Aspect 18, wherein measuring the cardiac rhythm cycle comprises assessing photoplethysmography.
28. The method of Aspect 18, wherein measuring cortical brain rhythm patterns comprises assessing electroencephalogram data.
29. The method of any of the preceding claims, for treating a mood disorder.
30. The method of Claim 29, wherein the mood disorder is selected from the group consisting of depression, bipolar disorder, anxiety disorder, mania, and schizoaffective disorder.
31. The method of any of Claims 1-28, for treating a cardiovascular disorder.
32. The method of Claim 31, wherein the cardiovascular disorder is selected from the group consisting of hypertension, coronary artery disease, angina pectoris, congestive heart failure, a cardiac dysrhythmia, and a cardiac dyssynchrony.
33. The method of any of Aspects 1-28, for treating an inflammatory disorder.
34. The method of any of Aspects 1-28, for treating a neurologic disorder selected from the group consisting of Parkinson's disease, a sleep disorder, and another neurologic disorder.
35. The method of any of Aspects 1-28, for treating a urologic disorder.
36. The method of Aspect **1,** further comprising stimulating the first peripheral nerve during a first phase of the rhythmic biologic signal, and stimulating the second peripheral nerve during a second phase of the rhythmic biological signal.
37. The method of Aspect 36, wherein the first peripheral nerve is associated with the parasympathetic nervous system, and the second peripheral nerve is associated with the sympathetic nervous system.
38. The method of Aspect 37, wherein the first phase is expiration, and the second phase is inspiration.
39. A wearable neurostimulation system for transcutaneously stimulating peripheral nerves of a patient regulated by a rhythmic biological signal, comprising:
   a first peripheral nerve effector configured to be positioned proximate a first location on the skin surface on a first ear of the patient;
   a second peripheral nerve effector configured to be positioned at a second location of the patient proximate the first ear or a second ear of the patient;
   a first biomedical sensor configured to detect activity relating to a rhythmic biological signal of the patient and convert the detected activity into a corresponding rhythmic detection signal;
   a controller in operable communication with the first biomedical sensor, the controller configured to analyze a rhythmic biological signal and recognize one or more features of the rhythmic biological signal of the patient in real-time,
   wherein the controller is configured to generate a first electrical nerve stimulation signal transcutaneously to the first peripheral nerve effector to stimulate a first peripheral nerve sufficient to modify a first neural feedback loop upon detection of a predetermined feature of the rhythmic biological signal, wherein the first peripheral nerve is the auricular branch of the vagus nerve,
   wherein the controller is configured to generate a second electrical nerve stimulation signal transcutaneously to the second peripheral nerve effector to stimulate a second peripheral nerve other than the auricular branch of the vagus nerve and associated to modify the first or a second neural feedback loop upon detection of the first or a second predetermined feature of the rhythmic biological signal,
   wherein the second electrical nerve stimulation signal is offset at a preselected time interval from the first electrical stimulation signal.
40. The system of Aspect 39, wherein the one or more features of the rhythmic biological signal comprise the phase, frequency, magnitude, or timing of the measured rhythmic biological signal.
41. The system of Aspect 39, wherein the controller is further configured to adjust one or more of the first electrical stimulation signal and the second electrical stimulation signal based on the one or more features of the measured rhythmic biological signal.
42. The system of Aspect 39, wherein the controller is further configured to adjust the first electrical stimulation signal and the second electrical stimulation signal based on the one or more features of the measured rhythmic biological signal.
43. The system of Aspect 39, wherein the controller is further configured to adjust the first electrical stimulation signal based on a first feature of the measured rhythmic biological signal, and adjust the second electrical stimulation signal based on a second feature of the measured rhythmic biological signal.
44. The system of Aspect 39, wherein the first peripheral nerve is associated with a sympathetic nervous pathway of the patient and the second peripheral nerve is associated with a parasympathetic nervous pathway of the patient.
45. The system of Aspect 39, wherein the rhythmic biological signal is a breathing cycle, cardiac rhythm cycle, sympathetic tonic rhythmic discharge, sleep-wake cycle, menstrual cycle, or cortical brain rhythm patterns.
46. The system of Aspect 39, wherein the rhythmic biological signal is the breathing cycle and the first predetermined phase is an exhalation phase of the breathing cycle.
47. The system of Aspect 39, wherein the rhythmic biological signal is the breathing cycle and the second predetermined phase is an inhalation phase of the breathing cycle.
48. The system of Aspect 39, wherein the rhythmic biological signal is the breathing cycle and the first biomedical sensor is operably attached to an effort belt configured to be placed around the rib-cage portion of the torso to detect breathing movements.
49. The system of Aspect 39, wherein the rhythmic biological signal is the breathing cycle and the first biomedical sensor comprises electrodes to evaluate changes in electrical impedance across the torso region over the breathing cycle.
50. The system of Aspect 39, wherein the rhythmic biological signal is the breathing cycle and the first biomedical sensor is operably attached to a nasal or oral patient interface to evaluate pulmonary activity by measuring respiratory flow and continuous expired concentration of carbon dioxide.
51. The system of Aspect 39, wherein the rhythmic biological signal is the breathing cycle and the first biomedical sensor comprises an optical transcutaneous sampling cell for measuring carbon dioxide or oxygen concentration within a blood vessel.
52. The system of Aspect 39, further comprising a second biomedical sensor.
53. The system of Aspect 51, wherein the second biomedical sensor comprises pulse sensors on the extremities to measure blood pressure and derive waveforms to calculate cardiac performance.
54. The system of Aspect 51, wherein the second biomedical sensor comprises electrically conductive sensors on the chest, torso, upper extremities, or in the ear to detect electrocardiogram, cardiac activity, heart rate, or heart rate variability.
55. The system of Aspect 51, where the second biomedical sensor comprises photoplethysmography sensors on the upper extremities or in the ear to detect cardiac activity, heart rate, or heart rate variability.
56. The system of Aspect 51, wherein the controller is configured to adjust the first electrical nerve stimulation signal upon identifying abnormal sympathetic activity in the patient.
57. The system of Aspect 51, wherein the controller is configured to adjust the first electrical nerve stimulation signal upon identifying abnormal parasympathetic activity in the patient.
58. The system of Aspect 51, wherein the controller is configured to adjust at least one of the first electrical stimulation signal and the second electrical nerve stimulation signal based on feedback received regarding the autonomic balance of the patient.
59. A method of transcutaneously stimulating a one or more peripheral nerves of a patient with a wearable neurostimulation device regulated by a measured rhythmic biological signal to treat a patient, comprising:
   positioning a first peripheral nerve effector proximate a skin surface on a first ear of the patient to stimulate a first peripheral nerve of the patient;
   signaling the patient to perform a first voluntary activity associated with a biological process for a first specified duration;
   signaling the patient to perform a second voluntary activity associated with the biological process for a second specified duration; and
   delivering a first electrical nerve stimulation signal transcutaneously to the first peripheral nerve effector to stimulate the first peripheral nerve sufficient to modify a first neural circuit to modulate nervous system activity of the patient during only at least part of the first specified duration,
   wherein the first peripheral nerve is the auricular vagus nerve.
60. The method of Aspect 59, further comprising:
   positioning a second peripheral nerve effector proximate a skin surface of the patient to stimulate a second peripheral nerve of the patient; and
   delivering a second a first electrical nerve stimulation signal transcutaneously to the first peripheral nerve effector to stimulate the second peripheral nerve sufficient to modify a first neural circuit to modulate nervous system activity of the patient during only at least part of the second specified duration.
61. The method of Aspects 59 or 60, wherein the second peripheral nerve is a nerve on an ear of the patient.
62. The method of any of Aspects 59-61, wherein the second peripheral nerve is on a contralateral side of the patient with respect to the first peripheral nerve.
63. The method of any of Aspects 59-61, wherein the second peripheral nerve is on an ipsilateral side of the patient with respect to the first peripheral nerve.
64. The method of any of Aspects 59-63, wherein the method does not comprise sensing any features of the biological process.
65. The method of any of Aspects 59-64, wherein signaling the patient to perform a first voluntary activity comprises displaying information on a screen.
66. The method of any of Aspects 59-64, wherein signaling the patient to perform a first voluntary activity comprises providing audio instructions.
67. The method of any of Aspects 59-64, wherein signaling the patient to perform a first voluntary activity comprises providing tactile cues.
68. The method of any of Aspects 58-67, wherein the biological process comprises respiration.
69. The method of Aspect 68, wherein the first voluntary activity comprises a first phase of respiration.
70. The method of Aspect 68, wherein the first voluntary activity comprises a second phase of respiration.
71. A system for transcutaneously stimulating a one or more peripheral nerves of a patient with a wearable neurostimulation device regulated by a measured rhythmic biological signal to treat a patient, comprising:
   a first peripheral nerve effector configured to be positioned proximate a skin surface on a first ear of the patient to stimulate a first peripheral nerve of the patient;
   a controller configured to signal the patient to perform a first voluntary activity associated with a biological process for a first specified duration; and to perform a second voluntary activity associated with the biological process for a second specified duration; and
   deliver a first electrical nerve stimulation signal transcutaneously to the first peripheral nerve effector to stimulate the first peripheral nerve sufficient to modify a first neural circuit to modulate nervous system activity of the patient during only at least part of the first specified duration,
   wherein the first peripheral nerve is the auricular vagus nerve.
72. The system of Aspect 71, further comprising:
   a second peripheral nerve effector proximate a skin surface of the patient to stimulate a second peripheral nerve of the patient; and
   wherein the controller is further configured to deliver a second a first electrical nerve stimulation signal transcutaneously to the first peripheral nerve effector to stimulate the second peripheral nerve sufficient to modify a first neural circuit to modulate nervous system activity of the patient during only at least part of the second specified duration.
73. The system of any of Aspects 70-72, wherein the second peripheral nerve is a nerve on an ear of the patient.
74. The system of any of Aspects 70-73, wherein the second peripheral nerve is on a contralateral side of the patient with respect to the first peripheral nerve.
75. The system of any of Aspects 70-73, wherein the second peripheral nerve is on an ipsilateral side of the patient with respect to the first peripheral nerve.
76. The system of any of Aspects 70-74, wherein the system does not comprise any sensors configured to sense any features of the biological process.
77. The system of any of Aspects 70-75, wherein the controller is configured to signal the patient to perform the first voluntary activity by activating a display.
78. The system of any of Aspects 70-75, wherein the controller is configured to signal the patient to perform the first voluntary activity by sending audio instructions.
79. The system of any of Aspects 70-75, wherein the controller is configured to signal the patient to perform the first voluntary activity by sending tactile cues.
80. The system of any of Aspects 70-79, wherein the biological process comprises respiration.
81. The system of Aspect 80, wherein the first voluntary activity comprises a first phase of respiration.
82. The system of Aspect 80, wherein the first voluntary activity comprises a second phase of respiration.
83. A method of transcutaneously neuromodulating a one or more peripheral nerves of a patient with a wearable neuromodulation device regulated by a measured rhythmic biological signal to treat a patient, comprising:
   positioning a first peripheral nerve effector proximate a skin surface on a first location of the patient to neuromodulate a first peripheral nerve of the patient;
   positioning a second peripheral nerve effector proximate a skin surface on a second location of the patient to neuromodulate a second peripheral nerve of the patient;
   measuring a rhythmic biological signal of the patient and determining one or more features of the measured rhythmic biological signal of the patient in real-time;
   delivering a first electrical nerve neuromodulation signal transcutaneously to the first peripheral nerve effector to neuromodulate the first peripheral nerve sufficient to modify a first neural circuit to modulate nervous system activity of the patient at least in part based on the measured rhythmic biological signal; and
   delivering a second electrical nerve stimulation signal transcutaneously to the second peripheral nerve effector to neuromodulate the second peripheral nerve sufficient to modify the first or a second neural feedback loop to modulate nervous system activity of the patient at least in part based on the measured rhythmic biological signal.
84. The method of Aspect 83, wherein the features of the measured rhythmic biological signal comprise the phase, frequency, magnitude, or timing of the measured rhythmic biological signal.
85. The method of any of Aspects 83-84, further comprising adjusting one or more of the first electrical neuromodulation signal and the second electrical neuromodulation signal based on the one or more features of the measured rhythmic biological signal.
86. The method of any of Aspects 83-85, further comprising adjusting the first electrical neuromodulation signal and the second electrical neuromodulation signal based on the one or more features of the measured rhythmic biological signal.
87. The method of any of Aspects 83-86, further comprising adjusting the first electrical neuromodulation signal based on a first feature of the measured rhythmic biological signal, and adjusting the second electrical neuromodulation signal based on a second feature of the measured rhythmic biological signal.
88. The method of any of Aspects 83-87, wherein the first location is on a first ear of the patient.
89. The method of any of Aspects 83-88, wherein the second location is on the first ear of the patient.
90. The method of any of Aspects 83-89, wherein the second location is on a second ear of the patient.
91. The method of any of Aspects 83-90, wherein the second location is not on the first ear of the patient.
92. A wearable neuromodulation system for transcutaneously stimulating peripheral nerves of a patient regulated by a rhythmic biological signal, comprising:
   a first peripheral nerve effector configured to be positioned proximate a first location on the skin surface on a first ear of the patient;
   a second peripheral nerve effector configured to be positioned at a second location of the patient;
   a first biomedical sensor configured to detect activity relating to a rhythmic biological signal of the patient and convert the detected activity into a corresponding rhythmic detection signal;
   a controller in operable communication with the first biomedical sensor, the controller configured to analyze the rhythmic detection signal and recognize one or more features of the rhythmic biological signal of the patient in real-time,
   wherein the controller is configured to generate a first electrical nerve neuromodulation signal transcutaneously to the first peripheral nerve effector to stimulate a first peripheral nerve sufficient to modify a first neural feedback loop upon detection of a predetermined feature of the rhythmic biological signal,
   wherein the controller is configured to generate a second electrical nerve stimulation signal transcutaneously to the second peripheral nerve effector to neuromodulation a second peripheral nerve associated to modify the first or a second neural feedback loop.
93. The system of Aspect 92, wherein the one or more features of the rhythmic biological signal comprise the phase, frequency, magnitude, or timing of the measured rhythmic biological signal.
94. The system of any of Aspects 92 or 93, wherein the controller is further configured to adjust one or more of the first electrical neuromodulation signal and the second electrical neuromodulation signal based on the one or more features of the measured rhythmic biological signal.
95. The system of any of Aspects 92-94, wherein the controller is further configured to adjust the first electrical neuromodulation signal and the second electrical neuromodulation signal based on the one or more features of the measured rhythmic biological signal.
96. The system of any of Aspects 92-95, wherein the controller is further configured to adjust the first electrical neuromodulation signal based on a first feature of the measured rhythmic biological signal, and adjust the second electrical neuromodulation signal based on a second feature of the measured rhythmic biological signal.
97. A method of transcutaneously neuromodulating one or more nerves of a patient with a neuromodulation device, comprising:
   positioning a first nerve effector proximate a first skin surface of the patient to neuromodulate a first nerve of the patient;
   positioning a second nerve effector proximate a second skin surface of the patient to neuromodulate a second peripheral nerve of the patient;
   measuring a rhythmic biological signal of the patient and determining one or more features of the measured rhythmic biological signal of the patient in real-time;
   delivering a first electromagnetic neuromodulation signal transcutaneously to the first peripheral nerve effector to neuromodulate the first peripheral nerve sufficient to modify a first neural circuit to modulate nervous system activity of the patient at least in part based on the measured rhythmic biological signal; and
   delivering a second electromagnetic neuromodulation signal transcutaneously to the second peripheral nerve effector to neuromodulate the second peripheral nerve sufficient to modify the first or a second neural feedback loop to modulate nervous system activity of the patient at least in part based on the measured rhythmic biological signal,
   wherein the first peripheral nerve is a vagus nerve, and the second peripheral nerve is a nerve other than the vagus nerve,
   wherein the second electromagnetic nerve neuromodulation signal is offset at a preselected time interval from the first electromagnetic neuromodulation signal.
98. The method of Aspect 97, wherein the first electromagnetic neuromodulation signal comprises an electrical neuromodulation signal.
99. The method of Aspect 97, wherein the first electromagnetic neuromodulation signal comprises a vibrational neuromodulation signal.
100. A wearable neuromodulation system for transcutaneously neuromodulating peripheral nerves of a patient regulated by a rhythmic biological signal, comprising:
   a first peripheral nerve effector configured to be positioned proximate a first location on the skin surface on a first ear of the patient;
   a second peripheral nerve effector configured to be positioned at a second location of the patient;
   a first biomedical sensor configured to detect activity relating to a rhythmic biological signal of the patient and convert the detected activity into a corresponding rhythmic detection signal;
   a controller in operable communication with the first biomedical sensor, the controller configured to analyze the rhythmic detection signal and recognize one or more features of the rhythmic biological signal of the patient in real-time,
   wherein the controller is configured to generate a first electromagnetic nerve neuromodulation signal transcutaneously to the first peripheral nerve effector to stimulate a first peripheral nerve sufficient to modify a first neural feedback loop upon detection of a predetermined feature of the rhythmic biological signal,
   wherein the controller is configured to generate a second electrical nerve stimulation signal transcutaneously to the second peripheral nerve effector to neuromodulation a second peripheral nerve associated to modify the first or a second neural feedback loop.
101. The system of Aspect 100, wherein the one or more features of the rhythmic biological signal comprise the phase, frequency, magnitude, or timing of the measured rhythmic biological signal.
102. The system of any of Aspects 100 or 101, wherein the controller is further configured to adjust one or more of the first neuromodulation signal and the second neuromodulation signal based on the one or more features of the measured rhythmic biological signal.
103. A method of transcutaneously stimulating one or more peripheral nerves of a patient with a wearable neurostimulation device regulated by a measured rhythmic biological signal to treat a patient, comprising:
   positioning a first peripheral nerve effector proximate a skin surface on a first ear of the patient to stimulate a first peripheral nerve of the patient;
   positioning a second peripheral nerve effector proximate a skin surface proximate a first or a second ear of the patient to stimulate a second peripheral nerve of the patient;
   measuring a rhythmic biological signal of the patient and determining one or more features of the measured rhythmic biological signal of the patient in real-time;
   delivering a first electrical nerve stimulation signal transcutaneously to the first peripheral nerve effector to stimulate the first peripheral nerve sufficient to modify a first neural circuit to modulate nervous system activity of the patient at least in part based on the measured rhythmic biological signal; and
   delivering a second electrical nerve stimulation signal transcutaneously to the second peripheral nerve effector to stimulate the second peripheral nerve sufficient to modify the first or a second neural feedback loop to modulate nervous system activity of the patient at least in part based on the measured rhythmic biological signal, and
   adjusting one or more of the first electrical stimulation signal and the second electrical stimulation signal based on the one or more features of the measured rhythmic biological signal,
   wherein the first peripheral nerve is the auricular vagus nerve, and the second peripheral nerve is a nerve other than the auricular vagus nerve and selected from the group consisting of the trigeminal nerve, greater auricular nerve, auriculotemporal nerve, and the lesser occipital nerve,
   wherein the second electrical nerve stimulation signal is offset at a preselected time interval from the first electrical stimulation signal,
   wherein the rhythmic biological signal is a respiratory signal,
   wherein the features of the measured rhythmic biological signal comprise the phase, frequency, magnitude, or timing of the measured rhythmic biological signal, and
   wherein at least one of the first electrical nerve stimulation signal and the second electrical nerve stimulation signal comprises burst stimulation.
104. The method of Aspect 103, further comprising stimulating the first peripheral nerve during a first phase of the rhythmic biologic signal, and stimulating the second peripheral nerve during a second phase of the rhythmic biological signal.
105. The method of Aspects 103-104, wherein the first peripheral nerve is associated with the parasympathetic nervous system, and the second peripheral nerve is associated with the sympathetic nervous system.
106. The method of Aspects 104-105, wherein the first phase is expiration, and the second phase is inspiration.
107. A wearable neurostimulation system for transcutaneously stimulating peripheral nerves of a patient regulated by a rhythmic biological signal, comprising:
   a first peripheral nerve effector configured to be positioned proximate a first location on the skin surface on a first ear of the patient;
   a second peripheral nerve effector configured to be positioned at a second location of the patient proximate the first ear or a second ear of the patient;
   a first biomedical sensor configured to detect activity relating to a rhythmic biological signal of the patient and convert the detected activity into a corresponding rhythmic detection signal;
   a controller in operable communication with the first biomedical sensor, the controller configured to analyze the rhythmic biological signal and recognize one or more features of the rhythmic biological signal of the patient in real-time,
   wherein the controller is configured to generate a first electrical nerve stimulation signal transcutaneously to the first peripheral nerve effector to stimulate a first peripheral nerve sufficient to modify a first neural feedback loop upon detection of a predetermined feature of the rhythmic biological signal, wherein the first peripheral nerve is the auricular branch of the vagus nerve,
   wherein the controller is configured to generate a second electrical nerve stimulation signal transcutaneously to the second peripheral nerve effector to stimulate a second peripheral nerve other than the auricular branch of the vagus nerve and associated to modify the first or a second neural feedback loop upon detection of the first or a second predetermined feature of the rhythmic biological signal,
   wherein the second electrical nerve stimulation signal is offset at a preselected time interval from the first electrical stimulation signal,
   wherein the rhythmic biological signal is a respiratory signal,
   wherein the features of the measured rhythmic biological signal comprise the phase, frequency, magnitude, or timing of the measured rhythmic biological signal, and
   wherein at least one of the first electrical nerve stimulation signal and the second electrical nerve stimulation signal comprises burst stimulation.
108. The system of Aspect 107, wherein the rhythmic biological signal is the breathing cycle and the first predetermined phase is an exhalation phase of the breathing cycle.
109. The system of Aspects 107-108, wherein the rhythmic biological signal is the breathing cycle and the second predetermined phase is an inhalation phase of the breathing cycle.
110. A wearable neurostimulation system for transcutaneously stimulating peripheral nerves of a patient regulated by a rhythmic biological signal, comprising:
   a first peripheral nerve effector configured to be positioned proximate a first location on the skin surface on a first ear of the patient;
   a second peripheral nerve effector configured to be positioned at a second location of the patient proximate the first ear or a second ear of the patient;
   a first biomedical sensor configured to detect activity relating to a rhythmic biological signal of the patient and convert the detected activity into a corresponding rhythmic detection signal;
   a controller in operable communication with the first biomedical sensor, the controller configured to analyze the rhythmic biological signal and recognize one or more features of the rhythmic biological signal of the patient in real-time,
   wherein the controller is configured to generate a first electrical nerve stimulation signal transcutaneously to the first peripheral nerve effector to stimulate a first peripheral nerve sufficient to modify a first neural feedback loop upon detection of a predetermined feature of the rhythmic biological signal, wherein the first peripheral nerve is the auricular branch of the vagus nerve of a first ear,
   wherein the controller is configured to generate a second electrical nerve stimulation signal transcutaneously to the second peripheral nerve effector to stimulate a second peripheral nerve which is the auricular branch of the vagus nerve of a second ear and associated to modify the first or a second neural feedback loop upon detection of the first or a second predetermined feature of the rhythmic biological signal,
   wherein the second electrical nerve stimulation signal is offset at a preselected time interval from the first electrical stimulation signal,
   wherein the rhythmic biological signal is a respiratory signal,
   wherein the features of the measured rhythmic biological signal comprise the phase, frequency, magnitude, or timing of the measured rhythmic biological signal, and
   wherein at least one of the first electrical nerve stimulation signal and the second electrical nerve stimulation signal comprises burst stimulation.
111. The system of Aspect 110, wherein the rhythmic biological signal is the breathing cycle and the first predetermined phase is an exhalation phase of the breathing cycle.
112. The system of Aspects 110-111, wherein the rhythmic biological signal is the breathing cycle and the second predetermined phase is an inhalation phase of the breathing cycle.

## Claims

1. A neurostimulation system for transcutaneously stimulating one or more peripheral nerves correlated with one or more rhythmic biological signals of a patient, the system comprising:
one or more electrodes configured to be worn in or on one or more ears of the patient;
a stimulator in operable communication with the one or more electrodes;
a power source;
a processor or controller; and
memory,
wherein the processor or controller is configured to cause the stimulator to provide stimulation signals to the one or more electrodes so as to provide electrical stimulation to the one or more peripheral nerves that is correlated with the one or more rhythmic biological signals of the patient,
wherein the one or more rhythmic biological signals comprises respiratory activity of the patient, and
wherein the electrical stimulation is correlated with the one or more rhythmic biological signals based on (i) assessment of sounds associated with the respiratory activity or (ii) synchronization with a user signaling device in communication with the processor or controller that is configured to provide instructional feedback to the patient to guide voluntary breathing and the electrical stimulation is delivered when the patient is being instructed to exhale or inhale.

2. The system of Claim 1, wherein the electrical stimulation is correlated with the one or more rhythmic biological signals based on assessment of sounds associated with the respiratory activity, and wherein the system further comprises a microphone configured to assess sounds associated with the respiratory activity.

3. The system of Claim 1, wherein the electrical stimulation is correlated with the one or more rhythmic biological signals based on assessment of sounds associated with the respiratory activity, and wherein the system further comprises one or more microphone sensors placed in proximity to the respiratory airways or over the throat to audibly detect phases of the respiratory cycle via the variation of sound.

4. The system of Claim 1, wherein the electrical stimulation is correlated with the one or more rhythmic biological signals based on synchronization with a user signaling device in communication with the processor or controller that is configured to provide instructional feedback to the patient to guide voluntary breathing and the electrical stimulation is delivered when the patient is being instructed to exhale or inhale, and wherein the user signaling device comprises one or more of: a visual display presented on a screen, audio instructions, and discrete tactile cues.

5. The system of Claim 4, wherein the user signaling device comprises a visual display presented on a screen, and wherein the computer screen can instruct the patient to inhale for a specified duration and then exhale for a specified duration.

6. The system of Claim 4, wherein the user signaling device comprises a computer monitor, a tablet computer, a smart phone, or a smart watch.

7. The system of Claim 4, wherein the electrical stimulation is configured to be delivered when the patient is being instructed to exhale.

8. The system of any one of the preceding claims, wherein the electrical stimulation treats rheumatoid arthritis and/or inflammatory bowel disease.

9. The system of any one of the preceding claims, wherein the system does not have any implantable components.

10. The system of any one of the preceding claims, wherein the one or more peripheral nerves comprise an auricular branch of the vagus nerve.

11. The system of any one of the preceding claims, wherein the one or more peripheral nerves comprise one or more branches of a vagus nerve.

12. The system of any one of the preceding claims, further comprising:
a) a pulse sensor on an extremity to measure blood pressure and derive waveforms to calculate cardiac performance;
b) an electrically conductive sensor on a chest, a torso, an upper extremity, or in the ear to detect electrocardiogram, cardiac activity, heart rate, or heart rate variability;
c) a photoplethysmography sensor on an upper extremity or in the ear to detect cardiac activity, heart rate, or heart rate variability; or
d) a sensor configured to measure at least one of electrodermal activity, heart rate variability, thermometry, pupillometry and electrocardiogram information of the patient.

13. The system of Claim 1, wherein the stimulator comprises a pulse generator, and wherein the stimulator is in operable communication with the one or more electrodes via a wired or wireless connection.

14. The system of Claim 13, wherein the electrical stimulation is correlated with the one or more rhythmic biological signals based on assessment of sounds associated with the respiratory activity or, and wherein the processor or controller is configured to receive one or more signals generated by one or more sensors to control timing and parameters of the electrical stimulation generated by the pulse generator based on instructions stored in the memory.

15. The system of claim 1, further comprising a communication module to transmit data to other devices or remote server via wired or wireless communication protocols.
